# EUROPEAN PATENT SPECIFICATION

(11) **EP 4 251 620 B1**
(45) Date of publication and mention of the grant of the patent: **16.04.2025**
(21) Application number: 21811098.9
(22) Date of filing: 26.11.2021
(51) Int. Cl.: C07D 403/12, C07D 403/14, C07D 413/12, C07D 417/12, C07D 417/14, A61K 31/517, A61P 11/00

(54) **AMINO QUINAZOLINE DERIVATIVES AS P2X3 INHIBITORS**
AMINOCHINAZOLINDERIVATE ALS P2X3-HEMMER
DÉRIVÉS D'AMINO-QUINAZOLINE UTILISÉS EN TANT QU'INHIBITEURS DE P2X3

(30) Priority: 27.11.2020 EP 20210189
(43) Date of publication of application: 04.10.2023
(73) Proprietor: Chiesi Farmaceutici S.p.A., 43122 Parma (IT)
(72) Inventor: FIORELLI, Claudio, 43122 Parma (IT); BRUNO, Paolo, 43122 Parma (IT); BIAGETTI, Matteo, 43122 Parma (IT); PIZZIRANI, Daniela, 43122 Parma (IT); PALA, Daniele, 43122 Parma (IT); RONCHI, Paolo, 43122 Parma (IT); GUARIENTO, Sara, 43122 Parma (IT); BAKER-GLENN, Charles, 43122 Parma (IT); VAN DE POËL, Hervé, 43122 Parma (IT); HIRST, Kim Louise, 43122 Parma (IT)
(74) Representative: Chiesi Farmaceutici S.p.A.
(86) International application number: PCT/EP2021/083141
(87) International publication number: WO 2022/112490

(56) References cited:
- WO-A1-2020/239951
- WO-A1-2020/239952
- US-A1- 2011 009 432
- SZÁNTÓ GÁBOR ET AL: "New P2X3 receptor antagonists. Part 2: Identification and SAR of quinazolinones", BIOORGANIC & MEDICINAL CHEMISTRY LETTERS, ELSEVIER, AMSTERDAM, NL, vol. 26, no. 16, 6 July 2016 (2016-07-06), pages 3905 - 3912, XP029663839, ISSN: 0960-894X, DOI: 10.1016/J.BMCL.2016.07.013

## Description

### FIELD OF THE INVENTION

The present invention relates to compounds inhibiting P2X purinoceptor 3 (hereinafter P2X₃ inhibitors); particularly the invention relates to compounds that are amino quinazoline derivatives, methods of preparing such compounds, pharmaceutical compositions containing them and therapeutic use thereof.

The compounds of the invention may be useful in the treatment of many disorders associated with P2X₃ receptors mechanisms, such as respiratory diseases including cough, asthma, idiopathic pulmonary fibrosis (IPF) and chronic obstructive pulmonary disease (COPD).

### BACKGROUND OF THE INVENTION

P2X receptors are cell surface ion channels activated by extracellular Adenosine 5-TriPhosphate (ATP). P2X receptor family are trimeric assemblies composed of seven distinct subunit subtypes (P2X1-7) that assemble as homomeric and heteromeric channels. All subunits share a common topology containing intracellular termini, two transmembrane helices forming the ion channels and a large extracellular domain containing the ATP binding site. Homomeric P2X₁, P2X₂, P2X₃, P2X₄, P2X₅, and P2X₇ channels and heteromeric P2X_{2/3} and P2X_{1/5} channels have been fully characterized following heterologous expression. P2X receptors are abundantly distributed, and functional responses are seen in neurons, glia, epithelia, endothelia, bone, muscle, and hemopoietic tissues. On smooth muscles, P2X receptors respond to ATP released from sympathetic motor nerves (e.g., in ejaculation). On sensory nerves, they are involved in the initiation of afferent signals in several viscera (e.g., bladder, intestine) and play a key role in sensing tissue-damaging and inflammatory stimuli. Paracrine roles for ATP signaling through P2X receptors are likely in neurohypophysis, ducted glands, airway epithelia, kidney, bone and hemopoietic tissues. (RA. North: Molecular Physiology of P2X Receptors; Physiol Rev, Vol 82, Oct 2002). All P2X receptors are non-selective cation channels permeable to Na+ and Ca+ ions and are activated by ATP; however, the pharmacology of the receptor subtypes varies with respect to sensitivity to ATP and to small molecules antagonists. (K Kaczmarek-Hajek et al: Molecular and functional properties of P2X receptors - recent progress and persisting challenges; Purinergic Signalling 8:375-417, 2012)

In humans, the P2X₃ receptor has been reported in heart and spinal cord at the mRNA level and in DRG, intestine (myenteric plexus neurons), urinary bladder (urothelium and suburothelium), and dental pulp at the protein level (Garcia-Guzman M et al: Molecular characterization and pharmacological properties of the human P2X3 purinoceptor: Brain Res Mol Brain Res. 1997;47(1-2):59-66).

The neurophysiological role of P2X₃ receptors in sensory nerve function in the airways is similar to that mediating somatic nociception (Undem BJ and Nassenstein C: Airway nerves and dyspnea associated with inflammatory airway disease, Respir Physiol Nerobiol 167: 36-44, 2009). This similarity has driven hypotheses concerning the involvement of P2X₃ receptors in the symptoms of airway dysfunction including cough and bronchial hyper-reactivity (Ford AP: In pursuit of P2X₃ antagonists: novel therapeutics for chronic pain and and afferent sensitization, Purinergic signal 8 (suppl 1):3-26, 2012; North RA, Jarvis MF P2X Receptors as Drug Targets; Mol Pharmacol, 83:759-769, 2013). P2X₃ subunits are also co-localized in many neurons, particularly within DRG, nodose ganglia, nucleus tractus solitarius, and taste buds (Cheung KK, Burnstock G: Localization of P2X3 receptors and coexpression with P2X2 receptors during rat embryonic neurogenesis. J Comp Neurol 443(4):368-382 2002)

P2X₃ antagonists have been proposed for the treatment of diabetic neuropathic pain (Guo J et al: Contributions of purinergic P2X3 receptors within the midbrain periaqueductal gray to diabetes-induced neuropathic pain, J Physiol Sci Jan;65(1):99-104 2015).

P2X₃ and P2X_{2/3} channels play an important role in the development of articular hyperalgesia of arthritic joints (Teixeira JM et al: P2X3 and P2X2/3 Receptors Play a Crucial Role in Articular Hyperalgesia Development Through Inflammatory Mechanisms in the Knee Joint Experimental Synovitis, Mol Neurobiol Oct;54(8):6174-6186, 2017).

P2X₃ are also a potential target for therapeutic treatment of bladder pain. They were also proposed to be analgesic targets to treat ureteral colicky pain and to facilitate ureteral stone passage (Canda AE et al: Physiology and pharmacology of the human ureter: basis for current and future treatments, Urol Int. 78(4):289-98, 2007).

P2X₃ over-expression is involved in poor recurrence-free survival in hepatocellular carcinoma patients and identifies the P2X₃ as a potential therapeutic target (Maynard JP et al: P2X3 purinergic receptor overexpression is associated with poor recurrence-free survival in hepatocellular carcinoma patients Oncotarget Dec 1;6(38):41162-79, 2015).

It has been suggested that P2X₃ antagonists may improve recovery of erectile function (Li CL et al: Effects of intracavernous injection of P2X3 and NK1 receptor antagonists on erectile dysfunction induced by spinal cord transection in rats, Andrologia. Feb;47(1):25-9, 2015).

ATP enhances citric acid-evoked and histamine-evoked cough in preclinical models, effects that can be attenuated by P2X₃ selective antagonists (Kamei J and Takahashi Y: Involvement of ionotropic purinergic receptors in the histamine-induced enhancement of the cough reflex sensitivity in guinea pigs, Oct 10;547(1-3):160-4, 2006). In humans, local delivery of ATP initiates cough and bronchospasm (Basoglu OK et al: Effects of aerosolized adenosine 5'-triphosphate vs adenosine 5'-monophosphate on dyspnea and airway caliber in healthy nonsmokers and patients with asthma, Chest. Oct;128(4):1905-9, 2005).

The therapeutic promise of P2X₃ antagonists for the treatment of chronic cough was first recognized by Ford and Undem (Ford AP, Undem BJ: The therapeutic promise of ATP antagonism at P2X3 receptors in respiratory and urological disorders, Front Cell Neurosci, Dec 19;7:267, 2013). P2X₃ are expressed by airway afferent nerves and mediate hypersensitivity of the cough reflex, which is dramatically reduced by the oral P2X₃ antagonist, AF-219 (Abdulqawi et al: P2X3 receptor antagonist (AF-219) in refractory chronic cough: a randomised, double-blind, placebo-controlled phase 2 study, Lancet 385, 1198-205, 2015).

ATP is a key neurotransmitter in the taste system, acting largely via P2X_{2/3} heteromultimer receptors. Consequently, disruption of taste function may be an unintentional consequence of therapeutic trials of pain, chronic cough and other conditions using purinergic P2X₃ antagonists (Vandenbeuch A et al: Role of the ectonucleotidase NTPDase2 in taste bud function, Proc Natl Acad Sci U S A, Sep 3;110(36):14789-94, 2013. Bo X et al: Localization of ATP-gated P2X2 and P2X3 receptor immunoreactive nerves in rat taste buds, Neuroreport, 10(5):1107-11, 1999).

Various compounds have been described in the literature as P2X₃ and/or P2X_{2/3} Inhibitors.

WO2017058645 (Afferent Pharmaceuticals INC) discloses the use of diaminopyrimidine P2X₃/P2X_{2/3} antagonists for the treatment of disorders including cough, chronic cough and urge to cough, including cough associated with a respiratory disease or disorder, administering an efficacious amount of the compound disclosed. However, amino quinazoline derivatives are not disclosed.

WO2017011729 (Patara Pharma LLC), discloses the use of cromolyn or a pharmaceutically acceptable salt thereof and P2X₃ and/or a P2X_{2/3} receptor antagonist as antitussive agent, for the treatment of lung diseases and conditions.

WO2016091776, (Evotec AG), discloses 1,3-thiazol-2-yl substituted benzamide compounds that inhibit P2X₃ receptor and to pharmaceutical compositions containing such compounds, and the use of compounds for the treatment of several disorders, including the respiratory diseases.

WO2016088838 (Shionogi), discloses purine derivatives compounds having a novel P2X₃ and/or P2X_{2/3} receptor antagonizing effect.

WO2016084922, (Shionogi), discloses triazine derivatives compounds having a novel P2X₃ and/or P2X_{2/3} receptor antagonizing effect

WO2008123963 (Renovis) relates to fused heterocyclic compounds of the class tetrahydropyrido[4,3-d]pyrimidines and pharmaceutical compositions comprising such compounds. Also provided are methods for preventing and/or treating several disorders, such as neurodegenerative disorders, pain, asthma, autoimmune disorders administering the disclosed comoounds.

WO2008130481 (Renovis) discloses 2-cyanophenyl fused heterocyclic compounds of the class tetrahydropyrido[4,3-d]pyrimidines and pharmaceutical compositions comprising such compounds.

WO2010033168 (Renovis) discloses a series of benzamides substituted with phenyl or pyridyl which are stated to be useful for treatment of diseases associated with P2X purinergic receptors, and more particularly to P2X₃ receptor and/ or P2X_{2/3} receptor antagonists. However, amino quinazoline derivatives are not disclosed.

WO2009110985 (Renovis) relates to phenyl- and pyridyl-substituted benzamide compounds and pharmaceutical compositions comprising such compounds, but not thiazole-substituted benzamides, rendering said compounds different from the compounds of the present invention.

WO2008000645 (Roche) discloses tetrazole substituted arylamides compounds antagonists of P2X₃ and/or P2X_{2/3} receptors, useful for the treatment of genitourinary, pain, gastrointestinal and respiratory diseases, conditions and disorders.

Despite the above cited prior art, there is still the need of novel amino quinazoline compounds for treatment of diseases associated with P2X₃ receptors in many therapeutic areas such as in particular the respiratory diseases, preferably having a selective action on the P2X₃ receptor.

Of note, the state of the art does not describe or suggest amino quinazoline derivatives compounds of general formula (I) of the present invention which represent a solution to the aforementioned need.

### SUMMARY OF THE INVENTION

The present invention refers to compounds of formula (I) 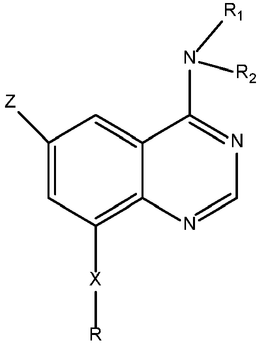 wherein
**X** is selected from S, SO₂, SO, or O;
**Z** is selected from the group consisting of heteroaryl and aryl, wherein any of such heteroaryl and aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R** is selected from the group consisting of (C₁-C₆)alkyl-, (C₁-C₆)alkyl-CN, (C₁-C₆)haloalkyl, -NR_{A}R_{B}, (C₃-C₈)heterocycloalkyl-(C₁-C₆)alkyl-, (C₃-C₈)heterocycloalkyl-, (C₃-C₈)heteroaryl-(C₁-C₆)alkyl-, (C₃-C₈)cycloalkyl-(C1-C₆)alkyl-, (C₃-C₈)cycloalkyl-, R_{A}O-(C₁-C₆)alkyl-O-(C₁-C₆)alkyl-, R_{A}NH-C(O)-(C₁-C₄)alkyl-, R_{A}R_{B}N-C(O)-(C₁-C₄)alkyl-, R_{A}O-C(O)(C₁-C₆)alkyl-,
   wherein any of such heterocycloalkyl is unsubstituted or substituted by one or more groups selected from OH, -C(O)R_{A}, -C(O)OR_{A}, (C₁-C₆)haloalkyl, -NH-C(O)R₁, and oxo; any of such cycloalkyl is substituted by one or more groups selected from -C(O)OR_{A} and (C₁-C₆)haloalkyl-,
   any of such heteroaryl may be optionally substituted by one or more groups selected from (C₁-C₆)alkyl-, -OH and (C₃-C₆)cycloalkyl-;
**R_{A}** and **R_{B}** are at each occurrence independently H or selected from the group consisting of (C₁-C₆)alkyl-, (C₁-C₆)haloalkyl-, -OR₁, -SO₂R_{C} and (C₃-C₆)cycloalkyl wherein such cycloalkyl may be optionally substituted by one or more -C(O)OR₁, or alternatively
**R_{A}** and **R_{B}** may form together with the nitrogen atom to which they are attached a 5 or 6 membered saturated heterocyclic monocyclic ring system optionally containing a further heteroatom which is nitrogen or oxygen, which may be optionally substituted by one or more groups selected from halo, -OR₁;
**R_{C}** is aryl; with the proviso that R is (C₁-C₆)alkyl- or unsubstituted (C₃-C₈)heterocycloalkyl- only when X is S or SO.

In a second aspect, the invention refers to a pharmaceutical composition comprising a compound of formula (I) or pharmaceutically acceptable salt thereof, either alone or in combination with another one or more active ingredient, in admixture with one or more pharmaceutically acceptable carrier or excipient.

In a third aspect, the invention provides a compound of formula (I) for the use as a medicament.

In a further aspect, the invention provides the use of a compound of formula (I) for use in treatment of any disease wherein the P2X₃ receptors are involved.

In a further aspect, the invention refers to a compound of formula (I) for use in the prevention and/or treatment of respiratory diseases including cough, sub-acute or chronic cough, treatment-resistant cough, idiopathic chronic cough, post-viral cough, iatrogenic cough, asthma, idiopathic pulmonary fibrosis (IPF), chronic obstructive pulmonary disease (COPD) and cough associated with respiratory diseases such as COPD, asthma and bronchospasm.

### DETAILED DESCRIPTION OF THE INVENTION

Unless otherwise provided, the term compound of formula (I) comprises in its meaning stereoisomer, tautomer or pharmaceutically acceptable salt or solvate.

The term "pharmaceutically acceptable salts", as used herein, refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

The term "halogen" or "halogen atoms" as used herein includes fluorine, chlorine, bromine, and iodine atom, preferably chlorine or fluorine.

The term "(Cₓ-C_{y}) alkyl" wherein x and y are integers, refers to a straight or branched chain alkyl radical having from x to y carbon atoms. Thus, when x is 1 and y is 6, for example, the term includes methyl, ethyl, n-propyl, isopropyl, n-butyl, isobutyl, sec-butyl, t-butyl, n-pentyl and n-hexyl.

As used herein, the term "(Cₓ-C_{y})alkylene" wherein x and y are integers, refers to a Cₓ-C_{y}alkyl radical having in total two unsatisfied valencies, such as a divalent methylene radical.

The expressions "(Cₓ-C_{y}) haloalkyl" wherein x and y are integers, refer to the above defined "Cₓ-C_{y}alkyl" groups wherein one or more hydrogen atoms are replaced by one or more halogen atoms, which can be the same or different.

Examples of said "(Cₓ-C_{y}) haloalkyl" groups may thus include halogenated, poly-halogenated and fully halogenated alkyl groups wherein all of the hydrogen atoms are replaced by halogen atoms, e.g. trifluoromethyl or difluoro methyl, trifluoroethyl groups.

By way of analogy, the terms "(C₁-C₆) hydroxyalkyl" or "(C₁-C₆) aminoalkyl" refer to the above defined "(C₁-C₆) alkyl" groups wherein one or more hydrogen atoms are replaced by one or more hydroxy (OH) or amino group respectively. Examples include respectively hydroxymethyl, aminomethyl, dimethylaminopropyl and the like.

In the present description, unless otherwise provided, the aminoalkyl encompasses alkyl groups (i.e. "(C₁-C₆) alkyl" groups) substituted by one or more amino group (-NR^{A}R^{B}). Thus, an example of aminoalkyl is a mono-aminoalkyl group such as R^{A}R^{B}N-(C₁-C₆) alkyl.

The term "(Cₓ-C_{y}) cycloalkyl" wherein x and y are integers, refers to saturated cyclic hydrocarbon groups containing the indicated number of ring carbon atoms. Examples include cyclopropyl, cyclobutyl, cyclopentyl, cyclohexyl, cycloheptyl.

The term "aryl" refers to mono cyclic carbon ring systems which have 6 ring atoms wherein the ring is aromatic. Examples of suitable aryl monocyclic ring systems include, for instance, phenyl.

The term "heteroaryl" refers to a mono- or bi-cyclic aromatic radical containing one or more heteroatoms selected from S, N and O, and includes radicals having two such monocyclic rings, or one such monocyclic ring and one monocyclic aryl ring, which are fused through a common bond. Examples of suitable 5,6-membered heteroaryl are: are thienyl, furyl, pyrrolyl, imidazolyl, thiazolyl, isothiazolyl, pyrazolyl, oxazolyl, isoxazolyl, isothiazolyl, triazolyl, thiadiazolyl, oxadiazolyl, pyridinyl, pyridazinyl, pyrimidinyl, pyrazinyl, tetrazolyl and triazinyl.

The term "heterocyclyl" or "heterocyclic" relate to a saturated mono-, bi- or tricyclic non-aromatic radical containing one or more heteroatoms selected from S, N and O. In the case of bicyclic heterocyclic systems, included within the scope of the term are fused, spiro and bridged bicyclic systems.

The term "(Cₓ-C_{y}) heterocycloalkyl" wherein x and y are integers, refers to saturated or partially unsaturated monocyclic (Cₓ-C_{y}) cycloalkyl groups in which at least one ring carbon atom is replaced by at least one heteroatom (e.g. N, S or O) or may bear an -oxo (=O) substituent group. Said heterocycloalkyl (i.e. heterocyclic radical or group) may be further optionally substituted on the available positions in the ring, namely on a carbon atom, or on an heteroatom available for substitution. Substitution on a carbon atom includes spiro disubstitution as well as substitution on two adjacent carbon atoms, in both cases thus form additional condensed 5 to 6 membered heterocyclic ring. Examples of (Cₓ-C_{y}) heterocycloalkyl are represented by: pyrrolidinyl, imidazolidinyl, thiazolidinyl, piperazinyl, piperidinyl, morpholinyl, thiomorpholinyl, dihydro- or tetrahydro-pyridinyl, tetrahydrothiophenyl, azetidinyl, oxetanyl, tetrahydropyranyl, pyranyl, 2H- or 4H-pyranyl, dihydro- or tetrahydrofuranyl, dihydroisoxazolyl, pyrrolidin-2-one-yl, dihydropyrrolyl radicals and the like.

Specific examples of said heterocycle radicals are tetrahydrothiophene 1,1-dioxide, 3,3-difluoropyrrolidinyl, 1-pyrrolidinyl, 1-methyl-2-pyrrolidinyl, 1-piperidinyl, 1-piperazinyl, 4-morpholinyl.

The expressions "Aryloxyl" and "Aryl (C₁-C₆) alkoxyl" likewise "heteroAryloxyl" and "Heteroaryl (C₁-C₆) alkoxyl" refer to Aryl or Heteroaryl groups attached through an oxygen bridge and chained Aryl-alkoxyl or HeteroAryl-alkoxyl groups. Examples of such groups are phenyloxy, benzyloxy and pyridinyloxy respectively.

The term "aryl (C₁-C₆) alkyl" refers to an aryl ring linked to a straight-chained or branched alkyl groups wherein the number of carbon atoms is from 1 to 6, e.g. phenylmethyl (i.e. benzyl), phenylethyl or phenylpropyl.

The term (C_{z}-Cₖ)heterocycloalkyl-(Cₓ-C_{y})alkyl wherein z and k are integers, refers to an heterocyclic ring linked to a straight-chained or branched alkyl groups having from x to y carbon atoms.

Likewise, the term "heteroaryl (Cₓ-C_{y})alkyl" or "aryl (Cₓ-C_{y})alkyl" refers to an heteroaryl or aryl ring linked to a straight-chained or branched alkyl groups having from x to y carbon atoms.

The expression "ring system" refers to mono- or bicyclic or polycyclic ring systems which may be saturated, partially unsaturated or unsaturated, such as aryl, (C₃-C₁₀) cycloalkyl, (C₃-C₆) heterocycloalkyl or heteroaryl.

The terms "group", "radical" or "fragment" or "substituent" are synonymous and are intended to indicate functional groups or fragments of molecules attachable to a bond or other fragments or molecules. Thus, as an example, a "heterocyclic radical" herein refers to a mono- or bi-cyclic saturated or partially saturated heterocyclic moiety (group, radical), preferably a 4 to 11 membered monocyclic radical, at least one further ring carbon atom in the said heterocyclic radical is optionally replaced by at least one further heteroatom independently selected from N, S or O and/or may bear an -oxo (=O) substituent group, said heterocyclic radical is further optionally including spiro disubstitution as well as substitution on two adjacent or vicinal atoms forming an additional 5 to 6 membered cyclic or heterocyclic, saturated, partially saturated or aromatic ring. Examples of said heterocycle radicals are 1-pyrrolidinyl, 1-piperidinyl, 1-piperazinyl, 4-morpholinyl and the like.

A dash ("-") that is not between two letters or symbols is meant to represent the point of attachment for a substituent. When graphically represented the point of attachment in a cyclic functional group is indicated with a dot ("•") localized in one of the available ring atom where the functional group is attachable to a bond or other fragment of molecules.

An oxo moiety is represented by (O) as an alternative to the other common representation, e.g. (=O). Thus, in terms of general formula, the carbonyl group is herein represented as -C(O)-, in general, the bracketed group is a lateral group, not included into the chain, and brackets are used, when deemed useful, to help disambiguating linear chemical formulas; e.g. the sulfonyl group -SO₂- might be also represented as -S(O)₂- to disambiguate e.g. with respect to the sulfinic group -S(O)O-.

Whenever basic amino or quaternary ammonium groups are present in the compounds of formula I, physiologically acceptable anions may be present, selected among chloride, bromide, iodide, trifluoroacetate, formate, sulfate, phosphate, methanesulfonate, nitrate, maleate, acetate, citrate, fumarate, tartrate, oxalate, succinate, benzoate,
p-toluenesulfonate, pamoate and naphthalene disulfonate. Likewise, in the presence of acidic groups such as COOH groups, corresponding physiological cation salts may be present as well, for instance including alkaline or alkaline earth metal ions.

It will be apparent that compounds of formula (I) when contain one or more stereogenic center, may exist as optical stereoisomers.

Where the compounds according to the invention have at least one stereogenic center, they may accordingly exist as enantiomers. Where the compounds according to the invention possess two or more stereogenic centers, they may additionally exist as diastereoisomers. All such single enantiomers, diastereoisomers and mixtures thereof in any proportion are encompassed within the scope of the present invention. The absolute configuration (R) or (S) for carbon bearing a stereogenic center is assigned on the basis of Cahn-Ingold-Prelog nomenclature rules based on groups' priorities.

The invention further concerns the corresponding deuterated derivatives of compounds of formula (I).

All preferred groups or embodiments described above and herebelow for compounds of formula I may be combined among each other and apply as well *mutatis mutandis.*

As above indicated, the present invention refers to a series of compounds represented by the general formula (I) as herein below described in details, which are endowed with an antagonist property versus receptor P2X₃.

Differently from similar compounds of the prior art, the compounds of formula (I) of the present invention are able to act as antagonist P2X₃ in a substantive and effective way, particularly appreciated by the skilled person when looking at a suitable and efficacious compounds useful for the treatment of respiratory disease, in particular chronic cough.

As indicated in the experimental part, the compounds of formula (I) have an activity as shown in Table 2, wherein for each compound is reported the potency expressed as half maximal inhibitory concentration (pIC₅₀) on receptors.

As further advantage, the compound of formula (I) have surprisingly been found to effectively and selectively inhibit mainly the P2X₃ receptor and said compounds are useful for the treatment of respiratory disease avoiding adverse effect, such as loss of taste response. In fcat, as it can be appreciated in Table 3, the compounds of formula (I) show a greater acitivity versus the receptor P2X₃ in comparison to the receptor P2X_{2/3}.

Thus, in one aspect the present invention relates to a compound of general formula (I) as P2X₃ antagonist 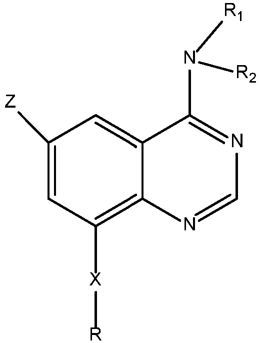 wherein
**X** is selected from S, SO₂, SO, or O;
**Z** is selected from the group consisting of heteroaryl and aryl, wherein any of such heteroaryl and aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R** is selected from the group consisting of (C₁-C₆)alkyl-, (C₁-C₆)alkyl-CN, (C₁-C₆)haloalkyl, -NR_{A}R_{B}, (C₃-C₈)heterocycloalkyl-(C₁-C₆)alkyl-, (C₃-C₈)heterocycloalkyl-, (C₃-C₈)heteroaryl-(C₁-C₆)alkyl-, (C₃-C₈)cycloalkyl-(C1-C₆)alkyl-, (C₃-C₈)cycloalkyl-, R_{A}O-(C₁-C₆)alkyl-O-(C₁-C₆)alkyl-, R_{A}NH-C(O)-(C₁-C₄)alkyl-, R_{A}R_{B}N-C(O)-(C₁-C₄)alkyl-, R_{A}O-C(O)(C₁-C₆)alkyl-,
   wherein any of such heterocycloalkyl is unsubstituted or substituted by one or more groups selected from OH, -C(O)R_{A}, -C(O)OR_{A}, (C₁-C₆)haloalkyl, -NH-C(O)R₁, and oxo; any of such cycloalkyl is substituted by one or more groups selected from -C(O)OR_{A} and (C₁-C₆)haloalkyl-,
   any of such heteroaryl may be optionally substituted by one or more groups selected from (C₁-C₆)alkyl-, -OH and (C₃-C₆)cycloalkyl-;
**R_{A}** and **R_{B}** are at each occurrence independently H or selected from the group consisting of (C₁-C₆)alkyl-, (C₁-C₆)haloalkyl-, -OR₁, -SO₂R_{C} and (C₃-C₆)cycloalkyl wherein such cycloalkyl may be optionally substituted by one or more -C(O)OR₁, or alternatively
**R_{A}** and **R_{B}** may form together with the nitrogen atom to which they are attached a 5 or 6 membered saturated heterocyclic monocyclic ring system optionally containing a further heteroatom which is nitrogen or oxygen, which may be optionally substituted by one or more groups selected from halo, -OR₁;
**R_{C}** is aryl;
with the proviso that **R** is (C₁-C₆)alkyl- or unsubstituted (C₃-C₈)heterocycloalkyl- only when X is S or SO.

In a preferred embodiment **Z** is selected from the group consisting of heteroaryl and aryl wherein the heteroaryl is selected from pyridine and pyrimidine.

In a further preferred embodiment **R₂** is heteroaryl(C₁-C₄)alkyl- wherein the hereroayl is selected from the group consisting of pyridazine, thiadiazole, pyrimidine and oxadiazole.

In a preferred embodiment, the invention refers to at least one of the compounds listed in the Table 1 below and pharmaceutical acceptable salts thereof.

**Table 1 List of preferred compounds of Formula (I)**

| **Ex. N.** | **Structure** | **Chemical Name** |
|---|---|---|
| Example 1 | 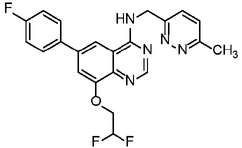 | 8-(2,2-difluoroethoxy)-6-(4-fluorophenyl)-N-((6-methylpyridazin-3-yl)methyl)quinazolin-4-amine |
| Example 2 | 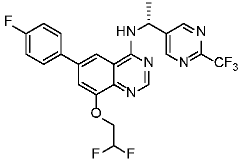 | (R)-8-(2,2-difluoroethoxy)-6-(4-fluorophenyl)-N-(1-(2-(trifluoromethyl)pyrimidin-5-yl)ethyl)quinazolin-4-amine |
| Example 4 | 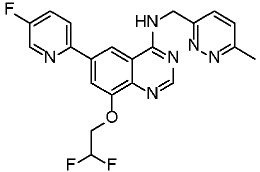 | 8-(2,2-difluoroethoxy)-6-(5-fluoropyridin-2-yl)-N-((6-methylpyridazin-3-yl)methyl)quinazolin-4-amine |
| Example 5 | 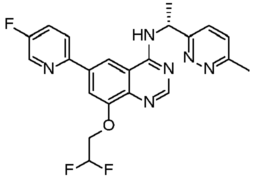 | (R)-8-(2,2-difluoroethoxy)-6-(5-fluoropyridin-2-yl)-N-(1-(6-methylpyridazin-3-yl)ethyl)quinazolin-4-amine |
| Example 6 | 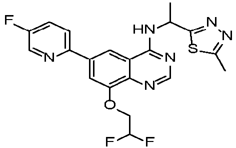 | Single enantiomer 1 of 8-(2,2-difluoroethoxy)-6-(5-fluoro-2-pyridyl)-N-[1-(5-methyl-1,3,4-thiadiazol-2-yl)ethyl]quinazolin-4-amine |
| Example 7 | 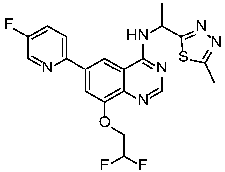 | Single enantiomer 2 of 8-(2,2-difluoroethoxy)-6-(5-fluoro-2-pyridyl)-N-[1-(5-methyl-1,3,4-thiadiazol-2-yl)ethyl]quinazolin-4-amine |
| Example 8 | 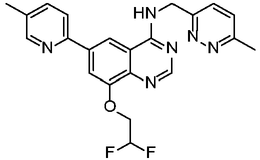 | 8-(2,2-Difluoroethoxy)-N-((6-methylpyridazin-3-yl) methyl)-6-(5-methylpyridin-2-yl) quinazolin-4-amine |
| Example 9 | 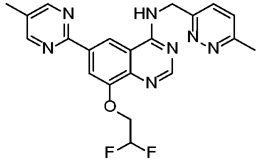 | 8-(2,2-difluoroethoxy)-N-[(6-methylpyridazin-3-yl)methyl]-6-(5-methylpyrimidin-2-yl)quinazolin-4-amine |
| Example 10 | 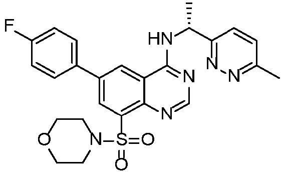 | (R)-6-(4-fluorophenyl)-N-(1-(6-methylpyridazin-3-yl)ethyl)-8-(morpholinosulfonyl)quinazolin-4-amine |
| Example 11 | 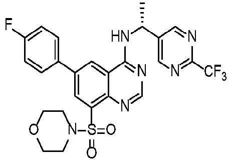 | ((*R*)-6-(4-fluorophenyl)-8-(morpholinosulfonyl)-N-(1-(2-(trifluoromethyl)pyrimidin-5-yl)ethyl)quinazolin-4-amine |
| Example 12 | 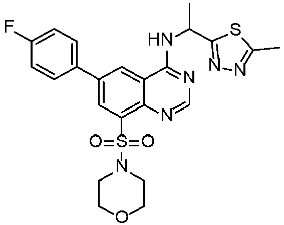 | (*Rac*)-6-(4-fluorophenyl)-N-(1-(5-methyl-1,3,4-thiadiazol-2-yl)ethyl)-8-(morpholinosulfonyl)quinazolin-4-amine |
| Example 13 | 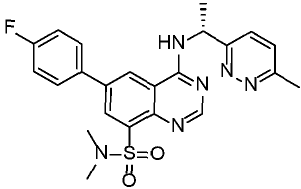 | (R)-6-(4-fluorophenyl)-N,N-dimethyl-4-((1-(6-methylpyridazin-3-yl)ethyl)amino)quinazoline-8-sulfonamide |
| Example 14 | 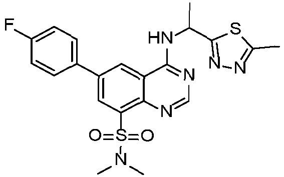 | 6-(4-fluorophenyl)-N,N-dimethyl-4-((1-(5-methyl-1,3,4-thiadiazol-2-yl)ethyl)amino)quinazoline-8-sulfonamide |
| Example 15 | 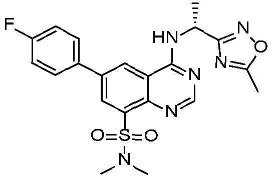 | 6-(4-fluorophenyl)-N,N-dimethyl-4-((1-(5-methyl-1,2,4-oxadiazol-3-yl)ethyl)amino)quinazoline-8-sulfonamide |
| Example 16 | 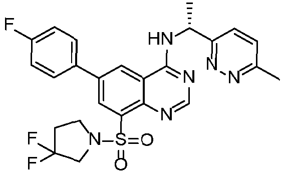 | (R)-8-((3,3-difluoropyrrolidin-1-yl)sulfonyl)-6-(4-fluorophenyl)-N-(1-(6-methylpyridazin-3-yl)ethyl)quinazolin-4-amine |
| Example 17 | 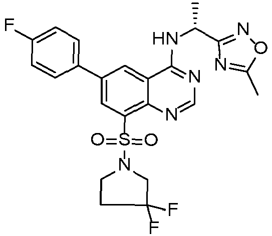 | (R)-8-((3,3-difluoropyrrolidin-1-yl)sulfonyl)-6-(4-fluorophenyl)-N-(1-(5-methyl-1,2,4-oxadiazol-3-yl)ethyl)quinazolin-4-amine |
| Example 18 | 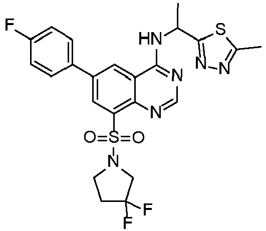 | 8-((3,3-difluoropyrrolidin-1-yl)sulfonyl)-6-(4-fluorophenyl)-N-(1-(5-methyl-1,3,4-thiadiazol-2-yl)ethyl)quinazolin-4-amine |
| Example 19 | 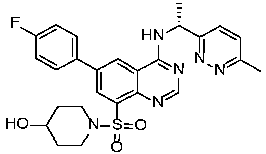 | (R)-1-((6-(4-fₗuorophenyl)-4-((1-(6-methylpyridazin-3-yl)ethyl)amino)quinazolin-8-yl)sulfonyl)piperidin-4-ol |
| Example 20 | 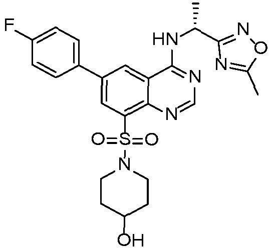 | (R)-1-((6-(4-fluorophenyl)-4-((1-(5-methyl-1,2,4-oxadiazol-3-yl)ethyl)amino)quinazolin-8-yl)sulfonyl)piperidin-4-ol |
| Example 21 | 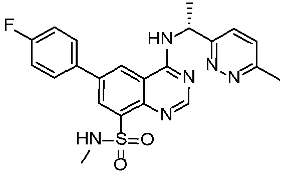 | (R)-6-(4-fluorophenyl)-N-methyl-4-((1-(6-methylpyridazin-3-yl)ethyl)amino)quinazoline-8-sulfonamide |
| Example 22 | 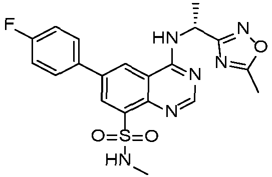 | (R)-6-(4-fluorophenyl)-N-methyl-4-((1-(5-methyl-1,2,4-oxadiazol-3-yl)ethyl)amino)quinazoline-8-sulfonamide |
| Example 23 | 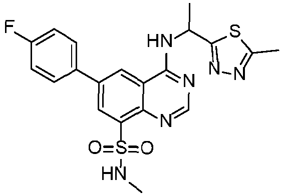 | (6-(4-fluorophenyl)-N-methyl-4-((1-(5-methyl-1,3,4-thiadiazol-2-yl)ethyl)amino)quinazoline-8-sulfonamide |
| Example 24 | 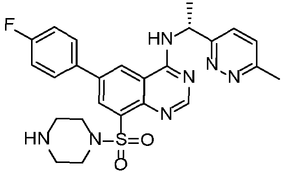 | (R)-6-(4-fluorophenyl)-N-(1-(6-methylpyridazin-3-yl)ethyl)-8-(piperazin-1-ylsulfonyl)quinazolin-4-amine |
| Example 25 | 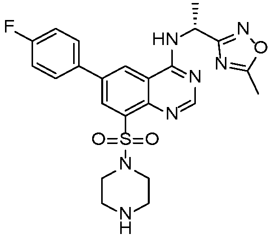 | (R)-6-(4-fluorophenyl)-N-(1-(5-methyl-1,2,4-oxadiazol-3-yl)ethyl)-8-(piperazin-1-ylsulfonyl)quinazolin-4-amine |
| Example 26 | 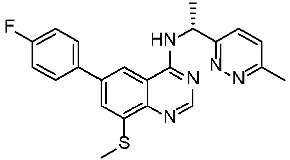 | (R)-6-(4-fluorophenyl)-N-(1-(6-methylpyridazin-3-yl)ethyl)-8-(methylthio) quinazolin-4-amine |
| Example 27 | 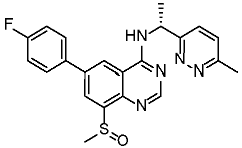 | 6-(4-fluorophenyl)-N-((R)-1-(6-methylpyridazin-3-yl)ethyl)-8-(methylsulfinyl) quinazolin-4-amine |
| Example 28 | 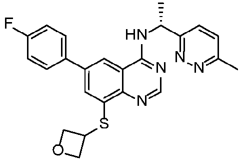 | (R)-6-(4-fluorophenyl)-N-(1-(6-methylpyridazin-3-yl)ethyl)-8-(oxetan-3-ylthio)quinazolin-4-amine |
| Example 29 | 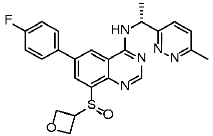 | 6-(4-fluorophenyl)-N-((R)-1-(6-methylpyridazin-3-yl)ethyl)-8-(oxetan-3-ylsulfinyl)quinazolin-4-amine |
| Example 30 | 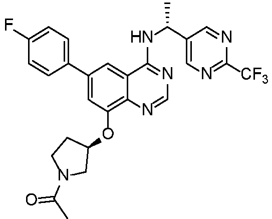 | (1-((*S*)-3-((6-(4-fluorophenyl)-4-(((*R*)-1-(2-(trifluoromethyl)pyrimidin-5-yl)ethyl)amino)quinazolin-8-yl)oxy)pyrrolidin-1-yl)ethan-1-one |
| Example 31 | 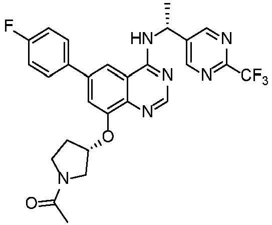 | (1-((*R*)-3-((6-(4-fluorophenyl)-4-(((*R*)-1-(2-(trifluoromethyl)pyrimidin-5-yl)ethyl)amino)quinazolin-8-yl)oxy)pyrrolidin-1-yl)ethan-1-one |
| Example 32 | 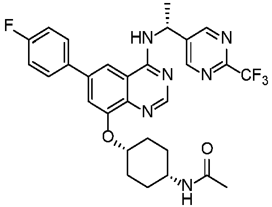 | *N-*((1*S*,4*s*)-4-((6-(4-fluorophenyl)-4-(((*R*)-1-(2-(trifluoromethyl)pyrimidin-5-yl)ethyl)amino)quinazolin-8-yl)oxy)cyclohexyl)acetamide |
| Example 33 | 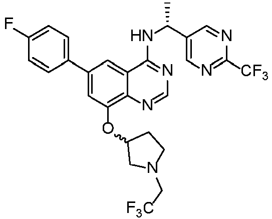 | 6-(4-fluorophenyl)-8-((1-(2,2,2-trifluoroethyl)pyrrolidin-3-yl)oxy)-*N*-((*R*)-1-(2-(trifluoromethyl)pyrimidin-5-yl)ethyl)quinazolin-4-amine |
| Example 34 | 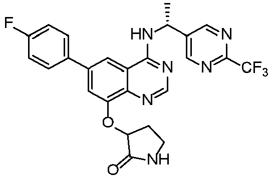 | 3-((6-(4-fluorophenyl)-4-(((*R*)-1-(2-(trifluoromethyl)pyrimidin-5-yl)ethyl)amino)quinazolin-8-yl)oxy)pyrrolidin-2-one |
| Example 35 | 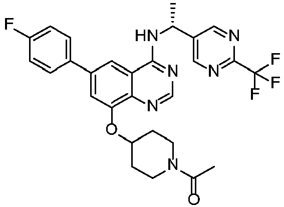 | (*R*)-1-(4-((6-(4-fluorophenyl)-4-((1-(2-(trifluoromethyl)pyrimidin-5-yl)ethyl)amino)quinazolin-8-yl)oxy)piperidin-1-yl)ethan-1-one |
| Example 36 | 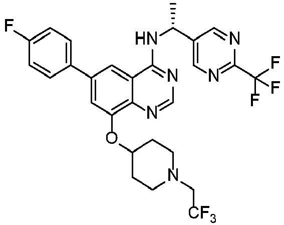 | (R)-6-(4-fluorophenyl)-8-((1-(2,2,2-trifluoroethyl)piperidin-4-yl)oxy)-N-(1-(2-(trifluoromethyl)pyrimidin-5-yl)ethyl)quinazolin-4-amine |
| Example 37 | 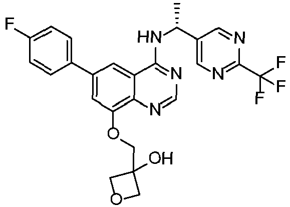 | (*R*)-3-(((6-(4-fluorophenyl)-4-((1-(2-(trifluoromethyl)pyrimidin-5-yl)ethyl)amino)quinazolin-8-yl)oxy)methyl)oxetan-3 -ol |
| Example 38 | 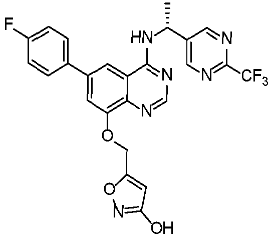 | (*R*)-5-(((6-(4-fluorophenyl)-4-((1-(2-(trifluoromethyl)pyrimidin-5-yl)ethyl)amino)quinazolin-8-yl)oxy)methyl)isoxazol-3 -ol |
| Example 39 | 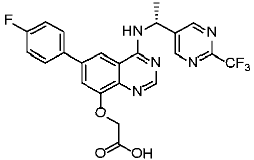 | (*R*)-2-((6-(4-fluorophenyl)-4-((1-(2-(trifluoromethyl)pyrimidin-5-yl)ethyl)amino)quinazolin-8-yl)oxy)acetic acid |
| Example 40 | 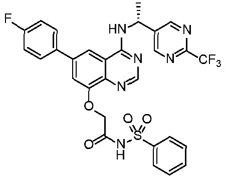 | (*R*)-2-((6-(4-fluorophenyl)-4-((1-(2-(trifluoromethyl)pyrimidin-5-yl)ethyl)amino)quinazolin-8-yl)oxy)-N-(phenylsulfonyl)acetamide |
| Example 41 | 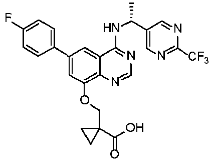 | (*R*)-1-(((6-(4-fluorophenyl)-4-((1-(2-(trifluoromethyl)pyrimidin-5-yl)ethyl)amino)quinazolin-8-yl)oxy)methyl)cyclopropane-1-carboxylic acid |
| Example 42 | 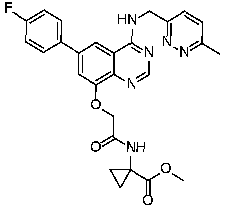 | Methyl 1-(2-((6-(4-fluorophenyl)-4-(((6-methylpyridazin-3-yl)methyl)amino)quinazolin-8-yl)oxy)acetamido)cyclopropane-1-carboxylate |
| Example 43 | 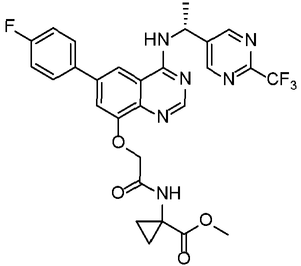 | Methyl (R)-1-(2-((6-(4-fluorophenyl)-4-((1-(2-(trifluoromethyl)pyrimidin-5-yl)ethyl)amino) quinazolin-8-yl)oxy) acetamido) cyclopropane-1-carboxylate |
| Example 44 | 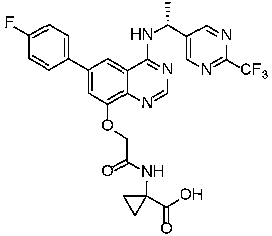 | (*R*)-1-(2-((6-(4-fluorophenyl)-4-((1-(2-(trifluoromethyl)pyrimidin-5-yl)ethyl)amino)quinazolin-8-yl)oxy)acetamido)cyclopropane-1-carboxylic acid |
| Example 45 | 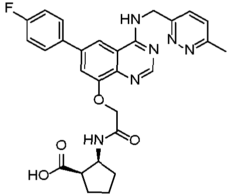 | (1*R*,2*S*)-2-(2-((6-(4-fluorophenyl)-4-(((6-methylpyridazin-3-yl)methyl)amino)quinazolin-8-yl)oxy)acetamido)cyclopentane-1-carboxylic acid |
| Example 46 | 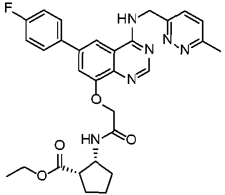 | Ethyl (1*S*,2*R*)-2-(2-((6-(4-fluorophenyl)-4-(((6-methylpyridazin-3-yl)methyl)amino)quinazolin-8-yl)oxy)acetamido)cyclopentane-1-carboxylate |
| Example 47 | 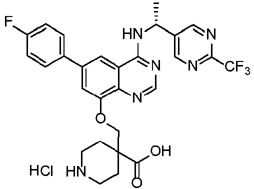 | (*R*)-4-(((6-(4-fluorophenyl)-4-((1-(2-(trifluoromethyl)pyrimidin-5-yl)ethyl)amino)quinazolin-8-yl)oxy)methyl)piperidine-4-carboxylic acid hydrochloride |
| Example 48 | 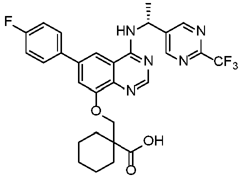 | (*R*)-1-(((6-(4-fluorophenyl)-4-((1-(2-(trifluoromethyl)pyrimidin-5-yl)ethyl)amino)quinazolin-8-yl)oxy)methyl)cyclohexane-1-carboxylic acid |
| Example 49 | 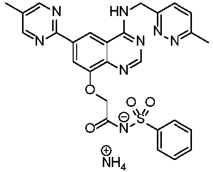 | Ammonium (2-((4-(((6-methylpyridazin-3-yl)methyl)amino)-6-(5-methylpyrimidin-2-yl)quinazolin-8-yl)oxy)acetyl)(phenylsulfonyl)am ide |
| Example 50 | 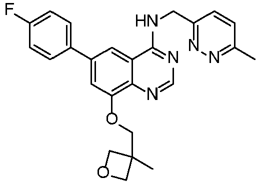 | 6-(4-fluorophenyl)-8-[(3-methyloxetan-3-yl)methoxy]-*N-*[(6-methylpyridazin-3-yl)methyl]quinazolin-4-amine |
| Example 51 | 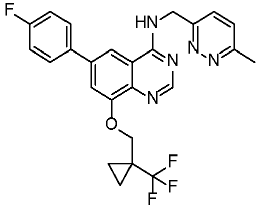 | 6-(4-fluorophenyl)-*N*-[(6-methylpyridazin-3-yl)methyl]-8-[[1-(trifluoromethyl)cyclopropyl]met hoxy]quinazolin-4-amine |
| Example 52 | 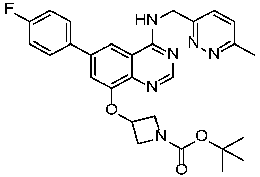 | *tert*-butyl3-[6-(4-fluorophenyl)-4-[(6-methylpyridazin-3-yl)methylamino]quinazolin-8-yl]oxyazetidine-1-carboxylate |
| Example 53 | 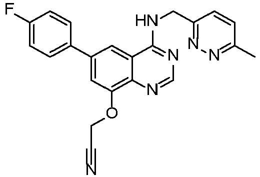 | 2-[6-(4-fluorophenyl)-4-[(6-methylpyridazin-3-yl)methylamino]quinazolin-8-yl]oxyacetonitrile |
| Example 54 | 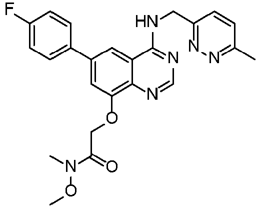 | 2-[6-(4-fluorophenyl)-4-[(6-methylpyridazin-3-yl)methylamino]quinazolin-8-yl]oxy-N-methoxy-N-methyl-acetamide |
| Example 55 | 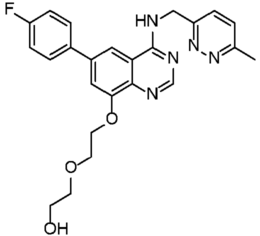 | 2-[2-[6-(4-fluorophenyl)-4-[(6-methylpyridazin-3-yl)methylamino]quinazolin-8-yl]oxyethoxy]ethanol |
| Example 56 | 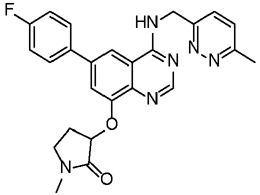 | 3-[6-(4-fluorophenyl)-4-[(6-methylpyridazin-3-yl)methylamino]quinazolin-8-yl]oxy-1-methyl-pyrrolidin-2-one |
| Example 57 | 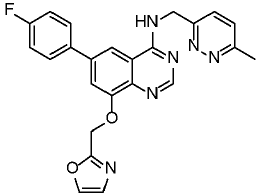 | 6-(4-fluorophenyl)-*N*-[(6-methylpyridazin-3-yl)methyl]-8-(oxazol-2-ylmethoxy)quinazolin-4-amine |
| Example 58 | 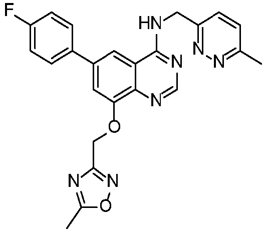 | 6-(4-fluorophenyl)-8-[(5-methyl-1,2,4-oxadiazol-3-yl)methoxy]-*N*-[(6-methylpyridazin-3-yl)methyl]quinazolin-4-amine |
| Example 59 | 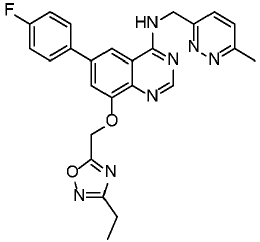 | 8-[(3-ethyl-1,2,4-oxadiazol-5-yl)methoxy]-6-(4-fluorophenyl)-*N*-[(6-methylpyridazin-3-yl)methyl]quinazolin-4-amine |
| Example 60 | 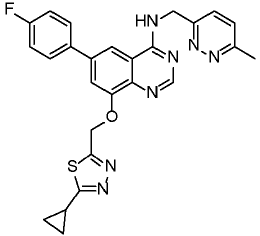 | 8-[(5-cyclopropyl-1,3,4-thiadiazol-2-yl)methoxy]-6-(4-fluorophenyl)-*N*-[(6-methylpyridazin-3-yl)methyl]quinazolin-4-amine |
| Example 61 | 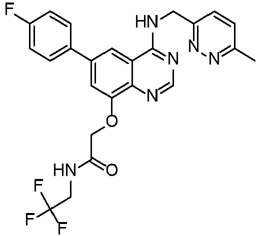 | 2-[6-(4-fluorophenyl)-4-[(6-methylpyridazin-3-yl)methylamino]quinazolin-8-yl]oxy-*N*-(2,2,2-trifluoroethyl)acetamide |
| Example 62 | 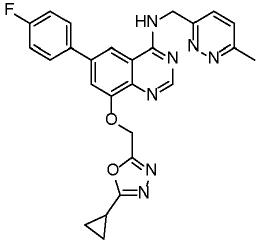 | 8-((5-cyclopropyl-1,3,4-oxadiazol-2-yl)methoxy)-6-(4-fluorophenyl)-N-((6-methylpyridazin-3-yl)methyl)quinazolin-4-amine |
| Example 63 | 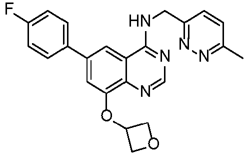 | 6-(4-fluorophenyl)-N-((6-methylpyridazin-3-yl)methyl)-8-(oxetan-3-yloxy)quinazolin-4-amine |
| Example 64 | 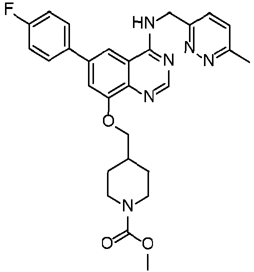 | Methyl 4-[[6-(4-fluorophenyl)-4-[(6-methylpyridazin-3-yl)methylamino]quinazolin-8-yl]oxymethyl]piperidine-1-carboxylate |
| Example 65 | 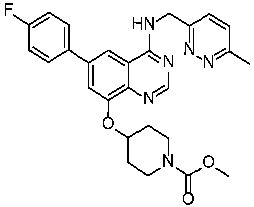 | Methyl 4-[6-(4-fluorophenyl)-4-[(6-methylpyridazin-3-yl)methylamino]quinazolin-8-yl]oxypiperidine-1-carboxylate |
| Example 66 | 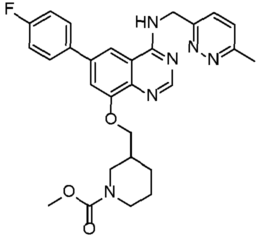 | Methyl 3-[[6-(4-fluorophenyl)-4-[(6-methylpyridazin-3-yl)methylamino]quinazolin-8-yl]oxymethyl]piperidine-1-carboxylate |
| Example 67 | 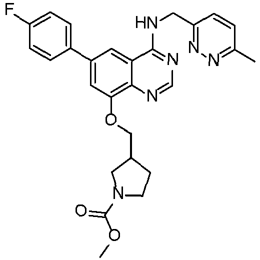 | Methyl 3-[[6-(4-fluorophenyl)-4-[(6-methylpyridazin-3-yl)methylamino]quinazolin-8-yl]oxymethyl]pyrrolidine-1-carboxylate |
| Example 68 | 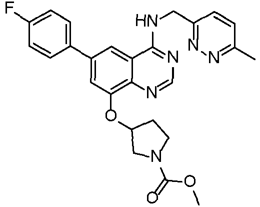 | Methyl 3-[6-(4-fluorophenyl)-4-[(6-methylpyridazin-3-yl)methylamino]quinazolin-8-yl]oxypyrrolidine-1-carboxylate |

In one preferred embodiment, the invention refers to a compound of formula (I), 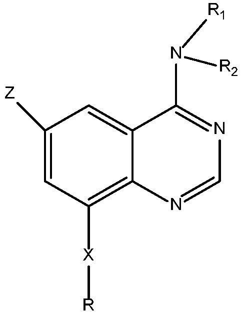 wherein
**X** is selected from S or SO;
**Z** is aryl, wherein such aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R** is selected from the group consisting of (C₁-C₆)alkyl- and (C₃-C₈)heterocycloalkyl-.

In one preferred embodiment, the invention refers to a compound of formula (I), wherein X is O, represented by the formula Ia 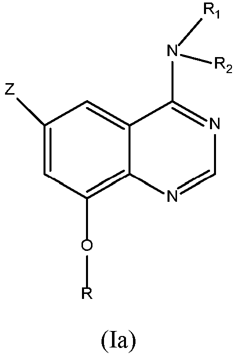 wherein
**Z** is selected from the group consisting of heteroaryl, aryl, wherein any of such heteroaryl and aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R** is selected from the group consisting of (C₁-C₆)alkyl-CN, (C₁-C₆)haloalkyl, -NR_{A}R_{B}, (C₃-C₈)heterocycloalkyl-(C₁-C₆)alkyl-, (C₃-C₈)heterocycloalkyl-, (C₃-C₈)heteroaryl-(C₁-C₆)alkyl-, (C₃-C₈)cycloalkyl-(C1-C₆)alkyl-, (C₃-C₈)cycloalkyl-, R_{A}O-(C₁-C₆)alkyl-O-(C₁-C₆)alkyl-, R_{A}NH-C(O)-(C₁-C₄)alkyl-, R_{A}R_{B}N-C(O)-(C₁-C₄)alkyl-, R_{A}O-C(O)(C₁-C₆)alkyl-,
   wherein any of such heterocycloalkyl is substituted by one or more groups selected from -OH, -C(O)R_{A}, -C(O)OR_{A}, (C₁-C₆)haloalkyl, -NH-C(O)R₁, and oxo;
   any of such cycloalkyl is substituted by one or more groups selected from -C(O)OR_{A} and (C₁-C₆)haloalkyl-,
   any of such heteroaryl may be optionally substituted by one or more groups selected from (C₁-C₆)alkyl-, -OH and (C₃-C₆)cycloalkyl-;
**R_{A}** and **R_{B}** are at each occurrence independently H or selected from the group consisting of (C₁-C₆)alkyl-, (C₁-C₆)haloalkyl-, -OR₁, -SO₂R_{C} and (C₃-C₆)cycloalkyl wherein such cycloalkyl may be optionally substituted by one or more -C(O)OR₁;
**R_{C}** is aryl.

In one preferred embodiment, the invention refers to a compound of formula (I), wherein X is SO₂, represented by the formula Ib 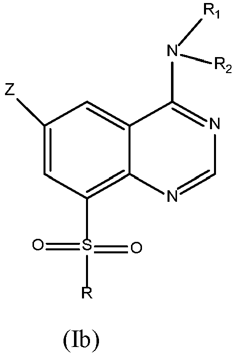 wherein
**Z** is aryl, wherein any of such aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R is** -NR_{A}R_{B};
**R_{A}** and **R_{B}** are at each occurrence independently H or (C₁-C₆)alkyl-, or alternatively
**R_{A}** and **R_{B}** may form together with the nitrogen atom to which they are attached a 5 or 6 membered saturated heterocyclic monocyclic ring system optionally containing a further heteroatom which is nitrogen or oxygen, which may be optionally substituted by one or more groups selected from halo, -OR₁.

The compounds of formula (I) including all the compounds or at least one of the here above listed can be generally prepared according to the procedure outlined in detail in the Schemes shown below using generally known methods.

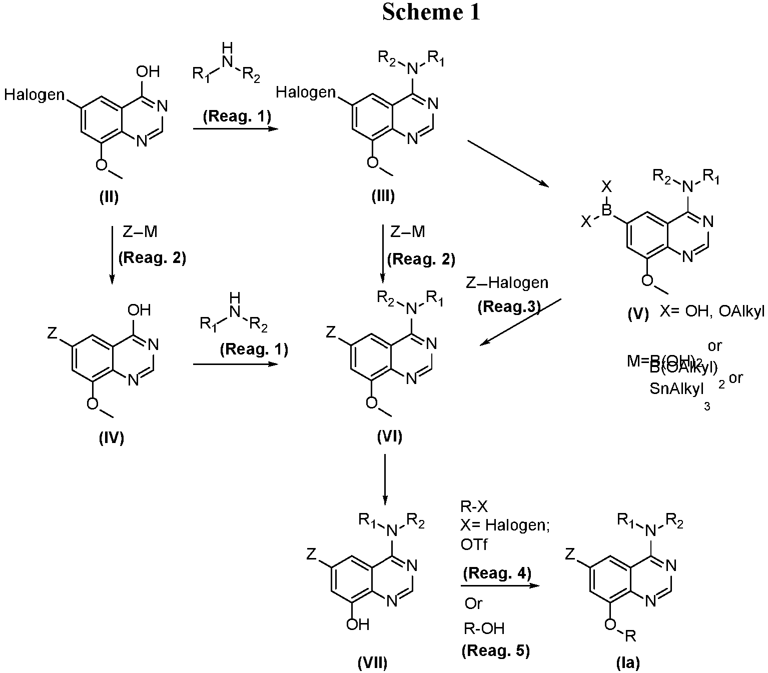

In one embodiment of the present invention, compound (Ia) may be prepared according to SCHEME 1 from compound (II). Compound (II) was prepared following the procedure described in J.Med Chem., 2015, 58 (8), 3548-3571.

Compound (III) may be prepared from Compound (II) by a deoxyamination reaction mediated by coupling reagents like PyBOP with a suitable amine (Reag.1).

Compound (VI) may be prepared from Compound (III) by a metal-catalyzed cross coupling reactions like Stille or Suzuki or similars as described in "Transition Metals for Organic Synthesis", 2nd Ed, 1, 2004 with a suitable reagent like (Reag. 2).

Alternatevely Compound (V) may be prepared from Compound (III) by metal-catalyzed Miyaura borylation reaction.

Compound (VI) may be prepared from Compound (V) by a metal-catalyzed cross coupling reactions like Stille, Suzuki or similar as described in "Transition Metals for Organic Synthesis", 2nd Ed, 1, 2004 with a suitable organohalogen compound like (Reag. 3).

In another embodiment, compound (IV) was prepared starting from compound (II) by a metal-catalyzed cross coupling reactions like Stille, Suzuki or similars as described in "Transition Metals for Organic Synthesis", 2nd Ed, 1, 2004 with a suitable Organometallic reagent like (Reag.2).

Compound (VI) may be prepared from Compound (IV) by deoxyamination reaction mediated by reagents like PyBOP or similar with a suitable amine (Reag.1).

Compound (VII) may be prepared from Compound (VI) by means of dealkylation reactions mediated by strong Lewis acids like BBr₃ or similar.

Compounds (Ia) were prepared from compounds (VII) by alkylation with a suitable alkylating agent (Reag. 4) like, alkyl chlorides, bromides, iodides, mesylates, tosylates or similar.

Alternatively, compounds (Ia) may be prepared from compounds (VII) and a suitable alcohol (Reag. 5) by Mitsunobu-like reactions mediated, for example, by DEAD/ PPh₃, DIAD/PPh₃ or CMT.

Some compounds (Ia) may contain a protected hydroxyl or amino group which were then removed under well-known procedures.

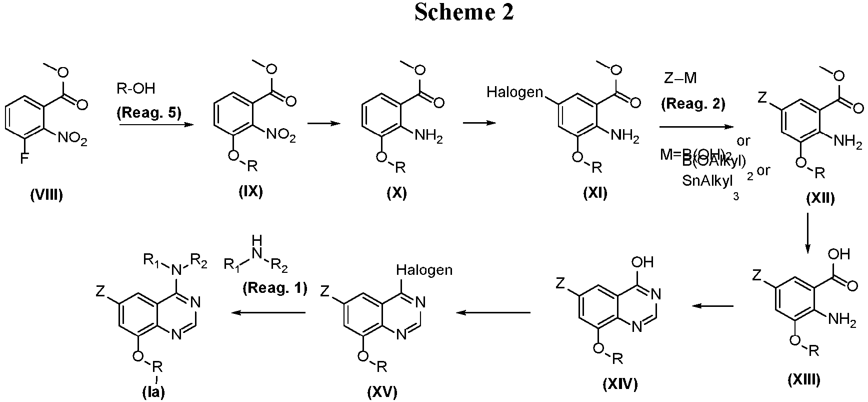

In one embodiment of the present invention, compound (Ia) may be prepared according to SCHEME 2 from compound (VIII).

Compounds (IX) may be prepared from compounds (VIII) and a suitable alcohol (Reag. 5) by aromatic substitution in the presence of an appropriate base like, for example, Potassium Carbonate (K₂CO₃).

Compounds (X) may be prepared from compounds (IX) by hydrogenation in the presence of a suitable catalyst like, for example, Palladium on carbon.

Compound (XI) may be prepared from Compound (X) by Halogention with suitable reagents like Bromine, NBS, NIS, Iodine, iodonium salts or similar.

Compound (XII) may be prepared from Compound (XI) by metal-catalyzed cross coupling reactions like Stille, Suzuki or similar ones with a suitable organometallic reagents (Reag. 2) like, for example, organoboron compounds.

Compound (XIII) may be prepared from Compound (XII) by ester hydrolysis mediated by a suitable base like, for example, lithium hydroxide (LiOH).

Compound (XIV) may be prepared from Compound (XIII) by quinazoline ring construction reaction with a suitable reagents like formamide or similars.

Compound (XV) may be prepared from Compound (XIV) by a deoxyahalogenation reaction mediated by reagents like, for example, Phosphorous oxychloride.

Compound (Ia) may be prepared from Compound (XV) by a reaction with a suitable amine (Reag.1) in the presence of a base like, for example TEA or DIPEA

Some compounds (Ia) may contain a protected hydroxyl or amino group which were then removed under well known procedures.

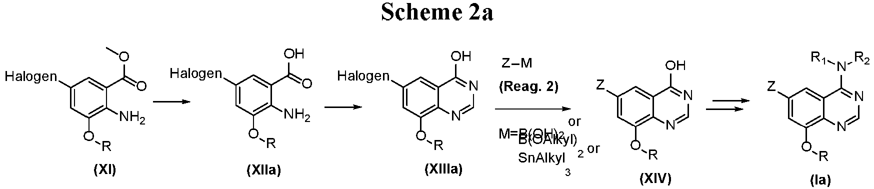

In another embodiment of the present invention, compound (Ia) may also be prepared according to SCHEME 2a from compound (XI).

Compound (XIIa) may be prepared from Compound (XI) by ester hydrolysis mediated by a suitable base like, for example, lithium hydroxide (LiOH).

Compound (XIIIa) may be prepared from Compound (XIIa) by quinazoline ring construction reaction with a suitable reagents like formamide or similars.

Compound (XIV) may be prepared from Compound (XIIIa) by metal-catalyzed cross coupling reactions like Stille, Suzuki or similar ones with a suitable organometallic reagents (Reag. 2) like, for example, organoboron compounds.

Compound (Ia) may be prepared from Compound (XIV) as already described in SCHEME 2.

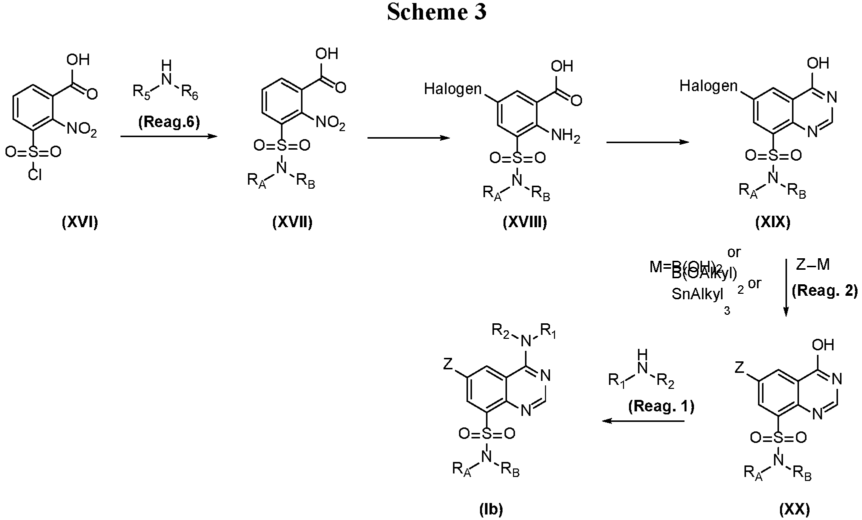

In another embodiment of the present invention, compound (Ib) may be prepared according to SCHEME 3 from compounds (XVI).

Compound (XVII) may be prepared from Compound (XVI) by amination reaction in the presence of a suitable amine (Reag. 6).

Compound (XVIII) may be prepared from Compound (XVII) by Halogention with suitable reagents like Bromine, NBS, NIS, Iodine, iodonium salts or similar

Compound (XIX) may be prepared from Compound (XVIII) by quinazoline ring construction reaction with a suitable reagents like formamide or similars.

Compound (XX) may be prepared from Compound (XIX) by metal-catalyzed cross coupling reactions like Stille, Suzuki or similar ones with a suitable organometallic reagents (Reag. 2) like, for example, organoboron compounds.

Compound (Ib) may be prepared from Compound (XX) by amination reaction in the presence of a suitable amine (Reag. 1).

Some compounds (Ib) may contain a protected hydroxyl or amino group which were then removed under well known procedures.

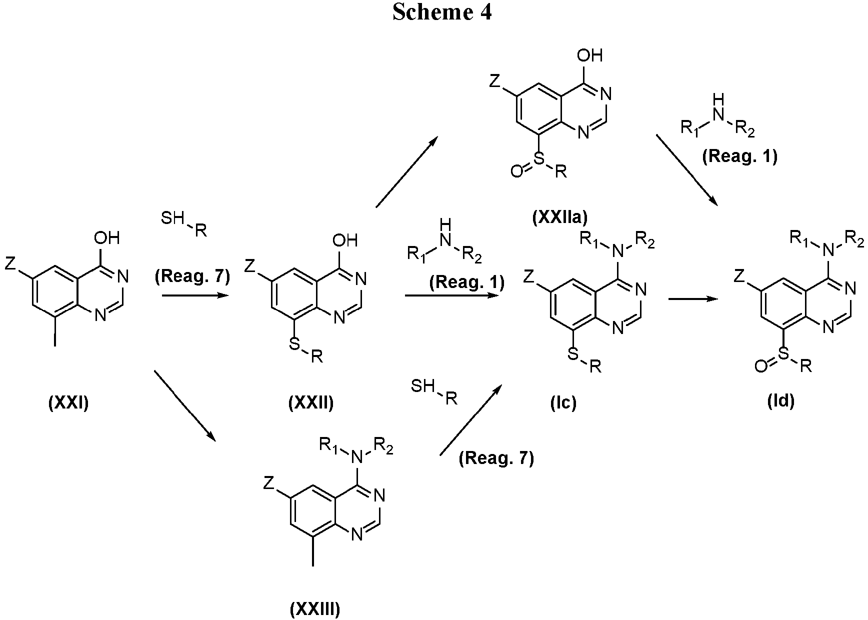

In another embodiment of the present invention, compound (Ic) and (Id) may be prepared according to SCHEME 4 from compounds (XXI).

Compound (XXII) may be prepared from Compound (XXI) by metal-catalyzed thiolation with suitable organosulfur reagents (Reag. 7).

Compound (Ic) may be prepared from Compound (XXII) by a deoxyamination reaction mediated by coupling reagents like PyBOP with a suitable amine (Reag.1).

Alternatevely Compound (XXIII) may be prepared from Compound (XXI) by a deoxyamination reaction mediated by coupling reagents like PyBOP with a suitable amine (Reag.1).

Compound (Ic) may be prepared from Compound (XXIII) by metal-catalyzed thiolation with suitable organosulfur reagents (Reag. 7).

Compound (Id) may be prepared from Compound (Ic) by oxidation reaction with reagents like, for example, mCPBA.

Alternatevely Compound (XXIIa) may be then prepared from Compound (XXII) by oxidation reaction with reagents like, for example, mCPBA.

Compound (Id) may be prepared from Compound (XXIIa) by a deoxyamination reaction mediated by coupling reagents like PyBOP with a suitable amine (Reag.1).

Compound (Id) may be prepared from Compound (XXIIa) by a deoxyamination reaction mediated by coupling reagents like PyBOP with a suitable amine (Reag.1).

Some compounds (Ic) may contain a protected hydroxyl or amino group which were then removed under well known procedures.

The compounds of the present invention have surprisingly been found to effectively inhibit P2X₃ receptor and said compounds are useful for the treatment of respiratory disease.

In one embodiment, representative compounds of formula (I) of the present invention have surprisingly been found to effectively and selectively inhibit P2X₃ receptor and said compounds are useful for the treatment of respiratory disease avoiding adverse effect, such as loss of taste response.

In a preferred embodiment, the compounds of formula (I) are selective P2X₃ antagonist wherein the selective P2X₃ antagonist is at least 10-fold selective for P2X₃ homomeric receptor antagonism versus P2X_{2/3} heteromeric receptor antagonism.

In a further preferred embodiment, the selective P2X₃ antagonist is at least 30-fold selective for P2X₃ homomeric receptor antagonism versus P2X_{2/3} heteromeric receptor antagonism.

In a further preferred embodiment, the selective P2X₃ antagonist is at least 50-fold selective for P2X₃ homomeric receptor antagonism versus P2X_{2/3} heteromeric receptor antagonism.

The present invention also provides a pharmaceutical composition comprising a compound of formula (I) or a pharmaceutically acceptable salt thereof in admixture with one or more pharmaceutically acceptable carrier or excipient, either alone or in combination with one or more further active ingredient.

In one aspect, the invention refers to a compound of formula (I) according to the invention for use as a medicament.

In a further aspect, the invention refers to the use of a compound of formula (I) of the invention, or a pharmaceutically acceptable salt thereof, in the manufacture of a medicament for the treatment of disorders associated with P2X₃ receptors mechanism, preferably for the treatment of respiratory diseases.

Preferably, the invention refers to a compound of formula (I) for use in the prevention and /or treatment of respiratory diseases, preferably cough, sub-acute or chronic cough, treatment-resistant cough, idiopathic chronic cough, post-viral cough, iatrogenic cough, asthma, idiopathic pulmonary fibrosis (IPF), chronic obstructive pulmonary disease (COPD) and cough associated with respiratory diseases such as COPD, asthma and bronchospasm.

More preferably, the invention refers to a compound of formula (I) for use in the prevention and /or treatment of chronic cough and cough associated with respiratory diseases such as COPD, asthma and bronchospasm.

The invention also provides a compound of formula (I) for use in a method for the prevention and/or treatment of disorders associated with P2X₃ receptors mechanisms, said method comprising administering to a patient in need of such treatment a therapeutically effective amount of a compound of the invention.

In particular the invention refers to a compound of formula (I) for use in a method for the prevention and/or treatment wherein the disorder is cough, sub-acute or chronic cough, treatment-resistant cough, idiopathic chronic cough, post-viral cough, iatrogenic cough, asthma, idiopathic pulmonary fibrosis (IPF), chronic obstructive pulmonary disease (COPD) and cough associated with respiratory diseases such as COPD, asthma and bronchospasm, wherein said method comprises the administration of a proper amount of a compound of formula (I) to a patient in the need thereof.

In a further preferred embodiment, the disorder is chronic cough.

The methods of treatment referred to in the description comprise administering a safe and effective amount of a compound of formula (I) or a pharmaceutically acceptable salt thereof to a patient in need thereof. As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan. The compounds of formula (I) or pharmaceutically acceptable salts thereof may be administered once or according to a dosing regimen wherein a number of doses are administered at varying intervals of time for a given period of time. Typical daily dosages may vary depending upon the particular route of administration chosen.

The invention also provides pharmaceutical compositions of compounds of formula (I) in admixture with one or more pharmaceutically acceptable carrier or excipient, for example those described in Remington's Pharmaceutical Sciences Handbook, XVII Ed., Mack Pub., N.Y., U.S.A.

Administration of the compounds of the invention and their pharmaceutical compositions may be accomplished according to patient needs, for example, orally, nasally, parenterally (subcutaneously, intravenously, intramuscularly, intrasternally and by infusion) and by inhalation.

Preferably the compounds of the present invention may be administered orally or by inhalation. More preferably the compounds of the present invention are administered orally.

Various solid oral dosage forms can be used for administering compounds of the invention including such solid forms as tablets, gelcaps, capsules, caplets, granules, lozenges and bulk powders. The compounds of the invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like. Time release capsules, tablets and gels are also advantageous in administering the compounds of the invention.

Preferably the compounds of the invention are administered in forms of tablets.

Various liquid oral dosage forms can also be used for administering compounds of the invention, including aqueous and non-aqueous solutions, emulsions, suspensions, syrups, and elixirs. Such dosage forms can also contain suitable known inert diluents such as water and suitable known excipients such as preservatives, wetting agents, sweeteners, flavorants, as well as agents for emulsifying and/or suspending the compounds of the invention. The compounds of the invention may be injected, for example, intravenously, in the form of an isotonic sterile solution.

For the treatment of the diseases of the respiratory tract, the compounds according to the invention are preferably administered by inhalation.

Inhalable preparations include inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in form of solutions or suspensions in an aqueous, alcoholic or hydroalcoholic medium and they may be delivered by jet or ultrasonic nebulizers known from the prior art or by soft-mist nebulizers.

Preferably, the compound of the present invention is administered orally.

The compounds of the invention can be administered as the sole active agent or in combination with other pharmaceutical active ingredients.

Preferably, the compound of the present invention can be combined with therapeutic agents or active ingredients useful for the treatment of disease which are related to or mediated by P2X₃ receptor.

The dosages of the compounds of the invention depend upon a variety of factors including among others the particular disease to be treated, the severity of the symptoms, the route of administration, and the like.

The invention is also directed to a device comprising a pharmaceutical composition comprising a compound of formula (I) according to the invention, in form of a single- or multi-dose dry powder inhaler or a metered dose inhaler.

The various aspects of the invention described in this application are illustrated buy the following examples which are not meant to limit the invention in any way. following examples illustrate the invention.

The example testing experiments described herein serve to illustrate the present invention and the invention is not limited to the examples given.

### PREPARATIONS OF INTERMEDIATES AND EXAMPLES

Chemical names were generated using the Dotmatics software. In some cases generally accepted names of commercially available reagents were used in place of Dotmatics software generated names.

All reagents, for which the synthesis is not described in the experimental part, are either commercially available, or are known compounds or may be formed from known compounds by known methods by a person skilled in the art.

(*R*)-1-(2-(trifluoromethyl)pyrimidin-5-yl)ethanamine HCl, (*R*)*-1-(6-*methylpyridazin-3-yl)ethan-1-amine HCl were prepared accordingly to the procedure descibed in WO2016/091776.

### ABBREVIATION - MEANING

Et₂O: diethyl ether;
Et₃N: triethyl amine;
TEA: triethyl amine;
DCC: N,N'-Dicyclohexylcarbodiimide;
PyBOP: (benzotriazol-1-yloxy)tripyrrolidinophosphonium hexafluorophosphate;
DMF: dimethylformamide;
EtOAc: Ethyl acetate;
RT: room temperature;
THF: tetrahydrofuran;
DCM: dichloromethane;
MeOH: methyl alcohol;
EtOH: ethylic alcohol;
TFA: Trifluoroacetic acid;
LC-MS: Liquid Chromatography/Mass Spectrometry;
HPLC: high pressure liquid chromatography;
MPLC: medium pressure liquid chromatography;
SFC: Supercritical Fluid Chromatography;
dppf: 1,1'- Bis( diphenylphosphino) ferrocene;
DIEA or DIPEA: N,N-Diisopropylethylamine;
MeCN: Acetonitrile;
MTBE: tert-Butyl methyl ether;
TBDMSCl: tert-Butyl(chloro)dimethylsilane;
DMSO: Dimethylsulfoxide;
Boc₂O: di-tert-butyl dicarbonate;
UPLC: Ultra Performance Liquid Chromatography.
mCPBA: m-Chloroperbenzoic acid

### General Experimental Details and methods

### Analytical Methods

### Liquid Chromatography-Mass Spectrometry

### Method 1

UPLC-MS was performed on a Waters Acquity I-Class with Waters Diode Array Detector coupled to a Waters SQD2 single quadrapole mass spectrometer using an Waters HSS C18 column (1.8 µm, 100 × 2.1 mm) being initially held at 5% acetonitrile/water (with 0.1% formic acid in each mobile phase) for 1.2 minutes, followed by a linear gradient of 5-100% within 3.5 minutes and then held at 100% for 1.5 minutes (F = 0.5 mL/min).

### Method 2

UPLC-MS was performed on a Waters Acquity I-Class with Waters Diode Array Detector coupled to a Waters SQD2 single quadrapole mass spectrometer using an Waters BEH Shield RP18 column (1.7 µm, 100 × 2.1 mm) being initially held at 5% acetonitrile/water (with 10 mM ammonium bicarbonate in each mobile phase) for 1.2 minutes, followed by a linear gradient of 5-100% within 3.5 minutes and then held at 100% for 1.5 minutes (F = 0.5 mL/min).

### Method 3

UPLC-MS was performed on a Waters DAD + Waters SQD2, single quadrapole UPLC-MS spectrometer using an Acquity UPLC BEH Shield RP18 1.7um 100 x 2.1mm (Plus guard cartridge), maintained at temp column being initially held at 5% acetonitrile/water (with 10 mM ammonium bicarbonate in each mobile phase) for 0.4 minutes, followed by a linear gradient of 5-95% within 6.4 minutes and then held at 95% for 1.2 minutes (F = 0.4 mL/min).

### Method 4

UPLC-MS was performed on a Waters DAD + Waters SQD2, single quadrapole UPLC-MS spectrometer using an Acquity UPLC BEH Shield RP18 1.7um 100 x 2.1mm (Plus guard cartridge), maintained at temp column being initially held at 5% Acetonitrile (Far UV grade) with 0.1% (V/V) formic acid / Water (High purity via PureLab Option unit) with 0.1% formic acid for 0.4 minutes, followed by a linear gradient of 5-95% within 6.4 minutes and then held at 95% for 1.2 minutes (F = 0.4 mL/min).

### Method 5

Aquity UPLC - QDa Mass Spectrometer with a C18-reverse-phase column (50 × 2.1 mm Acquity CSH with 1.7 µm particle size) maintained at 40 °C, elution with A: 95/5 water/acetonitrile + 0.05% formic acid; B: 95/5 acetonitrile/water + 0.05% formic acid.

### Gradient:

| Gradient - Time | flow (mL/min) | %A | %B |
|---|---|---|---|
| 0.00 | 1 | 99.0 | 1.0 |
| 1.50 | 1 | 0.1 | 99.9 |
| 1.90 | 1 | 0.1 | 99.9 |
| 2.00 | 1 | 99.0 | 1.0 |

Detection-MS, UV PDA
MS ionisation method-Electrospray (positive/negative ion).

### Method 5A

Aquity UPLC - QDa Mass Spectrometer with a C18-reverse-phase column (50 × 2.1 mm Acquity CSH with 1.7 µm particle size) maintained at 40 °C, elution with A: 95/5 water/acetonitrile + 0.05% formic acid; B: 95/5 acetonitrile/water + 0.05% formic acid.

### Gradient:

| Gradient - Time | flow (mL/min) | %A | %B |
|---|---|---|---|
| 0.00 | 1 | 99.0 | 1.0 |
| 3.50 | 1 | 0.1 | 99.9 |
| 3.90 | 1 | 0.1 | 99.9 |
| 4.00 | 1 | 99.0 | 1.0 |

Detection-MS, UV PDA
MS ionisation method-Electrospray (positive/negative ion).

### Method 6

Aquity UPLC - QDa Mass Spectrometer with a C18-reverse-phase column (50 × 2.1 mm Acquity BEH with 1.7 µm particle size) maintained at 40 °C, elution with A: 95/5 water/acetonitrile + 0.05% conc. ammonia; B: 95/5 acetonitrile/water + 0.05% conc. ammonia.

### Gradient:

| Gradient - Time | flow (mL/min) | %A | %B |
|---|---|---|---|
| 0.00 | 1 | 99.0 | 1.0 |
| 1.50 | 1 | 0.1 | 99.9 |
| 1.90 | 1 | 0.1 | 99.9 |
| 2.00 | 1 | 99.0 | 1.0 |

Detection-MS, UV PDA
MS ionisation method-Electrospray (positive/negative ion)

### Method 7

Dionex UHPLC Ultimate 3000 with DAD detector/Thermo Scientific MSQ Pluse quipped with a Kinetex^{®} 2.6 µm XB-C18 (4.6x50mm), 110A maintained at 25 °C, elution with A: 0.1 % v/v water solution of formic acid, B: 0.1 % v/v acetonitrile solution of formic acid

### Gradient:

| Time [min] | Flow [ml/min] | Mobile phase A [%] | Mobile phase B [%] |
|---|---|---|---|
| 0.0 | 1.0 | 95 | 5 |
| 1.0 | 1.0 | 95 | 5 |
| 4.75 | 1.0 | 20 | 80 |
| 5.25 | 1.0 | 20 | 80 |
| 6.0 | 1.0 | 95 | 5 |
| 7.0 | 1.0 | 95 | 5 |

Detection-MS, UV PDA
MS ionisation method-Electrospray (positive/negative ion)

### NMR

¹H Nuclear magnetic resonance (NMR) spectroscopy was carried out using a Bruker or Varian instruments operating at 300 or 400 MHz using the stated solvent at around room temperature unless otherwise stated. In all cases, NMR data were consistent with the proposed structures. Characteristic chemical shifts (δ) are given in parts-per-million using conventional abbreviations for designation of major peaks: e.g. s, singlet; d, doublet; t, triplet; q, quartet; dd, doublet of doublets; dt, doublet of triplets; m, multiplet; br, broad.

### Preparative reverse-phase HPLC conditions

Preparative HPLC purification was performed by reverse phase HPLC using a Waters Fractionlynx preparative HPLC system (2525 pump, 2996/2998 UV/VIS detector, 2767 liquid handler) or an equivalent HPLC system such as a Gilson Trilution UV directed system. The Waters 2767 liquid handler acted as both auto-sampler and fraction collector. The columns used for the preparative purification of the compounds were a Waters Sunfire OBD Phenomenex Luna Phenyl Hexyl or Waters Xbridge Phenyl at 10 µm 19 × 150 mm or Waters CSH Phenyl Hexyl, 19 × 150, 5 µm column. Appropriate focused gradients were selected based on acetonitrile and methanol solvent systems under either acidic or basic conditions. The modifiers used under acidic/basic conditions were formic acid or trifluoroacetic acid (0.1% V/V) and ammonium bicarbonate (10 mM) respectively. The purification was controlled by Waters Fractionlynx software through monitoring at 210-400 nm and triggered a threshold collection value at 260 nm and, when using the Fractionlynx, the presence of target molecular ion as observed under API conditions. Collected fractions were analysed by LCMS (Waters Acquity systems with Waters SQD).

### Chiral Supercritical Fluid Chromatography (SFC) separation protocol

The diastereomeric separation of compounds was achieved by Supercritical Fluid Chromatography (SFC) using a Waters Thar Prep 100 preparative SFC system (P200 CO2 pump, 2545 modifier pump, 2998 UV/VIS detector, 2767 liquid handler with Stacked Injection Module). The Waters 2767 liquid handler acted as both auto-sampler and fraction collector. Appropriate isocratic methods were selected based on methanol, ethanol or isopropanol solvent systems under un-modified or basic conditions. The standard SFC method used was modifier, CO2, 100 mL/min, 120 Bar backpressure, 40 °C column temperature. The modifier used under basic conditions was diethylamine (0.1% V/V). The modifier used under acidic conditions was either formic acid (0.1% V/V) or trifluoroacetic acid (0.1% V/V). The SFC purification was controlled by Waters Fractionlynx software through monitoring at 210-400 nm and triggered at a threshold collection value, typically 260 nm. Collected fractions were analysed by SFC (Waters/Thar SFC systems with Waters SQD). The fractions that contained the desired product were concentrated by vacuum centrifugation.

### Supercritical Fluid Chromatography - Mass Spectrometry analytical conditions

### Method 8

SFC-MS was performed on a Waters/Thar SFC systems with Waters SQD using a Lux Cellulose-3 column with a 15% methyl alcohol/CO₂ (with 0.1% diethylamine) isocratic run at 5 mL/min, 120 Bar backpressure, 40 °C column temperature.

### Method 9

SFC-MS was performed on a Waters/Thar SFC systems with Waters SQD using a Lux Cellulose-3 column with a 20% methyl alcohol/CO₂ (with 0.1% diethylamine) isocratic run at 5 mL/min, 120 Bar backpressure, 40 °C column temperature.

### Method 10

SFC-MS was performed on a Waters/Thar SFC systems with Waters SQD using a Lux Cellulose-4 column with a 55% ethyl alcohol/CO₂ (with 0.1% diethylamine) isocratic run at 5 mL/min, 120 Bar backpressure, 40 °C column temperature.

### Method 11

SFC-MS was performed on a Waters/Thar SFC systems with Waters SQD using a Lux Cellulose-4 column with a 20% iso-propyl alcohol/CO₂ (with 0.1% diethylamine) isocratic run at 5 mL/min, 120 Bar backpressure, 40 °C column temperature.

### Method 12

SFC-MS was performed on a Waters/Thar SFC systems with Waters SQD using a Lux Cellulose-4 column with a 30% iso-propyl alcohol/CO₂ (with 0.1% diethylamine) isocratic run at 5 mL/min, 120 Bar backpressure, 40 °C column temperature.

### Method 13

SFC-MS was performed on a Waters/Thar SFC systems with Waters SQD using a Lux Cellulose-4 column with a 50% iso-propyl alcohol/CO₂ (with 0.1% diethylamine) isocratic run at 5 mL/min, 120 Bar backpressure, 40 °C column temperature.

### Method 14

SFC-MS was performed on a Waters/Thar SFC systems with Waters SQD using a Lux Cellulose-4 column with a 25% methyl alcohol/CO₂ (with 0.1% diethylamine) isocratic run at 5 mL/min, 120 Bar backpressure, 40 °C column temperature.

### Method 15

SFC-MS was performed on a Waters/Thar SFC systems with Waters SQD using a YMC Amylose-C column with a 15% ethyl alcohol/CO₂ (with 0.1% diethylamine) isocratic run at 5 mL/min, 120 Bar backpressure, 40 °C column temperature.

### Method 16

SFC-MS was performed on a Waters/Thar SFC systems with Waters SQD using a YMC Amylose-C column with a 25% iso-propyl alcohol/CO₂ (with 0.1% diethylamine) isocratic run at 5 mL/min, 120 Bar backpressure, 40 °C column temperature.

### Method 17

SFC-MS was performed on a Waters/Thar SFC systems with Waters SQD using a YMC Amylose-C column with a 35% iso-propyl alcohol/CO₂ (with 0.1% diethylamine) isocratic run at 5 mL/min, 120 Bar backpressure, 40 °C column temperature.

### Method 18

SFC-MS was performed on a Waters/Thar SFC systems with Waters SQD using a YMC Amylose-C column with a 55% iso-propyl alcohol/CO₂ (with 0.1% diethylamine) isocratic run at 5 mL/min, 120 Bar backpressure, 40 °C column temperature.

### Method 19

SFC-MS was performed on a Waters/Thar SFC systems with Waters SQD using a YMC Amylose-C column with a 15% methyl alcohol/CO₂ (with 0.1% diethylamine) isocratic run at 5 mL/min, 120 Bar backpressure, 40 °C column temperature.

### Method 20

SFC-MS was performed on a Waters/Thar SFC systems with Waters SQD using a YMC Amylose-C column with a 20% methyl alcohol/CO₂ (with 0.1% diethylamine) isocratic run at 5 mL/min, 120 Bar backpressure, 40 °C column temperature.

### Method 21

SFC-MS was performed on a Waters/Thar SFC systems with Waters SQD using a YMC Cellulose-C column with a 15% iso-propyl alcohol/CO₂ (with 0.1% diethylamine) isocratic run at 5 mL/min, 120 Bar backpressure, 40 °C column temperature.

### Method 22

SFC-MS was performed on a Waters/Thar SFC systems with Waters SQD using a YMC Cellulose-C column with a 15% methyl alcohol/CO₂ (with 0.1% diethylamine) isocratic run at 5 mL/min, 120 Bar backpressure, 40 °C column temperature.

### Method 23

SFC-MS was performed on a Waters/Thar SFC systems with Waters SQD using a YMC Cellulose-C column with a 25% methyl alcohol/CO₂ (with 0.1% diethylamine) isocratic run at 5 mL/min, 120 Bar backpressure, 40 °C column temperature.

### Method 24

SFC-MS was performed on a Waters/Thar SFC systems with Waters SQD using a YMC Cellulose-SC column with a 55% iso-propyl alcohol/CO₂ (with 0.1% diethylamine) isocratic run at 5 mL/min, 120 Bar backpressure, 40 °C column temperature.

### Method 25

SFC-MS was performed on a Waters/Thar SFC systems with Waters SQD using a Lux Cellulose-3 column with a 10% methyl alcohol/CO₂ (with 0.1% diethylamine) isocratic run at 5 mL/min, 120 Bar backpressure, 40 °C column temperature.

### Method 26

SFC-MS was performed on a Waters/Thar SFC systems with Waters SQD using a Lux Cellulose-3 column with a 25% methyl alcohol/CO₂ (with 0.1% diethylamine) isocratic run at 5 mL/min, 120 Bar backpressure, 40 °C column temperature.

### Method 27

SFC-MS was performed on a Waters/Thar SFC systems with Waters SQD using a Lux Cellulose-3 column with a 30% methyl alcohol/CO₂ (with 0.1% diethylamine) isocratic run at 5 mL/min, 120 Bar backpressure, 40 °C column temperature.

### Method 28

SFC-MS was performed on a Waters/Thar SFC systems with Waters SQD using a Lux Cellulose-4 column with a 40% iso-propyl alcohol/CO₂ (with 0.1% diethylamine) isocratic run at 5 mL/min, 120 Bar backpressure, 40 °C column temperature.

### Method 29

SFC-MS was performed on a Waters/Thar SFC systems with Waters SQD using a Lux Cellulose-4 column with a 40% methyl alcohol/CO₂ (with 0.1% diethylamine) isocratic run at 5 mL/min, 120 Bar backpressure, 40 °C column temperature.

### Method 30

SFC-MS was performed on a Waters/Thar SFC systems with Waters SQD using a Lux Cellulose-4 column with a 50% methyl alcohol/CO₂ (with 0.1% diethylamine) isocratic run at 5 mL/min, 120 Bar backpressure, 40 °C column temperature.

### Method 31

SFC-MS was performed on a Waters/Thar SFC systems with Waters SQD using a Lux Cellulose-4 column with a 55% iso-propyl alcohol/CO₂ (with 0.1% diethylamine) isocratic run at 5 mL/min, 120 Bar backpressure, 40 °C column temperature.

### Method 32

SFC-MS was performed on a Waters/Thar SFC systems with Waters SQD using a Lux Cellulose-4 column with a 55% methyl alcohol/CO₂ (with 0.1% diethylamine) isocratic run at 5 mL/min, 120 Bar backpressure, 40 °C column temperature.

### Method 33

SFC-MS was performed on a Waters/Thar SFC systems with Waters SQD using a YMC Amylose-C column with a 20% ethyl alcohol/CO₂ (with 0.1% diethylamine) isocratic run at 5 mL/min, 120 Bar backpressure, 40 °C column temperature.

### Method 34

SFC-MS was performed on a Waters/Thar SFC systems with Waters SQD using a YMC Amylose-C column with a 30% iso-propyl alcohol/CO₂ (with 0.1% diethylamine) isocratic run at 5 mL/min, 120 Bar backpressure, 40 °C column temperature.

### Method 35

SFC-MS was performed on a Waters/Thar SFC systems with Waters SQD using a YMC Amylose-C column with a 30% methyl alcohol/CO₂ (with 0.1% diethylamine) isocratic run at 5 mL/min, 120 Bar backpressure, 40 °C column temperature.

### Method 36

SFC-MS was performed on a Waters/Thar SFC systems with Waters SQD using a YMC Amylose-C column with a 40% methyl alcohol/CO₂ (with 0.1% diethylamine) isocratic run at 5 mL/min, 120 Bar backpressure, 40 °C column temperature.

### Method 37

SFC-MS was performed on a Waters/Thar SFC systems with Waters SQD using a YMC Amylose-C column with a 55% methyl alcohol/CO₂ (with 0.1% diethylamine) isocratic run at 5 mL/min, 120 Bar backpressure, 40 °C column temperature.

### Method 38

SFC-MS was performed on a Waters/Thar SFC systems with Waters SQD using a YMC Cellulose-C column with a 20% methyl alcohol/CO₂ (with 0.1% diethylamine) isocratic run at 5 mL/min, 120 Bar backpressure, 40 °C column temperature.

### Method 39

SFC-MS was performed on a Waters/Thar SFC systems with Waters SQD using a YMC Cellulose-SC column with a 35% iso-propyl alcohol/CO₂ (with 0.1% diethylamine) isocratic run at 5 mL/min, 120 Bar backpressure, 40 °C column temperature.

### Method 40

SFC-MS was performed on a Waters/Thar SFC systems with Waters SQD using a YMC Cellulose-SC column with a 45% iso-propyl alcohol/CO₂ (with 0.1% diethylamine)isocratic run at 5 mL/min, 120 Bar backpressure, 40 °C column temperature.

### Intermediate 1

### 2-Amino-5-bromo-3-methoxybenzoic acid hydrobromide

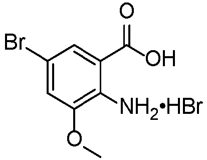

A solution of bromine (6.0 g, 1.9 mL, 37.70 mmol) in chloroform (15 mL) was added dropwise over a period of one hour to a suspension of 2-amino-3-methoxybenzoic acid (6.0 g, 35.90 mmol) in chloroform (180 mL) at 0 °C. The reaction was stirred for a further five hours and slowly allowed to warm to room temperature. The solvent was removed *in vacuo* and the residue was triturated with diethyl ether. The reaction was filtered to give the title compound as a beige solid (11.3 g, 96%).

LCMS (Method 4): [MH+] = 247 at 4.07 min.

### Intermediate 2

### 6-Bromo-8-methoxyquinazolin-4-ol

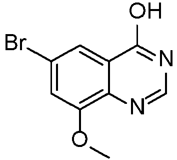

A solution 2-amino-5-bromo-3-methoxybenzoic acid hydrobromide (Intermediate 1) (10.0 g, 30.60 mmol) in formamide (40 mL) was heated to 165 °C for 18 hours. After return to room temperature, the reaction was diluted with water (100 mL) and poured into ice water (400 mL) and filtered. The solid was washed with water (200 mL) and diethyl ether (200 mL) to give the title compound as a light brown solid (5.9 g, 76%).

LCMS (Method 4): [MH+] = 255 at 3.07 min.

### Intermediate 3

### 6-(4-Fluorophenyl)-8-methoxyquinazolin-4-ol

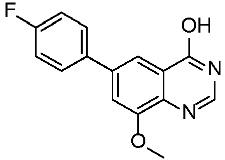

Nitrogen was bubbled for 5 min through a mixture of 6-bromo-8-methoxyquinazolin-4-ol (Intermediate 2) (1.18 g, 4.63 mmol), 4-fluorophenylboronic acid (710 mg, 5.09 mmol) and cesium carbonate (5.73 g, 17.58 mmol) in 1,4-dioxane (30 mL) and water (7.5 mL), then [1,1'-bis(diphenylphosphino)ferrocene]dichloropalladium(II) complex with dichloromethane (190 mg, 0.23 mmol) was added and the reaction was heated to 110 °C for 5 hours. After return to room temperature, the reaction was diluted with water (20 mL), filtered and the solid was washed with 10% methanol in diethyl ether then with diethyl ether to give the title compound (1.0 g, 80%) as a beige solid.

LCMS (Method 5): [MH+] = 271.1 at 0.81 min.

### Intermediate 4

### 6-(4-Fluorophenyl)-8-methoxy-N-(1-(3-methyl-1,2,4-oxadiazol-5-yl)ethyl)quinazolin-4-amine

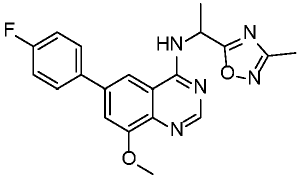

To a solution of 6-(4-fluorophenyl)-8-methoxyquinazolin-4-ol (Intermediate 3) (100 mg, 0.37 mmol) in N,N-dimethylformamide (2 mL) was successively added (benzotriazol-1-yloxy)tripyrrolidinophosphonium hexafluorophosphate (212 mg, 0.41 mmol) and di-isopropylethylamine (0.32 mL, 1.85 mmol). The resulting mixture was heated to 40 °C and stirred for 20 min, 1-(3-methyl-1,2,4-oxadiazol-5-yl)ethan-1-amine (67 mg, 0.41 mmol) was then added and the heating was maintained at 40 °C for 18 hours. After return to room temperature, the mixture was diluted with ethyl acetate (50 mL) and water (20 mL). The organic phase was washed with brine (2 × 20 mL), passed through a hydrophobic frit and the solvent was removed *in vacuo.* The residue was purified by preparative HPLC to give the title compound (43 mg, 31%) as a white solid.

¹H NMR (400 MHz, DMSO): δ 8.85 (d, J = 6.8 Hz, 1 H), 8.49 (s, 1 H), 8.24 (s, 1 H), 7.99 (dd, J = 5.6, 8.6 Hz, 2 H), 7.61 (s, 1 H), 7.44 (dd, J = 8.8, 8.8 Hz, 2 H), 5.88-5.82 (m, 1 H), 4.08 (s, 3 H), 2.37 (s, 3 H), 1.78 (d, J = 7.1 Hz, 3 H). LCMS (Method 4): [MH+] = 375 at 3.29 min.

The compounds reported in the table below were synthesised following the same procedure described for the preparation of 6-(4-Fluorophenyl)-8-methoxy-N-(1-(3-methyl-1,2,4-oxadiazol-5-yl)ethyl)quinazolin-4-amine (Intermediate 4).

| **Intermediate No.** | **Chemical Name Structure** | **Analytical Data ¹H NMR** |
|---|---|---|
| | | **LC-MS** |
| Intermediate 5 | 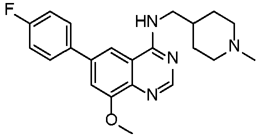 6-(4-Fluorophenyl)-8-methoxy-*N*-[(1-methyl-4-piperidyl)methyl]quinazolin-4-amine | ¹H NMR (400 MHz, DMSO): δ 8.43 (s, 1 H), 8.34 (dd, J = 5.5, 5.5 Hz, 1 H), 8.10 (d, J = 1.6 Hz, 1 H), 7.94-7.89 (m, 2 H), 7.50 (d, J = 1.6 Hz, 1 H), 7.39 (dd, J = 8.8, 8.8 Hz, 2 H), 4.01 (s, 3 H), 3.46 (s, 2 H), 2.89 (d, J = 11.4 Hz, 2 H), 2.27 (s, 3 H), 2.09-2.03 (m, 2 H), 1.76 (dd, J = 10.2, 10.2 Hz, 3 H), 1.34-1.24 (m, 2 H). LCMS (Method 4): [MH+] = 381 at 2.27 min. |

### Intermediate 6

### (R)-6-Bromo-8-methoxy-N-(1-(6-methylpyridazin-3-yl)ethyl)quinazolin-4-amine

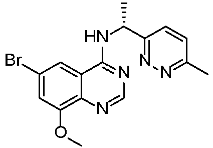

To a solution of 6-bromo-8-methoxyquinazolin-4-ol (Intermediate 2) (65 mg, 0.27 mmol) in *N,N*-dimethylformamide (1.5 mL) was successively added (benzotriazol-1-yloxy)tripyrrolidinophosphonium hexafluorophosphate (139 mg, 0.27 mmol) and di-isopropylethylamine (0.2 mL, 0.81 mmol). The resulting mixture was heated to 60 °C for one hour then (*R*)-1-(6-methylpyridazin-3-yl)ethan-1-amine (65 mg, 0.27 mmol) was added and the heating was maintained at 60 °C for 18 hours. After return to room temperature, the reaction mixture was directly concentrated onto silica gel and purified by chromatography on silica gel eluting with 0-100% (10% MeOH in ethyl acetate) in ethyl acetate to give the title compound as a beige solid (100 mg, quantitative yield).

LCMS (Method 4): [MH+] = 374 at 2.42 min.

The following intermediates reported in the table below were synthesised following the same procedure described for the preparation of (*R*)-6-Bromo-8-methoxy-N-(1-(6-methylpyridazin-3-yl)ethyl)quinazolin-4-amine (Intermediate 6):

| **Intermediate No.** | **Chemical name Structure** | **Analytical data LC-MS** |
|---|---|---|
| Intermediate 7 | 6-Bromo-8-methoxy-*N*-[(1*R*)-1-[2-(trifluoromethyl)pyrimidin-5-yl]ethyl]quinazolin-4 amine 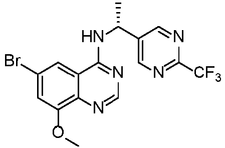 | LCMS (Method 4): [MH+] = 428 at 3.19 min |
| Intermediate 8 | 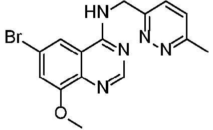 6-Bromo-8-methoxy-*N*-((6-methylpyridazin-3-yl)methyl)quinazolin-4-amine | LCMS (Method 3): [MH+] = 360 at 3.06 min. |

### Intermediate 9

### 6-(4-Fluoro-2-methoxyphenyl)-8-methoxy-N-((6-methylpyridazin-3-yl)methyl)quinazolin-4-amine

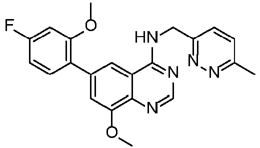

To a solution of 6-bromo-8-methoxy-*N*-((6-methylpyridazin-3-yl)methyl)quinazolin-4-amine (intermediate 8) (70 mg, 0.19 mmol) in 1,2-dimethoxyethane (3.0 mL) was added (4-fluoro-2-methoxyphenyl)boronic acid (41 mg, 0.24 mmol), [1,1'-*bis*(diphenylphosphino)ferrocene] dichloropalladium(II) complex with dichloromethane (8.2 mg, 0.01 mmol), caesium carbonate (130 mg, 0.40 mmol) and water (0.3 mL). The resulting mixture was heated to 95 °C for 18 h. After return to room temperature, the reaction was filtered through Celite^{®}. The Celite^{®} cake was rinsed with ethyl acetate (2 × 20 mL). Combined organic phases were washed with brine (2 × 20 mL), filtered through a hydrophobic frit and the solvent was removed *in vacuo.* The residue was purified by preparative HPLC to give the title compound as an off-white solid (35 mg, 45%).

¹H NMR (400 MHz, DMSO): δ 8.92 (dd, J = 5.8, 5.8 Hz, 1 H), 8.41 (s, 1 H), 7.91 (d, J = 1.5 Hz, 1 H), 7.54-7.46 (m, 3 H), 7.37 (d, J = 1.4 Hz, 1 H), 7.10 (dd, J = 2.4, 11.5 Hz, 1 H), 6.97-6.91 (m, 1 H), 4.99 (d, J = 5.6 Hz, 2 H), 3.93 (s, 3 H), 3.84 (s, 3 H), 2.59 (s, 3 H). LCMS (Method 3): [MH+] = 406 at 3.97 min.

The following compounds reported in the table below were prepared *via* adaptation of the above procedure starting from appropriate intermediate reported in table.

| **Intermediate No.** | **Chemical Name Structure** | **Analytical data ¹H NMR** | **Starting Intermediate** |
|---|---|---|---|
| | | **LC-MS** | |
| Intermediate 10 | (*R*)-6-(4-fluorophenyl)-8-methoxy-*N*-(1-(2-(trifluoromethyl)pyrimidin-5-yl)ethyl)quinazolin-4-amine 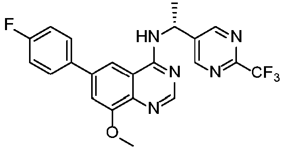 | LCMS (Method 5): 0.79 min, m/z 443.8 [M+2]+, | Intermediate 7 |
| | | ¹H NMR (400 MHz, DMSO-d6) δ ppm 9.12 (s, 2 H), 8.55 (d, J=7.0 Hz, 1 H), 8.37 (s, 1 H), 8.14 (d, J=1.3 Hz, 1 H), 7.83 - 7.96 (m, 2 H), 7.48 (d, J=1.3 Hz, 1 H), 7.35 (t, J=8.8 Hz, 2 H), 5.50 - 5.60 (m, 1 H), 3.97 (s, 3 H), 1.69 (d, J=7.0 Hz, 3 H). | |
| Intermediate 11 | 6-(4-fluorophenyl)-8-methoxy-*N*-((6-methylpyridazin-3-yl)methyl)quinazolin-4-amine 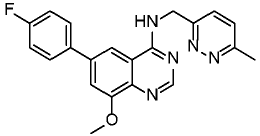 | LCMS (Method 6): 0.88 min, 375.9 m/z [M+H]+, | Intermediate 8 |
| | | ¹H NMR (400 MHz, ACETONITRILE-d3) δ □ppm 8.43 (s, 1 H), 7.66 - 7.84 (m, 3 H), 7.53 (d, J=8.8 Hz, 1 H), 7.44 (d, J=1.3 Hz, 1 H), 7.39 (d, J=8.8 Hz, 1 H), 7.20 - 7.30 (m, 2 H), 7.00 - 7.15 (m, 1 H), 5.06 (s, 2 H), 4.04 (s, 3 H), 2.62 (s, 3 H). | |

### Intermediate 12

### 6-(5,5-dimethyl-1,3,2-dioxaborinan-2-yl)-8-methoxy-N-((6-methylpyridazin-3-yl)methyl)quinazolin-4-amine

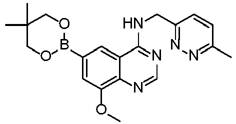

Nitrogen was bubbled for 5 min through a mixture of 6-bromo-8-methoxy-N-((6-methylpyridazin-3-yl)methyl)quinazolin-4-amine (Intermediate 8) (100 mg, 0.28 mmol), bis(neopentyl glycolato)diboron (66 mg, 0.29 mmol), [1,1'-bis(diphenylphosphino)ferrocene]-dichloropalladium (II) complex with dichloromethane (10 mg, 0.02 mmol) and potassium acetate (54 mg, 0.55 mmol) in 1,4-dioxane (3.0 mL). The mixture was heated to 100 °C for 3 hours. After return to room temperature, the mixture was taken on to the next step as a 1,4-dioxane solution without further purification.

### Intermediate 13

### Preparation of 8-methoxy-N-((6-methylpyridazin-3-yl)methyl)-6-(5-methylthiazol-2-yl)quinazolin-4-amine

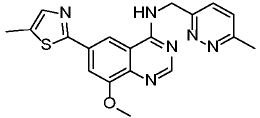

To the above solution of 6-(5,5-dimethyl-1,3,2-dioxaborinan-2-yl)-8-methoxy-N-((6-methylpyridazin-3-yl)methyl)quinazolin-4-amine (100 mg, 0.28 mmol) was added aqueous caesium carbonate (181 mg, 0.56 mmol, 0.4 mL), 2-bromo-5-methylthiazole (64 mg, 0.28 mmol) and tetrakis(triphenylphosphine)palladium(0) (20 mg, 0.02 mmol). The resulting mixture was heated to 95 °C for 16 hours. After return to room temperature, the mixture was filtered through Celite^{®} and the filter cake rinsed with ethyl acetate (2 × 10 mL). The organic phases were washed with saturated aqueous ammonium chloride (10 mL), passed through a hydrophobic frit and the solvent was removed *in vacuo.* The residue was purified by preparative HPLC to give the title compound as an off-white solid (25 mg, 24%).

¹H NMR (400 MHz, DMSO): δ 9.24 (dd, J = 5.7, 5.7 Hz, 1 H), 8.43 (s, 1 H), 8.37 (d, J = 1.5 Hz, 1 H), 7.75 (d, J = 1.5 Hz, 1 H), 7.68 (d, J = 1.1 Hz, 1 H), 7.57 (d, J = 8.7 Hz, 1 H), 7.50 (d, J = 8.7 Hz, 1 H), 5.02 (d, J = 5.8 Hz, 2 H), 4.00 (s, 3 H), 2.60 (s, 3 H), 2.50 (s, 3 H). LCMS (Method 3): [MH+] = 379 at 3.20 min.

The following compounds reported in the table below were prepared according to the same procedure described for the preparation of 8-methoxy-N-((6-methylpyridazin-3-yl)methyl)-6-(5-methylthiazol-2-yl)quinazolin-4-amine (Intermediate 13)

| **Intermediate No.** | **Chemical Name Structure** | **Analytical data ¹H NMR LC-MS** | **Starting Intermediate** |
|---|---|---|---|
| Intermediate 14 | 8-Methoxy-N-[(1*R*)-1-(6-methylpyridazin-3-yl)ethyl]-6-(5-methylpyrimidin-2-yl)quinazolin-4-amine 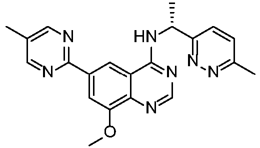 | ¹H NMR (400 MHz, DMSO): δ 9.05 (d, J = 1.4 Hz, 1 H), 9.00 (d, J = 7.5 Hz, 1 H), 8.86 (s, 2 H), 8.40 (s, 1 H), 8.21 (d, J = 1.4 Hz, 1 H), 7.64 (d, J = 8.7 Hz, 1 H), 7.50 (d, J = 8.8 Hz, 1 H), 5.81-5.76 (m, 1 H), 4.01 (s, 3 H), 2.59 (s, 3 H), 2.39 (s, 3 H), 1.72 (d, J = 7.2 Hz, 3 H). LCMS (Method 3): [MH+] = 388 at 3.64 min. Chiral analysis (Method 37) at 1.56 min. | Intermediate 6 |

### Intermediate 15

### 8-methoxy-N-((6-methylpyridazin-3-yl)methyl)-6-(4,4,5,5-tetramethyl-1,3,2-dioxaborolan-2-yl)quinazolin-4-amine

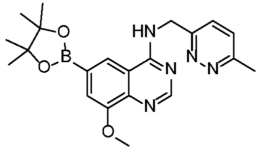

Nitrogen was bubbled for 5 min through a mixture of 6-bromo-8-methoxy-N-((6-methylpyridazin-3-yl)methyl)quinazolin-4-amine (Intermediate 8) (250 mg, 0.69 mmol), bis-(pinacolato)diboron (194 mg, 0.76 mmol), [1,1'-*bis*-(diphenylphosphino)-ferrocene]dichloropalladium(II) (25 mg, 0.03 mmol) and potassium acetate (204 mg, 2.08 mmol) in 1,4-dioxane (15.0 mL). The mixture was heated at 90°C for 18 hours. After return to room tempearature, the reaction was filtered through Celite^{®} and the solvent was removed *in vacuo.* The residue was taken on to the next step without further purification.

### Intermediate 16

### 8-methoxy-6-(5-methyl-1,3,4-thiadiazol-2-yl)-N-((6-methylpyridazin-3-yl)methyl)quinazolin-4-amine

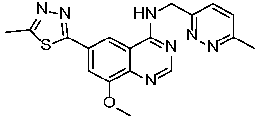

Nitrogen was bubbled for 5 min through a mixture of 2-bromo-5-methyl-1,3,4-thiadiazole (34 mg, 0.19 mmol), 8-methoxy-N-((6-methylpyridazin-3-yl)methyl)-6-(4,4,5,5-tetramethyl-1,3,2-dioxaborolan-2-yl)quinazolin-4-amine (70 mg, 0.17 mmol), potassium carbonate (36 mg, 0.26 mmol) and water (0.5 mL) in 1,4-dioxane (4.0 mL), then tetrakis(triphenylphosphine)palladium(0) (20 mg, 0.02 mmol) was added. The resulting mixture was heated to 95 °C for 16 hours. After return to room temperature, the reaction was filtered through Celite^{®}, rinced with ethyl acetate (20 mL). The organic phases were combined, passed through a hydrophobic frit and the solvent was removed *in vacuo.* The residue was purified by preparative HPLC to give the title compound as an off-white solid (21.0 mg, 32%).

¹H NMR (400 MHz, DMSO): δ 9.31 (dd, J = 5.8, 5.8 Hz, 1 H), 8.47 (s, 1 H), 8.44 (d, J = 1.7 Hz, 1 H), 7.80 (d, J = 1.4 Hz, 1 H), 7.58 (d, J = 8.7 Hz, 1 H), 7.51 (d, J = 8.7 Hz, 1 H), 5.03 (d, J = 5.8 Hz, 2 H), 4.03 (s, 3 H), 2.84 (s, 3 H), 2.60 (s, 3 H). LCMS (Method 3): [MH+] = 380 at 2.13 min.

The following compounds reported in the table below were prepared according to the same procedure described for the preparation of 8-methoxy-6-(5-methyl-1,3,4-thiadiazol-2-yl)-*N*-((6-methylpyridazin-3-yl)methyl)quinazolin-4-amine (Intermediate 16):

| **Intermediate No.** | **Chemical Name Structure** | **Analytical data ¹H NMR** | **Starting Intermediate** |
|---|---|---|---|
| | | **LC-MS** | |
| Intermediate 17 | 8-Methoxy-*N*-[(6-methylpyridazin-3-yl)methyl]-6-(5-methylpyrimidin-2-yl)quinazolin-4-amine 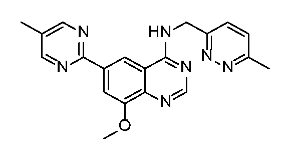 | ¹H NMR (400 MHz, DMSO): δ 9.29 (dd, J = 5.7, 5.7 Hz, 1 H), 8.93 (d, J = 1.5 Hz, 1 H), 8.84 (s, 2 H), 8.44 (s, 1 H), 8.22 (d, J = 1.5 Hz, 1 H), 7.54 (d, J = 8.7 Hz, 1 H), 7.49 (d, J = 8.7 Hz, 1 H), 5.01 (d, J = 5.8 Hz, 2 H), 4.02 (s, 3 H), 2.60 (s, 3 H), 2.38 (s, 3 H). LCMS (Method 3): [MH+] = 374 at 3.25 min. | Intermediate 8 |

### Intermediate 18

### (R)-6-(4-fluorophenyl)-4-((1-(2-(trifluoromethyl)pyrimidin-5-yl)ethyl)amino)-quinazolin-8-ol

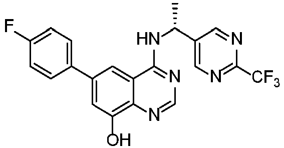

To a solution of 6-(4-fluorophenyl)-8-methoxy-*N*-[(1*R*)-1-[2-(trifluoromethyl)-pyrimidin-5-yl]ethyl]quinazolin-4-amine (Intermediate 10) (490 mg, 1.11 mmol) in chloroform (8 mL) at 0 °C was added dropwise boron tribromide (0.32 mL, 3.32 mmol). The reaction was then allowed to warm to room temperature and was heated to 65 °C for 18 hours. After return to room temperature, the reaction was cooled down in an ice-bath and quenched with methanol (2 mL). The solvent was removed *in vacuo.* The residue was diluted with ethyl acetate (50 mL) and washed with saturated aqueous NaHCO₃ solution (50 mL). The aqueous layer was then extracted with ethyl acetate (2 × 20 mL). The organic phases were combined, passed through a hydrophobic frit and the solvent was removed *in vacuo* to give the title compound as a grey solid (416 mg, 88%).

¹H NMR (400 MHz, DMSO): δ 9.18 (s, 2 H), 8.65 (d, J = 7.0 Hz, 1 H), 8.46 (s, 1 H), 8.09 (s, 1 H), 7.86 (dd, J = 5.5, 8.7 Hz, 2 H), 7.45 (d, J = 1.3 Hz, 1 H), 7.37 (dd, J = 8.8, 8.8 Hz, 2 H), 5.71-5.67 (m, 1 H), 1.75 (d, J = 7.0 Hz, 3 H), OH not observed.

The following compounds were prepared *via* adaptations of the above procedure starting from substrate reported in table.

| **Intermediate No.** | **Structure** | **Analytical data LC-MS** | **Substrate** |
|---|---|---|---|
| **Intermediate 19** | 6-(4-fluorophenyl)-4-(((6-methylpyridazin-3-yl)methyl)amino)quinazolin-8-ol 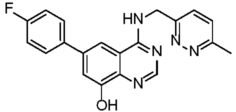 | LCMS: 0.51 min, 362.1 [M+H]+, Method 5 | **Intermediate 11** |
| **Intermediate 19a** | (R)-4-((1-(6-methylpyridazin-3-yl)ethyl)amino)-6-(5-methylpyrimidin-2-yl)quinazolin-8-ol 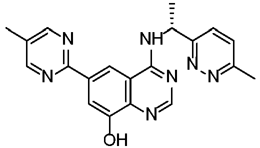 | LCMS (Method 4): [MH+] = 374 at 2.45 min | **Intermediate 14** |
| **Intermediate 19b** | 4-(((6-methylpyridazin-3-yl)methyl)amino)-6-(5-methylpyrimidin-2-yl)quinazolin-8-ol 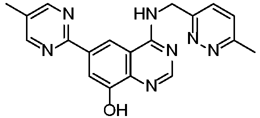 | LCMS (Method5): [MH+] = 360.4 at 0.92 | **Intermediate 17** |
| **Intermediate 20** | 6-(4-fluorophenyl)-4-(((1-methylpiperidin-4-yl)methyl)amino)quinazolin-8-ol 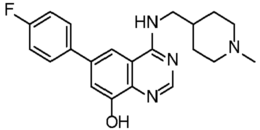 | LCMS (Method5 ): [MH+] = 367.3 at 0.44 | **Intermediate 5** |

### Example 1

### 8-(2,2-difluoroethoxy)-6-(4-fluorophenyl)-N-((6-methylpyridazin-3-yl)methyl)quinazolin-4-amine

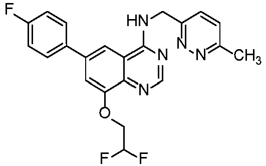

To a suspension of 6-(4-fluorophenyl)-4-(((6-methylpyridazin-3-yl)methyl)amino)quinazolin-8-ol (100 mg, 0.28 mmol) (Intermediate 19) and cesium carbonate (180 mg, 0.55 mmol) in DMF (1.6 mL), 1,1-difluoro-2-iodoethane (0.04 ml, 0.41 mmol) was added and the reaction was stirred at 70 °C for 2h. The mixture was concentrated under reduced pressure and the resulting crude was purified by flash chromatogrphy (Biotage Isolera, 30 g C18 cartridge, gradient elution from 0 to 70% B in A. A: water/acetonitrile 95:5 + 0.05% HCOOH, B: acetonitrile/water 95:5 + 0.05% HCOOH) to give the title product (14.9 mg, 0.03 mmol, 12.7 % yield) as a white solid.

¹H NMR (400 MHz, DMSO-d6) δ ppm 9.05 (br s, 1 H), 8.41 (s, 1 H), 8.21 - 8.25 (m, 1 H), 7.88 - 7.95 (m, 2 H), 7.60 - 7.66 (m, 1 H), 7.43 - 7.54 (m, 2 H), 7.34 (t, J=8.88 Hz, 2 H), 6.49 (dt, J=1.00 Hz, 1 H), 4.99 (d, J=5.48 Hz, 2 H), 4.58 (td, J=14.47, 3.73 Hz, 2 H), 2.55 (s, 3 H).

### Example 2

### (R)-8-(2,2-difluoroethoxy)-6-(4-fluorophenyl)-N-(1-(2-(trifluoromethyl)pyrimidin-5-yl)ethyl)quinazolin-4-amine

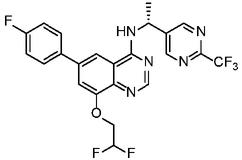

A suspension of (R)-6-(4-fluorophenyl)-4-((1-(2-(trifluoromethyl)pyrimidin-5-yl)ethyl)amino)quinazolin-8-ol (100 mg, 0.23 mmol) (Intermediate 18), cesium carbonate (152 mg, 0.47 mmol) and 1,1-difluoro-2-iodoethane (0.03 ml, 0.35 mmol) in DMF (1.6 mL) was stirred at 70 °C for 2h.

The mixture was then cooled down and partitoned between DCM (10 mL) and 1 M aq HCl (10 mL) and the organic phase separated then concentrated under reduced pressure and the crude purified by RP flash chromatography (Biotage Isolera, 12 g C18 cartridge, gradient elution from 0 to 100% B in A. A: water/acetonitrile 95:5 + 0.05% HCOOH, B: acetonitrile/water 95:5 + 0.05% HCOOH) to give the title product (53.5 mg, 0.108 mmol, 46.6 % yield) as a yellow solid.

¹H NMR (600 MHz, DMSO-d6) δ ppm 9.16 (s, 2 H) 8.66 (d, J=6.80 Hz, 1 H) 8.44 (s, 1 H) 8.28 (d, J=1.54 Hz, 1 H) 7.95 (dd, J=8.78, 5.45 Hz, 2 H) 7.65 (d, J=1.67 Hz, 1 H) 7.39 (t, J=8.43 Hz, 2 H) 6.38 - 6.65 (m, 1 H) 5.67 (quin, J=6.99 Hz, 1 H) 4.60 (td, J=14.43, 3.72 Hz, 2 H) 1.73 (d, J=7.18 Hz, 3 H).

LCMS (Method 5): [MH+] = 493,67 at 0.86 min.

### Intermediate 21

### Methyl 3-(2,2-difluoroethoxy)-2-nitrobenzoate

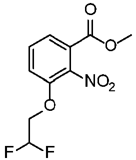

To a 10 mL microwave vial was added methyl 3-fluoro-2-nitrobenzoate (600 mg, 3.00 mmol) in DMF (3 mL) and 2,2-difluoroethanol (544 mg, 6.63 mmol). To the stirred solution was added potassium carbonate (833 mg, 6.03 mmol) and the mixture was heated to 120 °C in a microwave reactor for 20 minutes. This procedure was repeated three times, and the pooled reaction mixtures were cooled to room temperature, poured into water (65 mL) and extracted with diethyl ether (30 mL). The aqueous phase was then extracted with additional diethyl ether (2 × 30 mL). The combined organic layers were dried over magnesium sulfate, filtered and the solvent was removed *in vacuo.* The resulting residue was purified by flash column chromatography on silica gel eluting with 50% ethyl acetate in cyclohexane to give the title compound (1876 mg, 81%) as a pale yellow solid.

¹H NMR (400 MHz, CDCl₃): δ 7.71-7.68 (m, 1 H), 7.55-7.51 (m, 1 H), 7.30-7.26 (m, 1 H), 6.22-5.92 (m, 1 H), 4.34-4.26 (m, 2 H), 3.91-3.90 (m, 3 H). LCMS (Method 3): [MH+] = 262 at 4.17 min.

### Intermediate 22

### Methyl 2-amino-3-(2,2-difluoroethoxy)benzoate

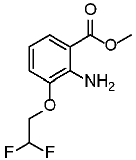

To methyl 3-(2,2-difluoroethoxy)-2-nitrobenzoate (1.85 g, 7.06 mmol) in THF (15 mL), methanol (15 mL) and water (5 mL), was added iron powder (2.59 g, 46.5 mmol) and ammonium chloride (1.24 g, 23.2 mmol). The reaction mixture was heated to 90 °C for 2 hours. After this time the reaction mixture was cooled to room temperature, filtered through Celite^{®} and concentrated *in vacuo.* The residue was partitioned between ethyl acetate (60 mL) and water (60 mL). The combined organic layers were extracted, dried over magnesium sulfate, filtered and the solvent removed *in vacuo* to give the title compound (1.48 g, 91%) as a pale yellow oil.
LCMS (Method 3): [MH+] = 232 at 4.47 min

### Intermediate 23

### Methyl 2-amino-5-bromo-3-(2,2-difluoroethoxy)benzoate

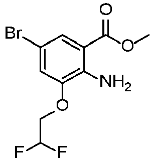

Bromine (0.26 mL, 5.00 mmol) was added dropwise to a solution of Methyl 2-amino-3-(2,2-difluoroethoxy) benzoate (1100 mg, 4.76 mmol) in chloroform (18 mL) at 0 °C. The stirring was maintained at 0 °C for 1 hour. The reaction mixture was quenched with sodium thiosulfate and the mixture was stirred for 10 minutes. A saturated sodium hydrogen carbonate solution (6 mL) was added and the reaction was stirred for 5 minutes. The two phases were separated. The aqueous phase was extracted with DCM (2 × 50 mL). The combined organic phases were concentrated *in vacuo* to give the title compound (1354 mg, 93%) as an off white solid.

¹H NMR (400 MHz, CDCl₃): δ 7.68 (d, J = 2.3 Hz, 1 H), 6.93 (d, J = 2.0 Hz, 1 H), 6.27-5.96 (m, 3 H), 4.25-4.16 (m, 2 H), 3.88-3.87 (m, 3 H).

### Intermediate 24

### Methyl 2-amino-3-(2,2-difluoroethoxy)-5-(5-fluoropyridin-2-yl)benzoate

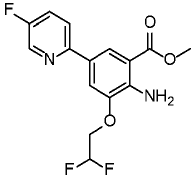

To methyl 2-amino-5-bromo-3-(2,2-difluoroethoxy)benzoate (422 mg, 1.36 mmol) and tetrakis(triphenylphosphine)palladium(0) (79 mg, 0.07 mmol) in dioxane (5 mL), was added 4-fluoro-2-(tributylstannyl)pyridine (525 mg, 1.36 mmol). The reaction mixture was stirred under a nitrogen atmosphere at 100 °C for 16 hours. After this time the reaction mixture was poured into 1 M aqueous potassium fluoride solution (10 mL) and rapidly stirred for 1 hour. The resulting suspension was filtered through Celite^{®}, washing with ethyl acetate (30 mL). The organic layers were extracted from water (10 mL), washed with saturated aqueous sodium chloride (2 × 15 mL), dried over magnesium sulfate, filtered and the solvent removed *in vacuo.* The resulting residue was purified by flash column chromatography on silica gel eluting with 15% ethyl acetate in cyclohexane to give the title compound (261 mg, 59%) as a colourless solid.

¹H NMR (400 MHz, CDCl₃): δ 8.46 (d, J = 3.0 Hz, 1 H), 8.09 (d, J = 1.9 Hz, 1 H), 7.71-7.68 (m, 2 H), 7.45-7.40 (m, 1 H), 6.31-6.02 (m, 3 H), 4.40-4.32 (m, 2 H), 3.92 (s, 3 H). LCMS (Method 4): [MH+] = 327 at 4.95 min.

### Intermediate 25

### 2-Amino-3-(2,2-difluoroethoxy)-5-(5-fluoropyridin-2-yl)benzoic acid

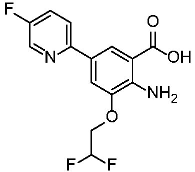

To methyl 2-amino-3-(2,2-difluoroethoxy)-5-(5-fluoropyridin-2-yl)benzoate (266 mg, 0.82 mmol) in methanol (5 mL), THF (5 mL) and water (1 mL) was added lithium hydroxide hydrate (29 mg, 1.22 mmol). The reaction mixture was stirred at 50 °C for 16 hours. After this time the reaction mixture was concentrated *in vacuo,* to give the title compound (282 mg, 100%) as a colourless solid.

¹H NMR (400 MHz, DMSO): δ 8.51 (d, J = 3.0 Hz, 1 H), 8.21 (s, 1 H), 7.83 (dd, J = 4.4, 8.9 Hz, 1 H), 7.72-7.66 (m, 1 H), 7.53-7.51 (m, 1 H), 6.44 (tt, J = 3.6, 54.8 Hz, 1 H), 4.38-4.29 (m, 2 H).

### Intermediate 26

### 8-(2,2-difluoroethoxy)-6-(5-fluoropyridin-2-yl)quinazolin-4(3H)-one

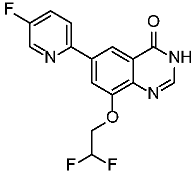

To amino-3-(2,2-difluoroethoxy)-5-(5-fluoropyridin-2-yl)benzoic acid (220 mg, 0.71 mmol) was added formamide (2 mL). The reaction mixture was stirred at 130 °C for 48 hours. After this time the reaction mixture was cooled to room temperature and poured into water (25 mL). The resulting solid was filtered, washed with water (10 mL), followed by diethyl ether (15 mL) and air dried to give the title compound (205 mg, 91%) as a colourless solid.

¹H NMR (400 MHz, DMSO): δ 12.42-12.40 (m, 1 H), 8.73 (d, J = 2.9 Hz, 1 H), 8.46 (d, J = 1.8 Hz, 1 H), 8.29 (dd, J = 4.3, 8.8 Hz, 1 H), 8.14 (s, 1 H), 8.08 (d, J = 1.8 Hz, 1 H), 7.94-7.87 (m, 1 H), 6.52 (tt, J = 3.7, 54.9 Hz, 1 H), 4.66-4.56 (m, 2 H). LCMS (Method 3): [MH+] = 322 at 3.41 min

### Intermediate 27

### 4-Chloro-8-(2,2-difluoroethoxy)-6-(5-fluoropyridin-2-yl)quinazoline

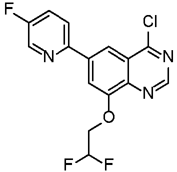

To 8-(2,2-difluoroethoxy)-6-(5-fluoropyridin-2-yl)quinazolin-4(3H)-one (200 mg, 0.62 mmol) in toluene (7 mL) was added DIPEA (402 mg, 3.11 mmol). The reaction mixture was stirred at 120 °C for 10 minutes, then phosphorus oxychloride (115 mg, 0.75 mmol) was added dropwise. The reaction mixture was then stirred at 120 °C for a further 2 hours. After this time the reaction mixture was cooled to room temperature and solvent removed *in vacuo.* The resulting residue was partitioned between dichloromethane (10 mL) and saturated aqueous sodium bicarbonate solution (10 mL). The organic layers were separated, dried, filtered and concentrated *in vacuo* to give the title compound (186 mg, 88%) as a pale brown solid that was directly taken on to the next step.

### Example 4

### 8-(2,2-difluoroethoxy)-6-(5-fluoropyridin-2-yl)-N-((6-methylpyridazin-3-yl)methyl)quinazolin-4-amine

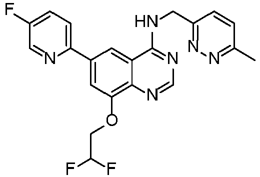

To crude 4-chloro-8-(2,2-difluoroethoxy)-6-(5-fluoropyridin-2-yl)quinazoline (65 mg, 0.19 mmol) and (6-methylpyridazin-3-yl)methanamine (28 mg, 0.23 mmol) in 1,4-dioxane (3 mL) was added DIPEA (124 mg, 0.95 mmol). The reaction mixture was stirred at 50 °C for 16 hours. After this time the reaction mixture was cooled to room temperature and solvents removed *in vacuo.* The resulting residue was purified by preparative HPLC to give the title compound (38 mg, 28%) as an off-white solid.

¹H NMR (400 MHz, DMSO): δ 9.20 (dd, J = 5.8, 5.8 Hz, 1 H), 8.74 (d, J = 3.0 Hz, 1 H), 8.67 (d, J = 1.5 Hz, 1 H), 8.48 (s, 1 H), 8.28 (dd, J = 4.1, 8.9 Hz, 1 H), 8.06 (d, J = 1.4 Hz, 1 H), 8.01-7.95 (m, 1 H), 7.58 (d, J = 8.7 Hz, 1 H), 7.51 (d, J = 8.8 Hz, 1 H), 6.54 (tt, J = 3.6, 54.6 Hz, 1 H), 5.05 (d, J = 5.8 Hz, 2 H), 4.65-4.56 (m, 2 H), 2.61 (s, 3 H). LCMS (Method 4): [MH+] = 427 at 2.77 min.

The following compound reported in the table below was prepared according to the same procedure described for the preparation of 8-(2,2-difluoroethoxy)-6-(5-fluoropyridin-2-yl)-N-((6-methylpyridazin-3-yl)methyl)quinazolin-4-amine:

| **Example No.** | **Chemical Name Structure** | **Analytical data ¹H NMR** |
|---|---|---|
| | | **LC-MS** |
| **Example 5** | 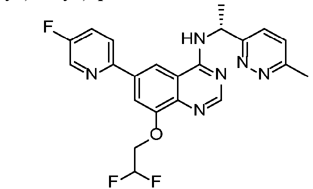 (R)-8-(2,2-difluoroethoxy)-6-(5-fluoropyridin-2-yl)-N-(1-(6-methylpyridazin-3-yl)ethyl)quinazolin-4-amine | ¹H NMR (400 MHz, DMSO): δ 8.85 (d, J = 7.3 Hz, 1 H), 8.77-8.74 (m, 2 H), 8.43 (s, 1 H), 8.34 (dd, J = 4.3, 8.9 Hz, 1 H), 8.06 (d, J = 1.4 Hz, 1 H), 8.02-7.96 (m, 1 H), 7.64 (d, J = 8.7 Hz, 1 H), 7.51 (d, J = 8.8 Hz, 1 H), 6.52 (tt, J = 19.4, 41.8 Hz, 1 H), 5.81-5.75 (m, 1 H), 4.64-4.54 (m, 2 H), 2.60 (s, 3 H), 1.74 (d, J = 7.0 Hz, 3 H). LCMS (Method 4): [MH+] = 441 at 2.95 min. |

The following compounds reported in the table below were obtained as single isomers by chiral preparative SFC purification of the corresponding racemic mixture, which was prepared according to the same procedure described for the preparation of 8-(2,2-difluoroethoxy)-6-(5-fluoropyridin-2-yl)-N-((6-methylpyridazin-3-yl)methyl)quinazolin-4-amine:

| **Example No.** | **Chemical name Structure** | **Analytical data ¹H NMR** |
|---|---|---|
| | | **LC-MS** |
| **Example 6** | Single enantiomer 1 of 8-(2,2-difluoroethoxy)-6-(5-fluoro-2-pyridyl)-N-[1-(5-methyl-1,3,4-thiadiazol-2-yl)ethyl]quinazolin-4-amine 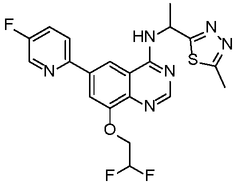 | ¹H NMR (400 MHz, DMSO): δ 9.03-8.99 (m, 1 H), 8.74 (d, J = 2.9 Hz, 1 H), 8.67 (d, J = 1.5 Hz, 1 H), 8.56-8.55 (m, 1 H), 8.32-8.27 (m, 1 H), 8.07 (d, J = 1.5 Hz, 1 H), 8.01-7.93 (m, 1 H), 6.69-6.38 (m, 1 H), 6.03-5.95 (m, 1 H), 4.65-4.56 (m, 2 H), 2.66 (s, 3 H), 1.82 (d, J = 7.0 Hz, 3 H). LCMS (Method 4): [MH⁺] = 447 at 3.15 min. Chiral analysis (Method 22) at 4.5 min. |
| **Example 7** | Single enantiomer 2 of 8-(2,2-difluoroethoxy)-6-(5-fluoro-2-pyridyl)-N-[1-(5-methyl-1,3,4-thiadiazol-2-yl)ethyl]quinazolin-4-amine 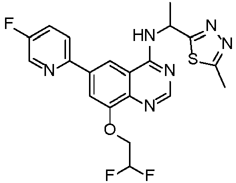 | ¹H NMR (400 MHz, DMSO): δ 9.02 (d, J = 7.5 Hz, 1 H), 8.74 (d, J = 2.9 Hz, 1H), 8.68 (d, J = 1.6 Hz, 1 H), 8.56 (s, 1 H), 8.30 (dd, J = 4.1, 8.9 Hz, 1 H), 8.07 (d, J = 1.5 Hz, 1 H), 7.98 (ddd, J = 8.7, 8.7, 3.0 Hz, 1 H), 6.69-6.39 (m, 1 H), 6.03-5.95 (m, 1 H), 4.66-4.56 (m, 2 H), 2.66 (s, 3 H), 1.82 (d, J = 7.0 Hz, 3 H). LCMS (Method 4): [MH⁺] = 447 at 3.03 min. Chiral analysis (Method 22) at 5.3 min. |

### Example 8

### 8-(2,2-Difluoroethoxy)-N-((6-methylpyridazin-3-yl) methyl)-6-(5-methylpyridin-2-yl) quinazolin-4-amine

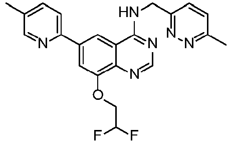

### Intermediate 28

### 2-Amino-5-bromo-3-(2,2-difluoroethoxy) benzoic acid

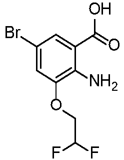

Methyl 2-amino-5-bromo-3-(2,2-difluoroethoxy) benzoate (1354 mg, 4.37 mmol) was dissolved in THF (30 mL) and methanol (30 mL). A solution of lithium hydroxide (157 mg, 6.55 mmol) in water (4 mL) was added and the reaction was stirred at 50 °C for 18 hours. The reaction mixture was concentrated *in vacuo* to give the title compound (1351 mg, >100%) as an orange solid.

¹H NMR (400 MHz, MeOD): δ 7.56 (d, J = 2.3 Hz, 1 H), 6.89 (d, J = 2.3 Hz, 1 H), 6.25 (t, J = 3.9 Hz, 1 H), 6.12 (t, J = 3.8 Hz, 1 H), 5.98 (t, J = 3.8 Hz, 1 H), 4.17-4.08 (m, 2 H).

### Intermediate 29

### 6-Bromo-8-(2,2-difluoroethoxy)quinazolin-4-ol

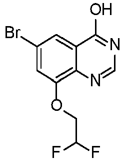

2-Amino-5-bromo-3-(2,2-difluoroethoxy)benzoic acid (1250 mg, 4.22 mmol) was dissolved in formamide (12 mL). The reaction was stirred at 130 °C for 24 hours. The mixture was diluted with water and filtered. The solid was then washed with water followed by 9:1 diethyl ether/methanol to give the title compound (781 mg, 61%) as a light brown solid.

¹H NMR (400 MHz, DMSO): δ 8.12-8.11 (m, 1 H), 7.82 (d, J = 2.0 Hz, 1 H), 7.63-7.62 (m, 1 H), 6.61-6.31 (m, 1 H), 4.56-4.46 (m, 2 H).

### Intermediate 29a

### 6-Bromo-4-chloro-8-(2,2-difluoroethoxy)quinazoline

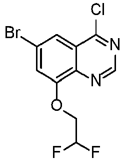

6-Bromo-8-(2,2-difluoroethoxy)quinazolin-4-ol (400 mg, 1.31 mmol) was dissolved on toluene (14 mL) and DIPEA (1.1 mL, 6.56 mmol) was added. The mixture was heated to 90°C for 5 minutes. Phosphorus (V) oxychloride (0.15 mL, 1.57 mmol) was added and the reaction mixture was heated at 90°C for 3 hours. The reaction mixture was concentrated *in vacuo* and the residue partitioned between DCM and sodium hydrogen carbonate. The two phases were separated and the aqueous phase was extracted with DCM (2 × 50 mL). The combined organic phases were concentrated *in vacuo* to give the title compound (502 mg, >100%) as a brown solid.

¹H NMR (400 MHz, CDCl₃): δ 9.10-9.09 (m, 1 H), 8.12-8.10 (m, 1 H), 7.45 (d, J = 1.8 Hz, 1 H), 6.47-6.16 (m, 1 H), 4.53-4.45 (m, 2 H).

### Intermediate 30

### 6-Bromo-8-(2,2-difluoroethoxy)-N-((6-methylpyridazin-3-yl)methyl)quinazolin-4-amine

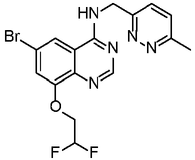

6-Bromo-4-chloro-8-(2,2-difluoroethoxy)quinazoline (424mg, 1.31 mmol) was dissolved in dioxane (6 mL). DIPEA (1.1 mL, 6.55 mmol) and (6-methylpyridazin-3-yl)methanamine (308 mg, 1.57 mmol) were added and the reaction mixture was heated at 95 °C for 3 hours. The mixture was concentrated *in vacuo* and the residue was partitioned between DCM and water. The two phases were separated. The aqueous phase was extracted with DCM (2 × 50 mL). The combined organic phases were concentrated *in vacuo.* The residue was purified by flash chromatography on silica gel eluting with 75% methanol in ethyl acetate to give the title compound (374 mg, 70%) as a light brown solid.

¹H NMR (400 MHz, DMSO): δ 9.05-9.00 (m, 1 H), 8.45-8.44 (m, 1 H), 8.24-8.22 (m, 1 H), 7.55-7.46 (m, 3 H), 6.49-6.47 (m, 1 H), 4.98-4.95 (m, 2 H), 4.57-4.46 (m, 2 H), 2.58 (s, 3 H).

### Example 8

### 8-(2,2-Difluoroethoxy)-N-((6-methylpyridazin-3-yl) methyl)-6-(5-methylpyridin-2-yl) quinazolin-4-amine

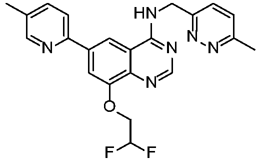

6-Bromo-8-(2,2-difluoroethoxy)-N-((6-methylpyridazin-3-yl)methyl)quinazolin-4-amine (180 mg, 0.590 mmol), [1,1'-bis(diphenylphosphino)ferrocene]dichloropalladium(II), complex with dichloromethane (24 mg, 0.0295 mmol), bis(pinacolato)diboron (180 mg, 0.708 mmol) and potassium acetate (69 mg, 0.708 mmol) were dissolved in dioxane (5 mL) and heated to 100 °C for 2 hours. A further aliquot of 6-Bromo-8-(2,2-difluoroethoxy)-N-((6-methylpyridazin-3-yl)methyl)quinazolin-4-amine (50 mg, 0.122 mmol) was added and the reaction mixture was heated at 100 °C for a further 16 hours. The reaction mixture was allowed to cool to room temperature and the mixture was halved. 2-Bromo-5-methylpyridine (107 mg, 0.620 mmol), cesium carbonate (384 mg, 1.18 mmol), [1,1'-bis(diphenylphosphino)ferrocene]-dichloropalladium(II), complex with dichloromethane (24 mg, 0.0295 mmol) and water (2 mL) were added. The reaction mixture was heated at 100 °C for 2 hours. The reaction mixture was then concentrated *in vacuo* and the residue was partitioned between DCM and water. The two phases were separated and the aqueous phase was extracted with DCM (2 × 50 mL). The combined organic phases were concentrated *in vacuo.* Purification by preparative HPLC afforded the title compound (5 mg, 4%) as a white solid.

¹H NMR (400 MHz, DMSO): δ 9.23 (t, J = 5.7 Hz, 1 H), 8.72-8.70 (m, 1 H), 8.62 (d, J = 2.0 Hz, 1 H), 8.51-8.50 (m, 1 H), 8.18-8.13 (m, 2 H), 7.88-7.84 (m, 1 H), 7.63-7.53 (m, 2 H), 6.72-6.42 (m, 1 H), 5.11-5.07 (m, 2 H), 4.69-4.60 (m, 2 H), 2.65-2.64 (m, 3 H), 2.44-2.43 (m, 3 H). LCMS (Analytical Method 3): [MH⁺] = 423 at 4.01 min.

The following compound reported in the table below was prepared according to the same procedure described for the preparation of 8-(2,2-Difluoroethoxy)-N-((6-methylpyridazin-3-yl) methyl)-6-(5-methylpyridin-2-yl) quinazolin-4-amine (Example 8):

| **Example No.** | **Chemical Name Structure** | **Analytical data ¹H NMR** |
|---|---|---|
| | | **LC-MS** |
| **Example 9** | 8-(2,2-difluoroethoxy)-*N*-[(6-methylpyridazin-3-yl)methyl]-6-(5-methylpyrimidin-2-yl)quinazolin-4-amine 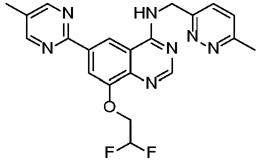 | ¹H NMR (400 MHz, DMSO): δ 9.37 (t, J = 5.8 Hz, 1 H), 9.02 (d, J = 1.5 Hz, 1 H), 8.85 (d, J = 0.6 Hz, 2 H), 8.48-8.48 (m, 1 H), 8.29 (d, J = 1.5 Hz, 1 H), 7.56-7.48 (m, 2 H), 5.02 (d, J = 5.8 Hz, 2 H), 4.65-4.56 (m, 2 H), 3.29 (s, 1 H), 2.60 (s, 3 H), 2.38 (s, 3 H). LCMS (Method 3): [MH⁺] = 424 at 3.37 min. |

### Intermediate 31

### 2-amino-3-(morpholinosulfonyl)benzoic acid

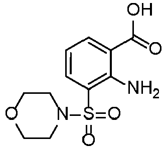

To asolution of morpholine (0.741 ml, 8.47 mmol) in water (50 ml), 3-(chlorosulfonyl)-2-nitrobenzoic acid (0.75 g, 2.82 mmol) was added in 10 min and stirred for 30 min, then, Pd/C 10 % (50% wet) (2.82 mmol) and aqueous hydrogen chloride 2 N aq (0.103 g, 2.82 mmol) were added and the reaction was stirred overnight under hydrogen atmosphere (baloon). The catalyst was filtered off and the solvent removed under reduced pressure. The crude was purified byC18 flash chromatography ((H2O/ACN)) 95:5 +0.1% HCOOH}:{(ACN/H2O) 95:5 + HCOOH 0.1 %} from 100:0 to 0:100 affording 2-amino-3-(morpholinosulfonyl)benzoic acid (0.80 g, 2.79 mmol, 99 % yield) as a brown solid.
LCMS(Method5): 0.75 min, [M+H]+ 287.75

The following compound reported in the table below was prepared *via* adaptation of the above procedure.

| **Intermediate No.** | **Chemical Name Structure** | **Analytical data LC-MS** | **Reagents** |
|---|---|---|---|
| Intermediate 32 | 2-amino-5-bromo-3-(N,N-dimethylsulfamoyl)benzoic acid 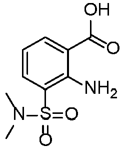 | LCMS(Method5): 0.98 min, [M+H]+ 322.76 | dimethylami ne 2M in THF |
| Intermediate 33 | 2-amino-3-((3,3-difluoropyrrolidin-1-yl)sulfonyl)benzoic acid 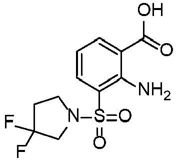 | LCMS(Method5A): 1.55 min, [M+H]+ 306.75 | 3,3-difluoropyrrol idine hydrochloride |
| Intermediate 34 | 2-amino-3-((4-hydroxypiperidin-1-yl)sulfonyl)benzoic acid 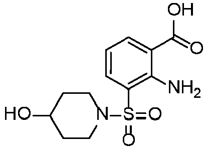 | LCMS(Method5): 0.67 min, 300.99 [M+H]+ | piperidin-4-ol |
| Intermediate 35 | 2-amino-3-(N-methylsulfamoyl)benzoic acid 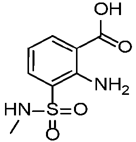 | LCMS(Method5): 0.63 min, [M+H]+ 230.9 | methyl amine solution 2M in THF |
| Intermediate 36 | 2-amino-3-((4-((benzyloxy)carbonyl)piper azin-1-yl)sulfonyl)benzoic acid 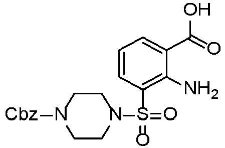 | LCMS(Method5): 1.07 min, [M+H]+ 419.72 | benzyl piperazine-1-carboxylate |

### Intermediate 37

### Preparation of 2-amino-5-bromo-3-(morpholinosulfonyl) benzoic acid

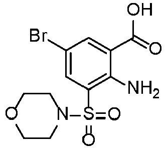

To a solution of 2-amino-3-(morpholinosulfonyl)benzoic acid (0.8 g, 2.79 mmol) (Intermediate 29) in DMF, NBS (0.567 ml, 2.79 mmol) was added and the solution stirred for 30 min. Then, solvent was removed and the crude was purified by C18 flash chromatography ((H₂O/ACN)) 95:5 +0.1% HCOOH}:{(ACN/H₂O) 95:5 + HCOOH 0.1 %} from 100:0 to 0:100 affording 2-amino-5-bromo-3-(morpholinosulfonyl)benzoic acid (0.85 g, 2.328 mmol, 83 % yield) as a white off solid.
LCMS(Method5): 0.98 min, 366.56 [M+H]+

The following compound reported in the table below was prepared *via* adaptation of the above procedure.

| **Intermediate No.** | **Chemical Name Structure** | **Analytical data LC-MS** | **Reagents** |
|---|---|---|---|
| Intermediate 38 | 2-amino-5-bromo-3-(N,N-dimethylsulfamoyl)benzoic acid 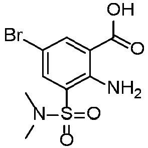 | LCMS(Method5): 0.98 min, [M+H]+ 322.76 | Intermediate 32 |
| Intermediate 39 | 2-amino-5-bromo-3-((3,3-difluoropyrrolidin-1-yl)sulfonyl)benzoic acid 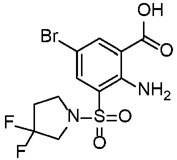 | LCMS(Method5): 1.11 min, [M+H]+ 384.56 | Intermediate 33 |
| Intermediate 40 | 6-bromo-8-((4-hydroxypiperidin-1-yl)sulfonyl)quinazolin-4-ol 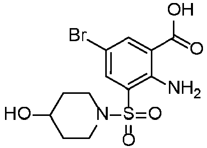 | LCMS(Method5): 0.86 min, 378.96 [M+H]+ | Intermediate 34 |
| Intermediate 41 | 2-amino-5-bromo-3-(N-methylsulfamoyl)benzoic acid 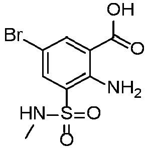 | LCMS(Method5): 0.84 min, [M+H]+ 308.61 | Intermediate 35 |
| Intermediate 42 | 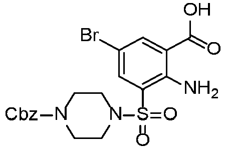 | LCMS(Method5): 1.07 min, [M+H]+ 497.77 | Intermediate 36 |

### Intermediate 43

### 6-bromo-8-(morpholinosulfonyl)quinazolin-4-ol

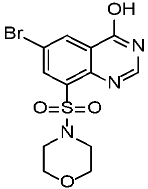

To a solution 2-amino-5-bromo-3-(morpholinosulfonyl)benzoic acid (600 mg, 1.643 mmol) (Intermediate 35) in Formamide (6 ml), ethanesulfonic acid (0.6 ml, 1.643 mmol) was added and the mixture heated to 120 C for 48h. The reaction was directly loaded on C18 cartridge by reverse chromatography ((H₂O/ACN)) 95:5 +0.1% HCOOH}:{(ACN/H₂O) 95:5 + HCOOH 0.1 %} from 100:0 to 0:100 affording 6-bromo-8-(morpholinosulfonyl)quinazolin-4-ol (0.230 g, 0.615 mmol, 37.4 % yield) as a brown solid.
LCMS(Method5): 0.72 min [M+H]+ 375.56

The following compound reported in the table below was prepared *via* adaptation of the above procedure.

| **Intermediate No.** | **Chemical Name Structure** | **Analytical data LC-MS** | **Reagents** |
|---|---|---|---|
| Intermediate 44 | 6-bromo-4-hydroxy-N,N-dimethylquinazoline-8-sulfonamide 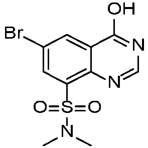 | LCMS(Method5): 0.74 min, [M+H]+ 331.71 | Intermediate 38 |
| Intermediate 45 | 6-bromo-8-((3,3-difluoropyrrolidin-1-yl)sulfonyl)quinazolin-4-ol 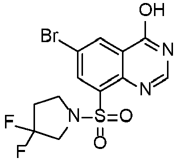 | LCMS(Method5): 0.88 min, [M+H]+ 393.66 | Intermediate 39 |
| Intermediate 46 | 6-bromo-8-((4-hydroxypiperidin-1-yl)sulfonyl)quinazolin-4-ol 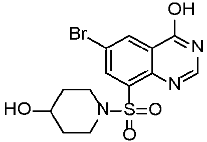 | LCMS(Method5): 0.66 min, 387.95 [M+H]+ | Intermediate 40 |
| Intermediate 47 | 6-bromo-4-hydroxy-N-methylquinazoline-8-sulfonamide 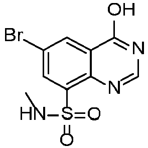 | LCMS(Method5): 0.69 min, [M+H]+ 317.81 | Intermediate 41 |
| Intermediate 48 | benzyl 4-((6-bromo-4-hydroxyquinazolin-8-yl)sulfonyl)piperazine-1-carboxylate 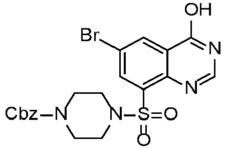 | LCMS(Method5A): 1.05 min, 506.67 [M+H]+ | Intermediate 42 |

### Intermediate 50

### 6-(4-fluorophenyl)-8-(morpholinosulfonyl) quinazolin-4-ol

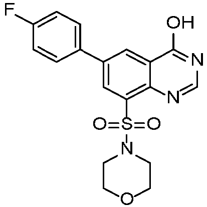

To a suspension of 6-bromo-8-(morpholinosulfonyl)quinazolin-4-ol (250 mg, 0.668 mmol) in Dioxane (10 ml),Water (3 ml), 4-fluorophenylboronic acid (187 mg, 1.336 mmol), K2CO3 (277 mg, 2.004 mmol) PdCl2(dppf) (56 mg, 0.07 mmol)were added and the reaction was stirred overnight. Then, solvent was removed and the crude was purified by C18 flash chromatography ((H2O/ACN)) 95:5 +0.1% HCOOH}:{(ACN/H2O) 95:5 + HCOOH 0.1 %} from 100:0 to 0:100 affording 6-(4-fluorophenyl)-8-(morpholinosulfonyl)quinazolin-4-ol (204 mg, 0.524 mmol, 78 % yield) as a brown solid.
LCMS(Method5): 0.87 min, [M+H]+ 389.81

The following compounds reported in the table below was prepared *via* adaptation of the above procedure.

| **Intermediate No.** | **Chemical Name Structure** | **Analytical data LC-MS** | **Reagents** |
|---|---|---|---|
| Intermediate 51 | 6-(4-fluorophenyl)-4-hydroxy-N,N-dimethylquinazoline-8sulfonamide 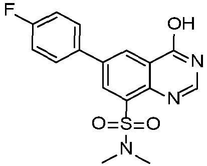 | LCMS(Method5): 0.91 min, [M+H]+ 347.81 | Intermediate 44 |
| Intermediate 52 | 8-((3,3-difluoropyrrolidin-1-yl)sulfonyl)-6-(4-fluorophenyl)quinazolin-4-ol 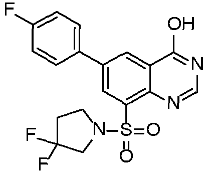 | LCMS(Method5): 1.01 min, 410.02[M+H]+ | Intermediate 45 |
| Intermediate 53 | 6-(4-fluorophenyl)-8-((4-hydroxypiperidin-1-yl)sulfonyl)quinazolin-4-ol 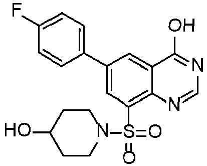 | LCMS(Method5): 0.80 min, 404.32 [M+H]+ | Intermediate 46 |
| Intermediate 54 | 6-(4-fluorophenyl)-4-hydroxy-N-methylquinazoline-8-sulfonamide 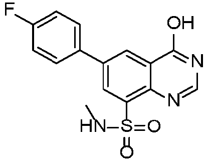 | LCMS(Method5): 0.85 min, [M+H]+ 333.81 | Intermediate 47 |
| Intermediate 55 | benzyl 4-((6-(4-fluorophenyl)-4-hydroxyquinazolin-8-yl)sulfonyl)piperazine-1-carboxylate 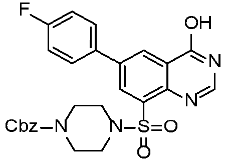 | LCMS(Method5A): 1.13 min, 522.52 [M+H]+ | Intermediate 48 |

### Example 10

### (R)-6-(4-fluorophenyl)-N-(1-(6-methylpyridazin-3-yl)ethyl)-8-(morpholinosulfonyl)quinazolin-4-amine

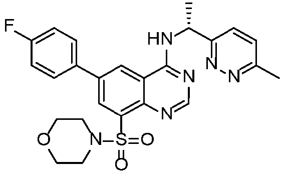

To a solution of 6-(4-fluorophenyl)-8-(morpholinosulfonyl)quinazolin-4-ol (60 mg, 0.154 mmol) in dry DMF (4 ml), PyBOP (96 mg, 0.185 mmol) and DIPEA (0.059 ml, 0.339 mmol) were added and the reaction was stirred for 30 min. Then, ((R)-1-(6-methylpyridazin-3-yl)ethan-1-amine dihydrochloride (32.4 mg, 0.154 mmol) was added and the reaction was further stirred for 30 min. Then, solvent was removed and the crude purified byC18 flash chromatography ((H2O/ACN)) 95:5 +0.1% HCOOH}:{(ACN/H₂O) 95:5 + HCOOH 0.1 %} from 100:0 to 0:100 affording (R)-6-(4-fluorophenyl)-N-(1-(6-methylpyridazin-3-yl)ethyl)-8-(morpholinosulfonyl)quinazolin-4-amine (10 mg, 0.020 mmol, 12.76 % yield) as a yellowish solid.
¹H NMR (400 MHz, *DMSO-d₆)* δ ppm 9.20 (s, 2 H), 9.01 (br d, *J*=6.58 Hz, 1 H), 8.95 (s, 1 H), 8.56 (s, 1 H), 8.54 (s, 1 H), 7.92 (br dd, *J*=8.22, 5.59 Hz, 2 H), 7.43 (br t, *J*=8.66 Hz, 2 H), 5.71 (br quin, *J*=6.80 Hz, 1 H), 4.33 (br t, *J*=4.82 Hz, 2 H), 3.52 - 3.61 (m, 4 H), 3.39 - 3.50 (m, 2 H), 1.75 (br d, *J*=7.02 Hz, 3 H)
LCMS(Method5): 1.58 min, 509.13 [M+H]+

The following compounds reported in the table below was prepared *via* adaptation of the above procedure with the appropriate intermediate.

| **Example No.** | **Chemical name Structure** | **Analytical data ¹H NMR / LC-MS** | **Reagents** |
|---|---|---|---|
| Example 11 | (R)-6-(4-fluorophenyl)-8-(morpholinosulfonyl)-N-(1-(2-(trifluoromethyl)pyrimidin-5-yl)ethyl)quinazolin-4-amine 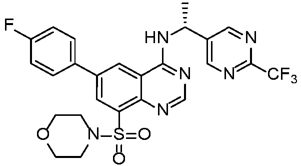 | ¹H NMR (400 MHz, *DMSO-d₆*) δ ppm 9.20 (s, 2 H), 9.01 (br d, *J*=6.58 Hz, 1 H), 8.95 (s, 1 H), 8.56 (s, 1 H), 8.54 (s, 1 H), 7.92 (br dd, *J*=8.22, 5.59 Hz, 2 H), 7.43 (br t, *J*=8.66 Hz, 2 H), 5.71 (br quin, *J*=6.80 Hz, 1 H), 4.33 (br t, *J*=4.82 Hz, 2 H), 3.52 - 3.61 (m, 4 H), 3.39 - 3.50 (m, 2 H), 1.75 (br d, *J*=7.02 Hz, 3 H) | Intermediate 50 |
| | | LCMS (Method5): [MH+] = 509.13 at 1.58 min. | |
| Example 12 | (*Rac*)-6-(4-fluorophenyl)-N-(1-(5-methyl-1,3,4-thiadiazol-2-yl)ethyl)-8-(morpholinosulfonyl)quinazol in-4-amine 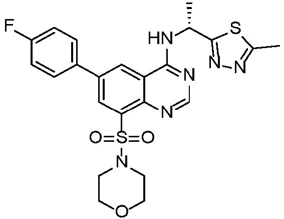 | ¹H NMR (600 MHz, DMSO-d6) δ ppm 9.22 (d, J=7.42 Hz, 1 H) 8.95 (d, J=2.06 Hz, 1 H) 8.66 (s, 1 H) 8.57 (d, J=1.92 Hz, 1 H) 7.92 (dd, J=8.80, 5.36 Hz, 2 H) 7.43 (t, J=8.35 Hz, 2 H) 6.01 (quin, J=7.11 Hz, 1 H) 3.57 - 3.60 (m, 4 H) 2.66 (s, 3 H) 2.52 - 2.55 (m, 4 H) 1.82 (d, J=7.01 Hz, 3 H) | Intermediate 50 |
| | | LCMS (Method5): [MH+] = 515.01 at 0.98 min | |
| Example 13 | (R)-6-(4-fluorophenyl)-N,N-dimethyl-4-((1-(6-methylpyridazin-3-yl)ethyl)amino)quinazoline-8-sulfonamide 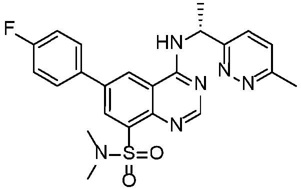 | ¹H NMR (400 MHz, DMSO-d6) δ ppm 9.06 (d, J=7.23 Hz, 1 H) 9.02 (d, J=1.97 Hz, 1 H) 8.54 (d, J=1.97 Hz, 1 H) 8.51 (s, 1 H) 7.93 (dd, J=8.77, 5.26 Hz, 2 H) 7.65 (d, J=8.77 Hz, 1 H) 7.50 (d, J=8.55 Hz, 1 H) 7.43 (t, J=8.77 Hz, 2 H) 5.78 (quin, J=7.07 Hz, 1 H) 2.86 (s, 6 H) 2.58 (s, 3 H) 1.73 (s, 3 H). | Intermediate 51 |
| | | LCMS (Method5): [MH+] = 466.62 at 0.92 min | |
| Example 14 | 6-(4-fluorophenyl)-N,N-dimethyl-4-((1-(5-methyl-1,3,4-thiadiazol-2-yl)ethyl)amino)quinazoline-8-sulfonamide 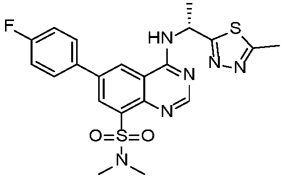 | ¹H NMR (400 MHz, DMSO-d6) δ ppm 9.16 (br d, J=7.45 Hz, 1 H) 8.89 (s, 1 H) 8.62 (s, 1 H) 8.52 (s, 1 H) 7.87 (dd, J=8.44, 5.59 Hz, 2 H) 7.39 (t, J=8.77 Hz, 2 H) 5.96 (quin, J=7.02 Hz, 1 H) 2.84 (s, 6 H) 2.62 (s, 3 H) 1.78 (d, J=7.02 Hz, 3 H) | Intermediate 51 |
| | | LCMS (Method5): [MH+] = 472.7 at 1.0 min | |
| Example 15 | 6-(4-fluorophenyl)-N,N-dimethyl-4-((1-(5-methyl-1,2,4-oxadiazol-3-yl)ethyl)amino)quinazoline-8-sulfonamide 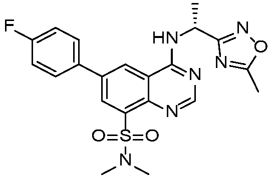 | ¹H NMR (400 MHz, DMSO-*d₆*) δ ppm 9.03 (d, *J*=7.13 Hz, 1 H) 8.95 (d, *J*=1.75 Hz, 1 H) 8.60 (s, 1 H) 8.55 (d, *J=*1.75 Hz, 1 H) 7.91 (dd, J=8.66, 5.37 Hz, 2 H) 7.42 (t, J=8.77 Hz, 2 H) 5.77 (quin, J=7.18 Hz, 1 H) 2.87 (s, 6 H) 2.57 (s, 3 H) 1.69 (d, J=7.02 Hz, 3 H). | Intermediate 51 |
| | | LCMS (Method5): [MH+] = 457.6 at 1.02 min | |
| Example 16 | (R)-8-((3,3-difluoropyrrolidin-1-yl)sulfonyl)-6-(4-fluorophenyl)-N-(1-(6-methylpyridazin-3-yl)ethyl)quinazolin-4-amine 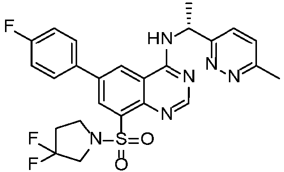 | ¹H NMR (400 MHz, DMSO-d6) δ ppm 9.07 (d, J=7.23 Hz, 1 H), 8.98 - 9.03 (m, 1 H), 8.48 - 8.54 (m, 1 H), 8.44 - 8.51 (m, 1 H), 7.87 - 7.94 (m, 2 H), 7.62 (d, J=8.60 Hz, 1 H), 7.47 (d, J=8.77 Hz, 1 H), 7.40 (t, J=8.88 Hz, 2 H), 5.75 (quin, J=7.13 Hz, 1 H), 4.01 (t, J=13.15 Hz, 2 H), 3.63 (t, J=7.34 Hz, 2 H), 2.55 (d, 3 H), 2.18 - 2.40 (m, 2 H), 1.69 (d, J=7.23 Hz, 3 H) | Intermediate 52 |
| | | LCMS (Method5): [MH+] = 528.7 at 1.06 min | |
| Example 17 | (R)-8-((3,3-difluoropyrrolidin-1-yl)sulfonyl)-6-(4-fluorophenyl)-N-(1-(5-methyl-1,2,4-oxadiazol-3-yl)ethyl)quinazolin-4-amine 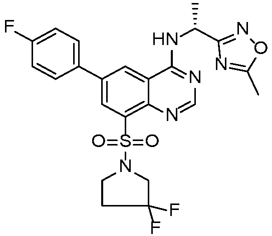 | ¹H NMR (400 MHz, *DMSO-d*6) δ ppm 9.05 (br d, *J*=7.67 Hz, 1 H), 8.94 (d, *J*=1.75 Hz, 1 H), 8.58 (s, 1 H), 8.54 (d, *J*=1.75 Hz, 1 H), 7.89 (dd, *J*=8.66, 5.37 Hz, 2 H), 7.39 (t, *J*=8.77 Hz, 2 H), 5.75 (quin, *J*=7.18 Hz, 1 H), 4.03 (t, *J*=13.04 Hz, 2 H), 3.65 (t, *J=7.23* Hz, 2 H), 2.54 (s, 3 H), 2.19 - 2.39 (m, 2 H), 1.65 (d, *J*=7.02 Hz, 3 H) | Intermediate 52 |
| | | LCMS (Method5): [MH+] = 518.8 at 1.15 min | |
| Example 18 | 8-((3,3-difluoropyrrolidin-1-yl)sulfonyl)-6-(4-fluorophenyl)-N-(1-(5-methyl-1,3,4-thiadiazol-2-yl)ethyl)quinazolin-4-amine 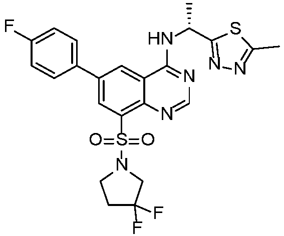 | ¹H NMR (400 MHz, DMSO-d6) δ ppm 9.20 (d, J=7.45 Hz, 1 H) 8.92 (d, J=1.75 Hz, 1 H) 8.63 (s, 1 H) 8.54 (d, J=1.75 Hz, 1 H) 7.84 - 7.91 (m, 2 H) 7.39 (t, J=8.77 Hz, 2 H) 5.97 (m, J=7.10, 7.10 Hz, 1 H) 3.97 - 4.07 (m, 2 H) 3.66 (td, J=7.29, 2.96 Hz, 2 H) 2.62 (s, 3 H) 2.25 - 2.32 (m, 2 H) 1.78 (d, J=7.02 Hz, 3 H) | Intermediate 52 |
| | | LCMS (Method5): [MH+] = 534.97 at 1.11 min | |
| Example 19 | (R)-1-((6-(4-fluorophenyl)-4-((1-(6-methylpyridazin-3-yl)ethyl)amino)quinazolin-8-yl)sulfonyl)piperidin-4-ol 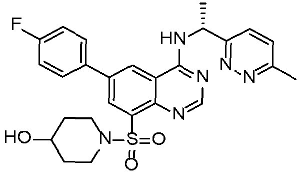 | ¹H NMR (400 MHz, DMSO-d6) δ ppm 8.94 - 9.04 (m, 2 H), 8.50 (d, J=0.88 Hz, 1 H), 8.47 (s, 1 H), 7.90 (dd, J=8.33, 5.48 Hz, 2 H), 7.62 (d, J=8.55 Hz, 1 H), 7.46 (d, J=8.55 Hz, 1 H), 7.39 (t, J=8.77 Hz, 2 H), 5.74 (quin, J=7.02 Hz, 1 H), 4.59 (d, J=3.95 Hz, 1 H), 3.35 - 3.59 (m, 3 H), 2.91 - 3.06 (m, 2 H), 2.55 (s, 3 H), 1.64 - 1.74 (m, 7 H) | Intermediate 53 |
| | | LCMS (Method5): [MH+] = 522.37 at 1.36 min | |
| Example 20 | (R)-1-((6-(4-fluorophenyl)-4-((1-(5-methyl-1,2,4-oxadiazol-3-yl)ethyl)amino)quinazolin-8-yl)sulfonyl)piperidin-4-ol 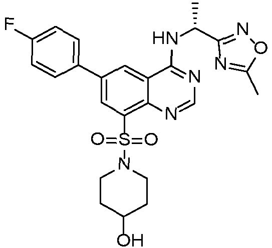 | ¹H NMR (400 MHz, DMSO-d6) δ ppm 8.99 (br d, J=7.45 Hz, 1 H), 8.91 (s, 1 H), 8.55 (s, 1H), 8.51 (s, 1 H), 7.88 (dd, J=8.33, 5.48 Hz, 2 H), 7.38 (t, J=8.77 Hz, 2 H), 5.74 (quin, J=7.18 Hz, 1 H), 4.60 (d, J=4.17 Hz, 1 H), 3.44 - 3.61 (m, 3 H), 2.92 - 3.08 (m, 3 H), 2.53 (s, 3 H), 1.60 - 1.73 (m, 5 H), 1.26 - 1.39 (m, 2 H) | Intermediate 53 |
| | | LCMS (Method5): [MH+] = 512.72 at 1.53 min | |
| Example 21 | (R)-6-(4-fluorophenyl)-N-methyl-4-((1-(6-methylpyridazin-3-yl)ethyl)amino)quinazoline-8-sulfonamide 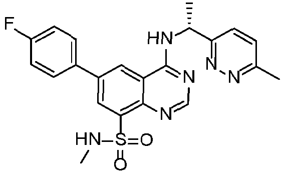 | ¹H NMR (600 MHz, DMSO-d6) δ ppm 9.13 (d, J=7.18 Hz, 1 H) 9.04 (d, J=2.05 Hz, 1 H) 8.52 (s, 1 H) 8.48 (d, J=2.05 Hz, 1 H) 7.94 (dd, J=8.78, 5.32 Hz, 2 H) 7.65 (d, J=8.72 Hz, 1 H) 7.50 (d, J=8.72 Hz, 1 H) 7.43 (t, J=8.85 Hz, 2 H) 6.98 (q, J=5.21 Hz, 1 H) 5.78 (quin, J=7.12 Hz, 1 H) 2.58 (s, 3 H) 2.42 (d, J=5.15 Hz, 3 H) 1.73 (d, J=7.06 Hz, 3 H) | Intermediate 54 |
| | | LCMS (Method5A): [MH+] = 452.92 at 1.61 min | |
| Example 22 | (R)-6-(4-fluorophenyl)-N-methyl-4-((1-(5-methyl-1,2,4-oxadiazol-3-yl)ethyl)amino)quinazoline-8-sulfonamide 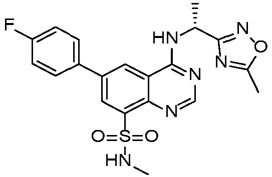 | ¹H NMR (600 MHz, DMSO-d6) δ ppm 9.11 (d, J=7.63 Hz, 1 H), 8.97 (d, J=2.05 Hz, 1 H), 8.60 (s, 1 H), 8.49 (d, J=2.05 Hz, 1 H), 7.92 (t, J=6.71 Hz, 2 H), 7.42 (t, J=8.41 Hz, 2 H), 7.01 (q, J=5.18 Hz, 1 H), 5.78 (quin, J=7.19 Hz, 1 H), 2.57 (s, 3 H), 2.44 (d, J=4.99 Hz, 3 H), 1.69 (d, J=7.04 Hz, 3 H) | Intermediate 54 |
| | | LCMS (Method5A): [MH+] = 442.81 at 1.81 min | |
| Example 23 | (6-(4-fluorophenyl)-N-methyl-4-((1-(5-methyl-1,3,4-thiadiazol-2-yl)ethyl)amino)quinazoline-8-sulfonamide 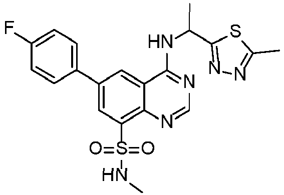 | ¹H NMR (400 MHz, DMSO-d6) δ ppm 9.22 (d, J=7.23 Hz, 1 H), 8.90 (d, J=1.75 Hz, 1 H), 8.62 (s, 1 H), 8.47 (d, J=1.75 Hz, 1 H), 7.88 (dd, J=8.66, 5.37 Hz, 2 H), 7.39 (t, J=8.88 Hz, 2 H), 6.99 (q, J=5.04 Hz, 1 H), 5.96 (t, J=7.13 Hz, 1 H), 2.62 (s, 3 H), 2.39 - 2.43 (m, 3 H), 1.79 (d, J=7.02 Hz, 3 H) | Intermediate 54 |
| | | LCMS (Method): [MH+] = 458.02 at 1.02 min | |

### Example 24

### (R)-6-(4-fluorophenyl)-N-(1-(6-methylpyridazin-3-yl)ethyl)-8-(piperazin-1-ylsulfonyl)quinazolin-4-amine

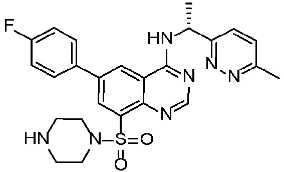

To a solution of benzyl 4-((6-(4-fluorophenyl)-4-hydroxyquinazolin-8-yl)sulfonyl)piperazine-1-carboxylate (100 mg, 0.191 mmol) (Intermediate 55) in dry DMF (5 ml), PyBOP (129 mg, 0.249 mmol) and DIPEA (0.100 ml, 0.574 mmol) were added and the reaction was stirred for 30 min. Then, (R)-1-(6-methylpyridazin-3-yl)ethan-1-amine dihydrochloride (40.2 mg, 0.191 mmol) was added and the reaction was further stirred for 30 min. The solvent was removed and the crude redissolved in dry DCM (5.00 ml) and borontribromide 1 M in DCM (1.914 ml, 1.914 mmol) was added and the reaction was stirred for 1 hr. EtOH (2 mL) was added then volatiles were removed and the crude purified by C18 flash chromatography ((H2O/ACN)) 95:5 +0.1% HCOOH}:{(ACN/H2O) 95:5 + HCOOH 0.1 %} from 100:0 to 0:100 affording (R)-6-(4-fluorophenyl)-N-(1-(6-methylpyridazin-3-yl)ethyl)-8-(piperazin-1-ylsulfonyl)quinazolin-4-amine (7 mg, 0.014 mmol, 7.21 % yield) as a white solid.
1H NMR (400 MHz, DMSO-d6) δ ppm 9.25 (br d, J=6.58 Hz, 1 H), 9.08 (d, J=1.75 Hz, 1 H), 8.64 (br s, 2 H), 8.54 (d, J=1.75 Hz, 1 H), 8.50 (s, 1 H), 7.88 - 7.98 (m, 3 H), 7.76 (d, J=8.77 Hz, 1 H), 7.41 (t, J=8.77 Hz, 2 H), 5.76 (quin, J=6.96 Hz, 1 H), 3.46 - 3.58 (m, 4 H), 3.09 (br s, 4 H), 2.62 (s, 3 H), 1.71 (d, J=7.02 Hz, 3 H)
UPLC PRECLI-WI-0183 minuti rt 4.29 min 508.18 m/z

The following compounds reported in the table below was prepared *via* adaptation of the above procedure.

| **Example No.** | **Chemical name Structure** | **Analytical data ¹H NMR / LC-MS** |
|---|---|---|
| Example 25 | (R)-6-(4-fluorophenyl)-N-(1-(5-methyl-1,2,4-oxadiazol-3-yl)ethyl)-8-(piperazin-1-ylsulfonyl)quinazolin-4-amine 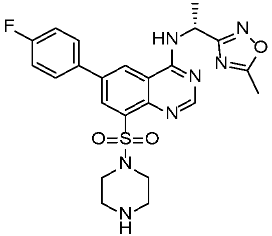 | ¹H NMR (600 MHz, DMSO-d6) δ ppm 9.11 (d, J=7.56 Hz, 1 H), 9.00 (d, J=2.30 Hz, 1 H), 8.62 (s, 1 H), 8.58 (d, J=1.97 Hz, 1 H), 7.93 (t, J=6.62 Hz, 2 H), 7.44 (t, J=8.71 Hz, 1 H), 5.78 - 5.82 (m, 1 H), 3.36 - 3.50 (m, 4 H), 2.97 - 3.06 (m, 4 H), 2.59 (s, 3 H), 1.70 (d, J=6.91 Hz, 3 H) |
| | | LCMS (Method5): [MH+] = 458.02 at 1.02 min |

### Intermediate 56

### Methyl 4-amino-4'-fluoro-[1,1'-biphenyl]-3-carboxylate

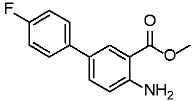

Nitrogen was bubbled for 5 min through a mixture of methyl 2-amino-5-bromobenzoate (2.00 g, 8.69 mmol), 4-fluorophenylboronic acid, pinacol ester (2.90 g, 12.04 mmol), potassium phosphate tribasic (3.69 g, 17.39 mmol), water (3.5 mL) in *N,N-*dimethylformamide (10.5 mL), then [1,1'-bis(diphenylphosphino)ferrocene]-dichloropalladium(II) complex with dichloromethane (710 mg, 0.87 mmol) was added. The resulting mixture was heated to 100 °C for 1.25 hours. After return to room temperature, the reaction was diluted with water (100 mL) and diethyl ether (100 mL) and the organic phase was separated. The aqueous phase was extracted further with diethyl ether (100 mL) and then Ethyl acetate (100 mL). The organic phases were combined, washed with water (100 mL), dried over MgSO₄ and the solvent was removed *in vacuo.* The residue was purified by chromatography on silica gel eluting with 5-35% Ethyl acetate in cyclohexane to give the title compound as an off-white solid (2.08 g, 97%).

¹H NMR (400 MHz, CDCl₃): δ 8.06 (d, J = 2.3 Hz, 1 H), 7.50-7.44 (m, 3 H), 7.08 (dd, J = 8.7, 8.7 Hz, 2 H), 6.74 (d, J = 8.6 Hz, 1 H), 5.78 (s, 2 H), 3.90 (s, 3 H).

### Intermediate 57

### Methyl 4-amino-4'-fluoro-5-iodo-[1,1'-biphenyl]-3-carboxylate

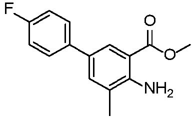

To a solution of methyl 4-amino-4'-fluoro-[1,1'-biphenyl]-3-carboxylate (2.08 g, 8.48 mmol) in dichloromethane (25 mL) was added bis(pyridine)iodonium tetrafluoroborate (4.73 g, 12.72 mmol) and TFA (2.1 mL, 27.42 mmol). The resulting mixture was stirred at room temperature for 2 days. HPLC analysis showed 70 % conversion and further bis(pyridine)iodonium tetrafluoroborate (1.25 g, 3.36 mmol) was added and the stirring was maintained for a further 2.5 hours. The reaction was diluted with dichloromethane (25 mL) and cautiously treated with a solution of NaHCO₃ (7 g, 83 mmol) in water (100 mL). The aqueous layer was collected and further extracted with dichloromethane (2 × 25 mL). The organic phases were combined, washed with an 8 % sodium thiosulphate aqueous solution (100 mL), filtered through a hydrophobic frit and the solvent was removed *in vacuo.* The residue was purified by chromatography on silica gel eluting with 0-25% Ethyl acetate in cyclohexane to give the title compound as an off-white solid (2.71 g, 86%).

¹H NMR (400 MHz, CDCl₃): δ 8.10-8.07 (m, 1 H), 8.04-8.00 (m, 1 H), 7.46-7.41 (m, 2 H), 7.09 (dd, J = 8.6, 8.6 Hz, 2 H), 6.48-6.37 (m, 2 H), 3.91 (s, 3 H).

### Intermediate 58

### 4-amino-4'-fluoro-5-iodo-[1,1'-biphenyl]-3-carboxylic acid

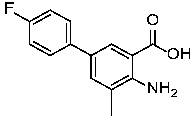

To a solution of methyl 4-amino-4'-fluoro-5-iodo-[1,1'-biphenyl]-3-carboxylate (2.59 g, 6.98 mmol) in 1,4-dioxane (25 mL) and water (5 mL) was added lithium hydroxide monohydrate (1.75 g, 41.87 mmol). The mixture was stirred at room temperature for 18 hours. The reaction was diluted with water (100 mL) and diethyl ether (100 mL) and separated. The aqueous phase was acidified with 1N HCl (45 mL) to pH = 1 and extracted with dichloromethane (3 × 50 mL). The organic phases were combined, filtered through a hydrophobic frit and the solvent was removed *in vacuo* to give the title compound as an off-white solid (2.39 g, 96%).

¹H NMR (400 MHz, DMSO): δ 13.17 (s, 1 H), 8.18 (d, J = 2.0 Hz, 1 H), 8.09 (d, J = 2.3 Hz, 1H), 7.65 (dd, J = 5.4, 8.5 Hz, 2H), 7.27 (dd, J = 8.8, 8.8 Hz, 2 H), 6.84 (s, 2 H).

### Intermediate 59

### 6-(4-fluorophenyl)-8-iodoquinazolin-4(3H)-one

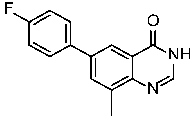

A solution of 4-amino-4'-fluoro-5-iodo-[1,1'-biphenyl]-3-carboxylic acid (2.39 g, 6.69 mmol) in formamide (4 mL) was heated to 130 °C for 16 hours. After return to room temperature, the reaction was diluted with water (20 mL) and stirred for 20 minutes before filtering. The solid was washed with water (3 × 5 mL) then 10% MeOH in diethyl ether (3 × 5 mL) to give the title compound as an off-white solid (2.14 g, 87%).

¹H NMR (400 MHz, DMSO): δ 12.57 (s, 1 H), 8.66 (s, 1 H), 8.36 (s, 1 H), 8.28 (s, 1 H), 7.89 (dd, J = 5.6, 7.8 Hz, 2 H), 7.38 (dd, J = 8.6, 8.6 Hz, 2 H).

### Intermediate 60

### 6-(4-fluorophenyl)-8-(methylthio)quinazolin-4-ol

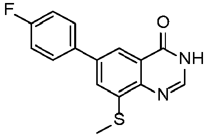

To a solution of 6-(4-fluorophenyl)-8-iodoquinazolin-4-ol (0.5 g, 1.366 mmol) in dry DMSO (5 ml), sodium methanethiolate (0.096 g, 1.366 mmol), COPPER(I) IODIDE (0.260 g, 1.366 mmol), cyclohexane-1,2-diamine (0.156 g, 1.366 mmol) were added and the reaction was stirred overnight at 130 C. The crude was directly loaded on C18 cartridge by reverse chromatography ((H2O/ACN)) 95:5 +0.1% HCOOH}:{(ACN/H₂O) 95:5 + HCOOH 0.1 %} from 100:0 to 0:100 affording 6-(4-fluorophenyl)-8-(methylthio)quinazolin-4-ol (0.25 g, 0.873 mmol, 63.9 % yield) as a brown solid.
LCMS(Method5): 0.96 min, 286.75 [M+H]+

### Example 26

### (R)-6-(4-fluorophenyl)-N-(1-(6-methylpyridazin-3-yl)ethyl)-8-(methylthio) quinazolin-4-amine CRDD456-64-1

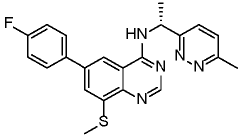

To a solution of 6-(4-fluorophenyl)-8-(methylthio)quinazolin-4-ol (40 mg, 0.140 mmol) (Intermediate 60) in dry DMF (4 ml), PyBOP (95 mg, 0.182 mmol) and DIPEA (0.073 ml, 0.419 mmol) were added and the reaction was stirred for 30 min. Then, (R)-1-(6-methylpyridazin-3-yl)ethan-1-amine dihydrochloride (29.4 mg, 0.140 mmol) was added and the reaction was further stirred for 30 min. Then, solvent was removed and the crude purified by C18 flash chromatography ((H2O/ACN)) 95:5 +0.1% HCOOH}:{(ACN/H2O) 95:5 + HCOOH 0.1 %} from 100:0 to 0:100 affording (R)-6-(4-fluorophenyl)-N-(1-(6-methylpyridazin-3-yl)ethyl)-8-(methylthio)quinazolin-4-amine (6 mg, 0.015 mmol, 10.59 % yield) as a white solid.
¹H NMR (600 MHz, DMSO-d6) δ ppm 8.77 (d, J=7.56 Hz, 1 H), 8.45 (d, J=1.64 Hz, 1 H), 8.40 (s, 1 H), 7.96 (t, J=6.54 Hz, 2 H), 7.68 (d, J=1.64 Hz, 1 H), 7.61 (d, J=8.88 Hz, 1 H), 7.49 (d, J=8.55 Hz, 1 H), 7.39 (t, J=8.88 Hz, 2 H), 5.74 - 5.79 (m, 1 H), 2.58 (s, 3 H), 2.56 (s, 3 H), 1.71 (d, J=7.23 Hz, 3 H)
UPLC PRECLI-WI-0183 minuti rt 5.98 min 406.23 m/z

### Intermediate 61

### 6-(4-fluorophenyl)-8-(methylsulfinyl)quinazolin-4-ol CRDD456-65-1

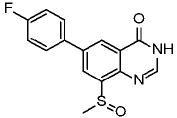

To a solution of 6-(4-fluorophenyl)-8-(methylthio)quinazolin-4-ol (100 mg, 0.349 mmol) in dry DCM (5 ml), mCPBA (60.3 mg, 0.349 mmol) was added and the reaction was stirred for 1 hr. Then, the reaction was diluted in DCM (20 mL) and washed with Na₂SO₃ saturated aqueous solution (20 mL) and NaHCO3 saturated aqueous solution (20 mL). The organic phase was dried over Na2SO4, filtered and the solvent was removed under reduced pressure. The crude was purified by C18 flash chromatography ((H2O/ACN)) 95:5 +0.1% HCOOH}:{(ACN/H₂O) 95:5 + HCOOH 0.1 %} from 100:0 to 0:100 affording6-(4-fluorophenyl)-8-(methylsulfinyl)quinazolin-4-ol (55 mg, 0.182 mmol, 52.1 % yield)
LCMS(Method5): 1.16 min, 302.75 [M+H]+

### Example 27

### 6-(4-fluorophenyl)-N-((R)-1-(6-methylpyridazin-3-yl)ethyl)-8-(methylsulfinyl) quinazolin-4-amine

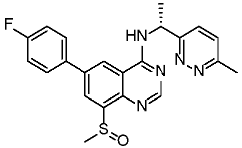

To a solution of 6-(4-fluorophenyl)-8-(methylsulfinyl)quinazolin-4-ol (50 mg, 0.165 mmol) (Intermediate 61) in dry DMF (4 ml), PyBOP (112 mg, 0.215 mmol) and DIPEA (0.087 ml, 0.496 mmol) were added and the reaction was stirred for 30 min. Then, (R)-1-(6-methylpyridazin-3-yl)ethan-1-amine dihydrochloride (38.2 mg, 0.182 mmol) was added and the reaction was further stirred for 30 min. Then, solvent was removed and the crude purified by C18 flash chromatography ((H2O/ACN)) 95:5 +0.1% HCOOH}:{(ACN/H2O) 95:5 + HCOOH 0.1 %} from 100:0 to 0:100 affording 6-(4-fluorophenyl)-N-((R)-1-(6-methylpyridazin-3-yl)ethyl)-8-(methylsulfinyl)quinazolin-4-amine (5 mg, 0.012 mmol, 7.17 % yield) as a white solid.
1H NMR (600 MHz, DMSO-d6) δ ppm 9.05 - 9.10 (m, 1 H), 8.93 (dd, J=5.09, 2.06 Hz, 1 H), 8.45 (d, J=5.22 Hz, 1 H), 8.34 (d, J=1.92 Hz, 1 H), 7.95 (t, J=6.73 Hz, 2 H), 7.65 (d, J=8.80 Hz, 1 H), 7.50 (d, J=8.52 Hz, 1 H), 7.44 (t, J=8.80 Hz, 2 H), 5.75 - 5.81 (m, 1 H), 2.90 - 2.93 (m, 3 H), 2.58 - 2.59 (m, 3 H), 1.73 (d, J=7.01, 3 H)
UPLC PRECLI-WI-0183 minuti rt 5.16 min 422.24 m/z

### Intermediate 61a

### (R)-6-(4-fluorophenyl)-8-iodo-N-(1-(2-(trifluoromethyl)pyrimidin-5-yl)ethyl)quinazolin-4-amine

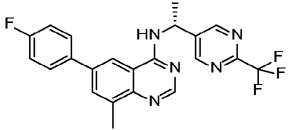

To a solution of 6-(4-fluorophenyl)-8-iodoquinazolin-4-(3H)-one (1.14 g, 3.11 mmol) in N,N-dimethylformamide (10 mL) was successively added (benzotriazol-1-yloxy)tripyrrolidinophosphonium hexafluorophosphate (2.03 g, 1.25 mmol) and di*iso*propylethylamine (2.7 mL, 15.57 mmol). The resulting mixture was heated to 45 °C for one hour then (*R*)-1-(2-(trifluoromethyl)pyrimidin-5-yl)ethan-1-amine hydrochloride (0.96 g, 4.2 mmol) was added and the heating was maintained at 45 °C for 2 hours. After return to room temperature, the mixture was diluted with ethyl acetate (75 mL) and water (175 mL). The organic phase was washed with brine (2 × 20 mL), passed through a hydrophobic frit and the solvent was removed *in vacuo.* The residue was purified by chromatography on silica gel eluting with 0-15% ethyl acetate in dichloromethane to give the title compound as an off-white solid (1.27 g, 75%).

¹H NMR (400 MHz, DMSO): δ 9.18 (s, 2 H), 8.88 (d, J = 6.9 Hz, 1 H), 8.71 (d, J = 1.8 Hz, 1 H), 8.67 (d, J = 1.8 Hz, 1 H), 8.51 (s, 1 H), 7.95-7.90 (m, 2 H), 7.40 (dd, J = 8.9, 8.9 Hz, 2 H), 5.73-5.67 (m, 1 H), 1.75 (d, J = 7.2 Hz, 3 H).

The following compound reported in the table below was prepared *via* adaptation of the above procedure using the appropriate amine.

| **Example No.** | **Chemical name Structure** | **Analytical data ¹H NMR** |
|---|---|---|
| | | **LC-MS** |
| Intermediate **61b** | 6-(4-fluorophenyl)-8-iodo-N-((6-methylpyridazin-3-yl)methyl)quinazolin-4-amine | LCMS (Method 5): 0.96 min, m/z 472.0. |
| | | ¹H NMR (400 MHz, DMSO-d6) δ ppm 9.26 (br t, J = 5.62 Hz, 1 H), 8.64 (s, 2 H), 8.47 (s, 1 H), 7.88 (br dd, J = 8.60, 5.51 Hz, 2 H), 7.53 (d, J = 8.60 Hz, 1 H), 7.45 (d, J = 8.60 Hz, 1 H), 7.34 (br t, J = 8.71 Hz, 2 H), 5.02 (br d, J = 5.29 Hz, 2 H), 2.56 (s, 3 H). |
| | 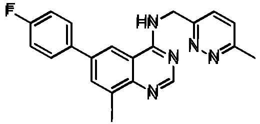 | |

### Example 28

### (R)-6-(4-fluorophenyl)-N-(1-(6-methylpyridazin-3-yl)ethyl)-8-(oxetan-3-ylthio)quinazolin-4-amine

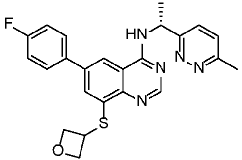

To a solution of (R)-6-(4-fluorophenyl)-8-iodo-N-(1-(6-methylpyridazin-3-yl)ethyl)quinazolin-4-amine (400 mg, 0.824 mmol) (Intermediate 61b) in dry, oxetane-3-thiol (74.3 mg, 0.824 mmol), COPPER(I) IODIDE (7.85 mg, 0.041 mmol), cyclohexane-1,2-diamine (0.020 ml, 0.165 mmol) and potassium phosphate (525 mg, 2.473 mmol) were added and the reaction was stirredovernight at 130 C. The reaction was directly loaded on C18 cartridge by reverse chromatography ((H2O/ACN)) 95:5 +0.1% HCOOH}:{(ACN/H2O) 95:5 + HCOOH 0.1 %} from 100:0 to 0:100 affording (R)-6-(4-fluorophenyl)-N-(1-(6-methylpyridazin-3-yl)ethyl)-8-(oxetan-3-ylthio)quinazolin-4-amine (100 mg, 0.223 mmol, 27.1 % yield) as a brown solid.
UPLC PRECLI-WI-0183 minuti rt 4.22 min 448.25 m/z
¹H NMR (600 MHz, *DMSO-d₆)* δ ppm 8.13 (dd, *J*=2.14, 1.48 Hz, 1 H), 7.90 (s, 1 H), 7.78 (dd, *J*=12.00, 8.71 Hz, 1 H), 7.70 - 7.75 (m, 2 H), 7.67 - 7.69 (m, 1 H), 7.49 (dd, *J*=8.88, 2.96 Hz, 1 H), 7.30 - 7.34 (m, 2 H), 5.68 - 5.75 (m, 1 H), 5.35 - 5.41 (m, 1 H), 4.19 - 4.28 (m, 2 H), 3.50 - 3.66 (m, 2 H), 2.58 (s, 3 H), 1.45 (dd, *J*=6.74, 4.77 Hz, 3 H).

### Example 29

### 6-(4-fluorophenyl)-N-((R)-1-(6-methylpyridazin-3-yl)ethyl)-8-(oxetan-3-ylsulfinyl)quinazolin-4-amine

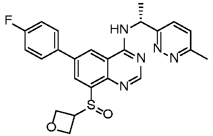

To a solution of (R)-6-(4-fluorophenyl)-N-(1-(6-methylpyridazin-3-yl)ethyl)-8-(oxetan-3-ylthio)quinazolin-4-amine (100 mg, 0.223 mmol) (Example 28) in dry DCM (5 ml), mCPBA (38.6 mg, 0.223 mmol) was added and the reaction was stirred for 1 h. Then, the reaction was diluted in DCM (20 mL) and washed with Na₂SO₃ sat. sol. (20 mL) and NaHCO₃ saturated aqueous solution (20 mL). The organic phase was dried over sodium sulfate, filtered and concentrated under reduced pressure. The crude was purified by C18 flash chromatography ((H2O/ACN)) 95:5 +0.1% HCOOH}:{(ACN/H₂O) 95:5 + HCOOH 0.1 %} from 100:0 to 0:100 affording 6-(4-fluorophenyl)-N-((R)-1-(6-methylpyridazin-3-yl)ethyl)-8-(oxetan-3-ylsulfinyl)quinazolin-4-amine (10 mg, 0.022 mmol, 9.65 % yield) as yellow pale solid.
LCMS(Method5): 0.65 min, 467.27 [M+H]+
¹H NMR (600 MHz, *DMSO-d₆)* δ ppm 8.59 (br s, 1 H), 8.39 (d, *J*=1.65 Hz, 1 H), 8.00 - 8.08 (m, 1 H), 7.75 - 7.89 (m, 3 H), 7.50 (br d, *J*=8.52 Hz, 1 H), 7.36 (t, *J*=8.25 Hz, 2 H), 5.71 - 5.78 (m, 1 H), 5.36 - 5.41 (m, 1 H), 4.18 - 4.22 (m, 2 H), 3.58 - 3.80 (m, 2 H), 2.62 (s, 3 H), 1.47 (dd, *J*=6.74, 3.16 Hz, 3 H).

### Intermediate 62

### (R)-6-(4-Fluorophenyl)-8-((tetrahydro-2H-pyran-4-yl)oxy)-N-(1-(2-(trifluoromethyl) pyrimidin-5-yl)ethyl)quinazolin-4-amine

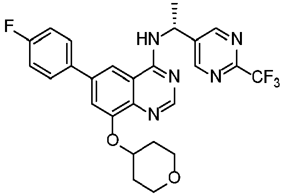

Nitrogen was bubbled for 5 min through a mixture of (*R*)-6-(4-fluorophenyl)-4-((1-(2-(trifluoromethyl)pyrimidin-5-yl)ethyl)amino)quinazolin-8-ol (40 mg, 0.01 mmol) (Intermediate 10), tetrahydro-4-pyranol (10 mg, 0.102 mmol) and cyanomethyltributylphosphorane (1 M in toluene, 0.14 mL, 0.14 mmol) in toluene (3.0 mL). The mixture was heated to 100 °C for 72 hours. After return to room temperature, the solvent was removed *in vacuo.* The residue was purified by preparative HPLC to give the title compound (17 mg, 38%) as an off white solid.

¹H NMR (400 MHz, DMSO): δ 9.18 (s, 2 H), 8.64 (d, J = 7.0 Hz, 1 H), 8.44 (s, 1 H), 8.23 (d, J = 1.6 Hz, 1 H), 7.94-7.89 (m, 2 H), 7.65 (d, J = 1.6 Hz, 1 H), 7.39 (dd, J = 8.8, 8.8 Hz, 2 H), 5.71-5.66 (m, 1 H) 5.02-4.94 (m, 1 H), 3.96-3.88 (m, 2 H), 3.51 (dd, J = 9.7, 9.7 Hz, 2 H), 2.07-2.00 (m, 2 H), 1.74 (d, J = 7.0 Hz, 5 H). LCMS (Method 3): [MH+] = 514 at 3.79 min.

The following compounds reported in the table below were prepared according to the same procedure described for the preparation of (R)-6-(4-Fluorophenyl)-8-((tetrahydro-2H-pyran-4-yl)oxy)-N-(1-(2-(trifluoromethyl) pyrimidin-5-yl)ethyl)quinazolin-4-amine:

| **Example No.** | **Chemical name Structure** | **Analytical data ¹H NMR** |
|---|---|---|
| | | **LC-MS** |
| **Example 30** | (1-((*S*)-3-((6-(4-fluorophenyl)-4-(((*R*)-1-(2-(trifluoromethyl)pyrimidin-5-yl)ethyl)amino)quinazolin-8-yl)oxy)pyrrolidin-1-yl)ethan-1-one | ¹H NMR (400 MHz, DMSO): δ 9.17 (s, 2 H), 8.68 (d, J = 6.8 Hz, 1 H), 8.43 (s, 1 H), 8.27 (dd, J = 2.3, 2.3 Hz, 1 H), 7.97-7.91 (m, 2 H), 7.63 (d, J = 1.5 Hz, 1 H), 7.60 (d, J = 1.5 Hz, 1 H), 7.40 (dd, J = 8.8, 8.8 Hz, 2 H), 5.71-5.66 (m, 1 H), 5.51 (d, J = 3.3 Hz, 1 H), 5.44 (d, J = 3.0 Hz, 1 H), 3.87 (dd, J = 4.5, 11.7 Hz, 1 H), 3.72-3.55 (m, 3 H), 2.29-2.22 (m, 1 H), 2.18-2.11 (m, 1 H), 1.98 (d, J = 18.3 Hz, 3 H), 1.74 (d, J = 7.0 Hz, 3 H). LCMS (Method 4): [MH+] = 541 at 3.52 min. |
| | 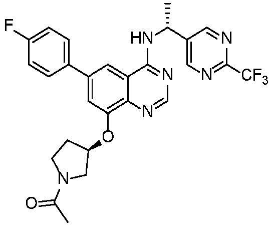 | |
| **Example 31** | (1-((*R*)-3-((6-(4-fₗuorophenyl)-4-(((*R)*-1-(2-(trifluoromethyl)pyrimidin-5-yl)ethyl)amino)quinazolin-8-yl)oxy)pyrrolidin-1-yl)ethan-1-one | ¹H NMR (400 MHz, DMSO): δ 9.18 (s, 2 H), 8.69 (d, J = 6.9 Hz, 1 H), 8.43 (s, 1 H), 8.27 (dd, J = 2.1, 2.1 Hz, 1 H), 7.97-7.91 (m, 2 H), 7.63 (d, J = 1.6 Hz, 1 H), 7.60 (d, J = 1.5 Hz, 1 H), 7.40 (dd, J = 8.8, 8.8 Hz, 2 H), 5.72-5.64 (m, 1 H), 5.51 (d, J = 3.1 Hz,1 H), 5.44 (d, J = 3.1 Hz, 1 H), 3.87 (dd, J = 4.5, 11.7 Hz, 1 H), 3.64 (s, 3 H), 2.27-2.21 (m, 1 H), 2.17-2.09 (m, 1 H), 1.98 (d, J = 14.9 Hz, 3 H), 1.74 (d, J = 7.0 Hz, 3 H). LCMS (Method 4): [MH+] = 541 at 3.57 min. |
| | 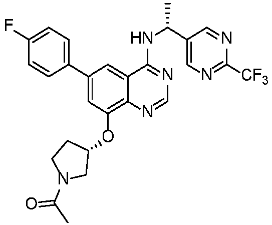 | |
| **Example 32** | *N-*((1*S*,4*s*)-4-((6-(4-fluorophenyl)-4-(((*R*)-1-(2-(trifluoromethyl)pyrimidin-5-yl)ethyl)amino)quinazolin-8-yl)oxy)cyclohexyl)acetamide | ¹H NMR (400 MHz, DMSO): δ 9.18 (s, 2 H), 8.63 (d, J = 7.0 Hz, 1 H), 8.44 (s, 1 H), 8.21 (d, J = 1.4 Hz, 1 H), 7.93-7.86 (m, 3 H), 7.57 (d, J = 1.3 Hz, 1 H), 7.39 (dd, J = 8.9, 8.9 Hz, 2 H), 5.71-5.65 (m, 1 H), 4.95-4.93 (m, 1 H), 3.72-3.69 (m, 1 H), 1.98 (dd, J = 4.5, 9.7 Hz, 2 H), 1.81 (s, 3 H), 1.76-1.60 (m, 6 H), 1.46-1.33 (m, 3 H). LCMS (Method 4): [MH+] = 569 at 3.68 min. |
| | 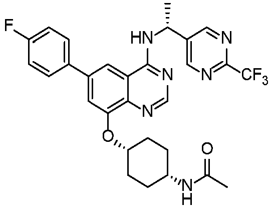 | |
| **Example 33** | 6-(4-fluorophenyl)-8-((1-(2,2,2-trifluoroethyl)pyrrolidin-3-yl)oxy)-*N*-((*R*)-1-(2-(trifluoromethyl)pyrimidin-5-yl)ethyl)quinazolin-4-amine | ¹H NMR (400 MHz, DMSO): δ 9.17 (s, 2 H), 8.65 (d, J = 7.0 Hz, 1 H), 8.43 (s, 1 H), 8.21 (s, 1 H), 7.92 (dd, J = 5.5, 8.8 Hz, 2 H), 7.47 (s, 1 H), 7.40 (dd, J = 8.8, 8.8 Hz, 2 H), 5.71-5.65 (m, 1 H), 5.29 (dd, J = 6.5, 6.5 Hz, 1 H), 3.40 (s, 2 H), 3.20 (dd, J = 6.0, 10.7 Hz, 1 H), 3.02-2.94 (m, 2 H), 2.75 (dd, J = 8.0, 14.6 Hz, 1 H), 2.41-2.31 (m, 1 H), 2.02-1.93 (m, 1 H), 1.74 (d, J = 7.2 Hz, 3 H). LCMS (Method 3): [MH+] = 581 at 5.25 min. |
| | 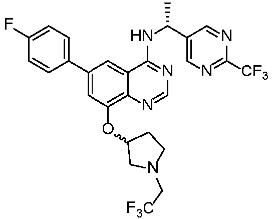 | |
| **Example 34** | 3-((6-(4-fluorophenyl)-4-(((*R*)-1-(2-(trifluoromethyl)pyrimidin-5-yl)ethyl)amino)quinazolin-8-yl)oxy)pyrrolidin-2-one | ¹H NMR (400 MHz, DMSO): δ 9.22 (s, 2 H), 8.74-8.73 (m, 1 H), 8.41 (s, 1 H), 8.26 (s, 1 H), 8.08 (s, 1 H), 7.96-7.92 (m, 2 H), 7.46 (dd, J = 8.9, 8.9 Hz, 2 H), 5.85 (dd, J = 6.7, 6.7 Hz, 1 H), 5.54 (dd, J = 7.3, 7.3 Hz, 1 H), 3.33-3.26 (m, 2 H), 2.70-2.61 (m, 1 H), 2.29-2.19 (m, 1 H), 1.81 (d, J = 7.2 Hz, 3 H). LCMS (Method 4): [MH+] = 513 at 3.55 min. |
| | 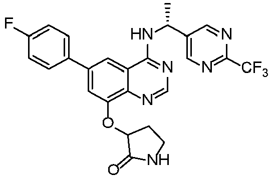 | |
| **Example 35** | *(R)*-1-(4-((6-(4-fluorophenyl)-4-((1-(2-(trifluoromethyl)pyrimidin-5-yl)ethyl)amino)quinazolin-8-yl)oxy)piperidin-1-yl)ethan-1-one | ¹H NMR (400 MHz, DMSO): δ 9.18 (s, 2 H), 8.65 (d, J = 7.0 Hz, 1 H), 8.44 (s, 1 H), 8.25 (d, J = 1.5 Hz, 1 H), 7.95-7.90 (m, 2 H), 7.69 (d, J = 1.3 Hz, 1 H), 7.40 (dd, J = 8.8, 8.8 Hz, 2 H), 5.71-5.66 (m, 1 H), 5.07-5.00 (m, 1 H), 3.89-3.82 (m, 1 H), 3.77-3.71 (m, 1 H), 3.36 (d, J = 56.9 Hz, 2 H), 2.03 (s, 3 H), 2.02-1.97 (m, 1 H), 1.96-1.89 (m, 1 H), 1.76-1.72 (m, 4 H), 1.70-1.59 (m, 1 H). LCMS (Method 3): [MH⁺] = 555 at 4.77 min. |
| | 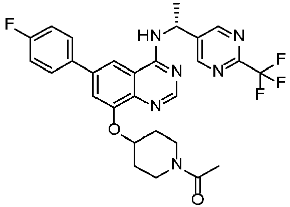 | |
| **Example 36** | (R)-6-(4-fluorophenyl)-8-((1-(2,2,2-trifluoroethyl)piperidin-4-yl)oxy)-N-(1-(2-(trifluoromethyl)pyrimidin-5-yl)ethyl)quinazolin-4-amine | ¹H NMR (400 MHz, DMSO): δ 9.17 (s, 2 H), 8.63 (d, J = 6.9 Hz, 1 H), 8.44-8.44 (m, 1 H), 8.22 (d, J = 1.6 Hz, 1 H), 7.91 (ddd, J = 3.2, 5.4, 12.1 Hz, 2 H), 7.61 (d, J = 1.5 Hz, 1 H), 7.42-7.37 (m, 2 H), 5.72-5.64 (m, 1 H), 4.86-4.79 (m, 1 H), 3.22 (q, J = 10.2 Hz, 2 H), 2.93 (dd, J = 6.0, 11.2 Hz, 2 H), 2.01-1.97 (m, 2 H), 1.77-1.72 (m, 4 H). LCMS (Method 3): [MH⁺] = 595 at 4.77 min. |
| | 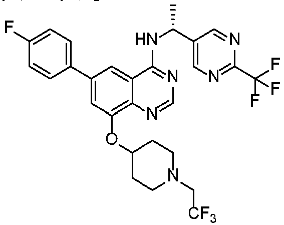 | |
| **Example 37** | (*R*)-3-(((6-(4-fluorophenyl)-4-((1-(2-(trifluoromethyl)pyrimidin-5-yl)ethyl)amino)quinazolin-8-yl)oxy)methyl)oxetan-3-ol | ¹H NMR (400 MHz, DMSO): δ 9.18 (s, 2 H), 8.66 (d, J = 6.9 Hz, 1 H), 8.45 (s, 1 H), 8.26 (d, J = 1.3 Hz, 1 H), 7.95 (dd, J = 5.5, 8.8 Hz, 2 H), 7.69 (d, J = 1.4 Hz, 1 H), 7.40 (dd, J = 8.8, 8.8 Hz, 2 H), 6.25 (s, 1 H), 5.72-5.66 (m, 1 H), 4.57 (dd, J = 6.7, 25.1 Hz, 4 H), 4.41 (s, 2 H), 1.75 (d, J = 7.2 Hz, 3 H). LCMS (Method 3): [MH⁺] = 516 at 4.51 min. |
| | 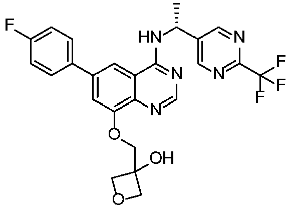 | |

### Intermediate 63

### (R)-8-((3-(benzyloxy)isoxazol-5-yl)methoxy)-6-(4-fluorophenyl)-N-(1-(2-(trifluoromethyl)pyrimidin-5-yl)ethyl)quinazolin-4-amine

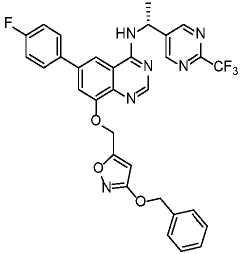

Nitrogen was bubbled for 5 min through a mixture of (R)-6-(4-fluorophenyl)-4-((1-(2-(trifluoromethyl)pyrimidin-5-yl)ethyl)amino)quinazolin-8-ol (95 mg, 0.221 mmol), (3-(benzyloxy)isoxazol-5-yl)methanol (75 mg, 0.265 mmol) and cyanomethyltributylphosphorane (64 mg, 0.265 mmol) in toluene (4.0 mL). The mixture was heated to 100 °C for 72 hours. After return to room temperature, the solvent was removed *in vacuo.* The residue was purified by preparative HPLC to give the title compound (140 mg) as an off white solid which was taken on to the next step without further purification.

### Example 38

### (R)-5-(((6-(4-fluorophenyl)-4-((1-(2-(trifluoromethyl)pyrimidin-5-yl)ethyl)amino)quinazolin-8-yl)oxy)methyl)isoxazol-3-ol

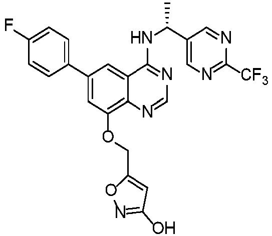

To a mixture of (*R*)-8-((3-(benzyloxy)isoxazol-5-yl)methoxy)-6-(4-fluorophenyl)-*N*-(1-(2-(trifluoromethyl)pyrimidin-5-yl)ethyl)quinazolin-4-amine (140 mg, 0.227 mmol) in acetic acid (1 mL) was added a 48 % solution of hydrogen bromide in water (2 mL). The mixture was heated at 100 °C for 2 hours. After return to room temperature, the reaction was evaporated in vacuo and the residue was purified by preparative HPLC to give the tittle compound (3.2 mg ,2.7 % over two steps) as an off-white solid.

¹H NMR (400 MHz, DMSO): δ 9.18 (s, 2 H), 8.69 (d, J = 6.9 Hz, 1 H), 8.43 (s, 1 H), 8.28 (d, J = 1.4 Hz, 1 H), 7.97-7.92 (m, 2 H), 7.71 (d, J = 1.4 Hz, 1 H), 7.41 (dd, J = 8.9, 8.9 Hz, 2 H), 6.16 (s, 1 H), 5.72-5.66 (m, 1 H), 5.41 (s, 2 H), 1.74 (d, J = 7.2 Hz, 3 H). OH not observed. LCMS (Method 3): [MH+] = 527 at 3.60 min.

### Intermediate 64

### Tert-butyl (R)-2-((6-(4-fluorophenyl)-4-((1-(2-(trifluoromethyl)pyrimidin-5-yl)ethyl)amino)quinazolin-8-yl)oxy)acetate

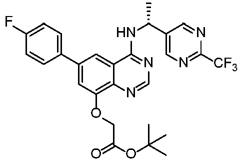

Tert-butyl 2-bromoacetate (0.057 ml, 0.384 mmol) was added to a stirred mixture of (R)-6-(4-fluorophenyl)-4-((1-(2-(trifluoromethyl)pyrimidin-5-yl)ethyl)amino) quinazolin-8-ol (150 mg, 0.349 mmol) (Intermediate 10) and potassium carbonate (155 mg, 1.122 mmol) in DMF (3 ml). Stirring went on at rt for 1 h, then the reaction mixture was diluted with EtOAC and washed with brine (2 X). The organic layer was dried over sodium sulfate, filtered and concentrated under reduced pressure. Purification by chromatography (Biotage Isolera, 10 g KP-Sil cartridge, gradient elution from 0 to 50% EtOAc in dichloromethane in 15 CV) yielded tert-butyl (R)-2-((6-(4-fluorophenyl)-4-((1-(2-(trifluoromethyl)pyrimidin-5-yl)ethyl)amino)quinazolin-8-yl)oxy)acetate (149 mg, 0.274 mmol, 78 % yield) a white powder.

LCMS (Method5 ): 0.97 min, m/z 544.2 [M+H]+,.

### Intermediate 65

### (R)-2-((6-(4-fluorophenyl)-4-((1-(2-(trifluoromethyl)pyrimidin-5-yl)ethyl)amino)quinazolin-8-yl)oxy)acetic acid

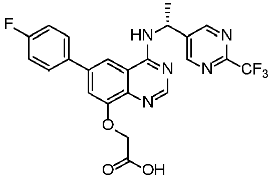

Tert-butyl (R)-2-((6-(4-fluorophenyl)-4-((1-(2-(trifluoromethyl)pyrimidin-5-yl)ethyl)amino)quinazolin-8-yl)oxy)acetate (149 mg, 0.274 mmol) was dissolved in TFA (2 mL). Stirring went on at rt for 16 h, then volatiles were removed under reduced pressure. Trituration in diethyl ether yielded (R)-2-((6-(4-fluorophenyl)-4-((1-(2-(trifluoromethyl)pyrimidin-5-yl)ethyl)amino)quinazolin-8-yl)oxy)acetic acid (134 mg, 0.275 mmol, 100 % yield) as a pale pink powder.
LCMS (Method5 ): 0.81 min, m/z 488.1 [M+H]+,
¹H NMR (400 MHz, DMSO-d6) δ ppm 9.22 (bs, 1 H), 9.16 (s, 2 H), 8.55 (s, 1 H), 8.29 (s, 1 H), 7.87 (dd, J=8.66, 5.37 Hz, 2 H), 7.65 (br s, 1 H), 7.38 (t, J=8.77 Hz, 2 H), 5.75 (quin, J=6.63 Hz, 1 H), 5.07 (s, 2 H), 1.73 (d, J=7.02 Hz, 3 H).

### Example 40

### (R)-2-((6-(4-fluorophenyl)-4-((1-(2-(trifluoromethyl)pyrimidin-5-yl)ethyl)amino)quinazolin-8-yl)oxy)-N-(phenylsulfonyl)acetamide

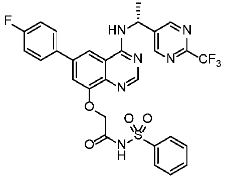

EDCI (67.8 mg, 0.354 mmol) was added to a stirred solution of (R)-2-((6-(4-fluorophenyl)-4-((1-(2-(trifluoromethyl)pyrimidin-5-yl)ethyl)amino)quinazolin-8-yl)oxy)acetic acid (115 mg, 0.236 mmol) (Intermediate 65), DIPEA (205 µl, 1.180 mmol), benzenesulfonamide (55.6 mg, 0.354 mmol), DMAP (43.2 mg, 0.354 mmol) in DMF (2 mL). Stirring went on for 16 h at rt. The reaction mixture was diluted with EtOAc, then formic acid (100 µl, 2.65 mmol) was added. The organic layer was washed with brine, dried over sodium solfate, filtered and concentrated under reduced pressure. Purification by column chromatography (Biotage Isolera, 30 g C18 cartridge, gradient elution from 0 to 60% B in A; A: water/acetonitrile 95:5 + 0.1% HCOOH, B: acetonitrile:water 95:5 +0.1% HCOOH) yielded (R)-2-((6-(4-fluorophenyl)-4-((1-(2-(trifluoromethyl)pyrimidin-5-yl)ethyl)amino)quinazolin-8-yl)oxy)-N-(phenylsulfonyl)acetamide (97.3 mg, 0.155 mmol, 65.8 % yield) as an off-white powder.
LCMS (Method5 ): 0.96 min, 627.2 [M+H]+
¹H NMR (400 MHz, DMSO-d6) δ ppm 9.14 (s, 2 H), 8.97 (br s, 1 H), 8.44 (s, 1 H), 8.24 (s, 1 H), 8.10 (s, 1 H), 7.72 - 7.88 (m, 4 H), 7.30 - 7.55 (m, 6 H), 5.65 - 5.74 (m, 1 H), 4.85 - 4.99 (m, 2 H), 2.64 (br s, 1 H), 2.50 - 1.71 (d, J=7.23 Hz, 3 H)

### Intermediate 66

### Ethyl (R)-1-(((6-(4-fluorophenyl)-4-((1-(2-(trifluoromethyl)pyrimidin-5-yl)ethyl)amino)quinazolin-8-yl)oxy)methyl)cyclopropane-1-carboxylate

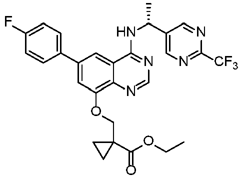

Caesium carbonate (152 mg, 0.466 mmol) was added to a stirred mixture of ethyl 1-(bromomethyl)cyclopropane-1-carboxylate (0.053 ml, 0.256 mmol) and (R)-6-(4-fluorophenyl)-4-((1-(2-(trifluoromethyl)pyrimidin-5-yl)ethyl)amino)quinazolin-8-ol (100 mg, 0.233 mmol) (Intermediate 10) in DMF (2 mL).. The reaction mixture was stirred for 4 h at 60 °C, then it was diluted with AcOEt and washed with brine. The organic layer was dried over sodium sulfate then filtered and concentrated under reduced pressure. Purification by chromatography (Biotage Isolera, 28 g NH cartridge, gradient elution from 0 to 10% ethyl acetate in dichloromethane in 15 CV) yielded ethyl (R)-1-(((6-(4-fluorophenyl)-4-((1-(2-(trifluoromethyl)pyrimidin-5-yl)ethyl)amino)quinazolin-8-yl)oxy)methyl)cyclopropane-1-carboxylate (68.1 mg, 0.123 mmol, 52.6 % yield).
LCMS (Method5 ): 0.96 min, 556.1 m/z [M+H]+

### Example 41

### (R)-1-(((6-(4-fluorophenyl)-4-((1-(2-(trifluoromethyl)pyrimidin-5-yl)ethyl)amino)quinazolin-8-yl)oxy)methyl)cyclopropane-1-carboxylic acid

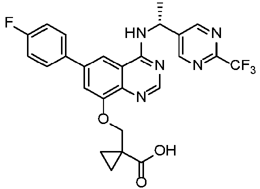

A solution of lithium hydroxide (15.2 mg, 0.635 mmol) in water (1 mL) was added to a solution of ethyl (*R*)-1-(((6-(4-fluorophenyl)-4-((1-(2-(trifluoromethyl)pyrimidin-5-yl)ethyl)amino)quinazolin-8-yl)oxy)methyl)cyclopropane-1-carboxylate (68.1 mg, 0.123 mmol) in THF (3 mL). Stirring went on for 16 h at rt. Volatiles were removed under reduced pressure. Purification by chromatography (Biotage Isolera, 30 g C18 cartridge, gradient elution from 0 to 45% B in A, A: water/acetonitrile 95:5 + 0.1% HCOOH, B: acetonitrile:water 95:5 +0.1% HCOOH in 15 CV) yielded (R)-1-(((6-(4-fluorophenyl)-4-((1-(2-(trifluoromethyl)pyrimidin-5-yl)ethyl)amino)quinazolin-8-yl)oxy)methyl)cyclopropane-1-carboxylic acid (5.1 mg, 9.67 µmol, 7.89 % yield) as an off-white powder.
LCMS (Method5 ): 0.80 min, 527.8 m/z [M+H]+
¹H NMR (400 MHz, DMSO-d6) δ ppm 12.34 - 12.62 (bs, 1 H), 9.12 (s, 2 H), 8.57 (br d, J=7.02 Hz, 1 H), 8.38 (s, 1 H), 8.13 - 8.18 (m, 1 H), 7.85 - 7.95 (m, 2 H), 7.48 (s, 1 H), 7.34 (t, J=8.77 Hz, 2 H), 5.64 (quin, J=6.85 Hz, 1 H), 4.30 (s, 2 H), 1.69 (d, J=7.02 Hz, 3 H), 1.01 - 1.28 (m, 4 H).

### Example 42

### Methyl 1-(2-((6-(4-fluorophenyl)-4-(((6-methylpyridazin-3-yl)methyl)amino)quinazolin-8-yl)oxy)acetamido)cyclopropane-1-carboxylate

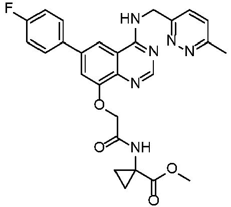

Methyl 1-(2-chloroacetamido)cyclopropane-1-carboxylate (49.6 mg, 0.259 mmol) was added to a stirred mixture of 6-(4-fluorophenyl)-4-(((6-methylpyridazin-3-yl)methyl)amino)quinazolin-8-ol (85 mg, 0.235 mmol) (Intermediate 19) and potassium carbonate (98 mg, 0.706 mmol) in DMF (3 ml). Stirring went on at 80 °C for 16 h, then the reaction mixture was diluted with THF and washed with saturated aqueous ammonium chloride, then with brine. The organic layer was dried over sodium sulfate, filtered and concentrated under reduced pressure. Purification by chromatography (Biotage Isolera, 28 g NH cartridge, gradient elution from 0 to 30% B in A; A: dichloromethane, B: dichloromethane/MeOH 90:10) yielded methyl 1-(2-((6-(4-fluorophenyl)-4-(((6-methylpyridazin-3-yl)methyl)amino)quinazolin-8-yl)oxy)acetamido)cyclopropane-1-carboxylate (44.0 mg, 0.085 mmol, 36.2 % yield) an off-white powder.
LCMS (Method5 ): 0.60 min, m/z 517.2 [M+H]+
¹H NMR (400 MHz, DMSO-d6) δ ppm 9.03 - 9.12 (m, 2 H), 8.43 (s, 1 H), 8.23 - 8.28 (m, 1 H), 7.88 (dd, J=8.77, 5.48 Hz, 2 H), 7.59 - 7.67 (m, 1 H), 7.43 - 7.55 (m, 2 H), 7.35 (t, J=8.88 Hz, 2 H), 5.00 (d, J=5.48 Hz, 2 H), 4.79 (s, 2 H), 3.43 (s, 3 H), 2.56 (s, 3 H), 1.25 - 1.45 (m, 2 H), 0.98 - 1.11 (m, 2 H).

The following compounds were prepared *via* adaptations of the above procedures starting from substrate reported in table.

| **Example No.** | **Structure** | **Analytical data ¹H NMR** | **Reagents** |
|---|---|---|---|
| | | **LC-MS** | |
| Example 43 | Methyl (R)-1-(2-((6-(4-fluorophenyl)-4-((1-(2-(trifluoromethyl)pyri midin-5-yl)ethyl)amino) quinazolin-8-yl)oxy) acetamido) cyclopropane-1-carboxylate | LCMS (Method 5): 0.84 min, m/z 585.1 [M+H]+, | Intermediate 10, Methyl 1-(2-chloroacetami do)cyclopropa ne-1-carboxylate, potassium carbonate, DMF, 80 °C |
| | | 1H NMR (400 MHz, DMSO-d6) δ ppm 9.13 (s, 2 H), 9.02 (s, 1 H), 8.67 (d, J=6.80 Hz, 1 H), 8.43 (s, 1 H), 8.27 (s, 1 H), 7.88 (t, J=5.97 Hz, 2 H), 7.61 (d, J=1.32 Hz, 1 H), 7.37 (t, J=8.88 Hz, 2 H), 5.65 (quin, J=6.85 Hz, 1 H), 4.77 (s, 2 H), 3.42 (s, 3 H), 1.70 (d, J=7.23 Hz, 3 H), 1.28 - 1.39 (m, 2 H), 1.00 - 1.09 (m, 2 H). | |
| | 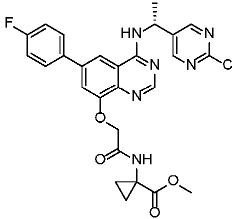 | | |
| Intermediate 67 | ethyl 2-[6-(4-fluorophenyl)-4-[(6-methylpyridazin-3-yl)methylamino]quin azolin-8-yl]oxyacetate | LCMS (Method 7): 2.69 min, [M+H]+ 448.4 ¹H NMR (300 MHz, CHLOROFORM-d) δ ppm 8.69 (s, 1 H), 7.85 (br s, 1 H), 7.50 - 7.61 (m, 4 H), 7.37 - 7.47 (m, 1 H), 7.24 (d, J=0.92 Hz, 1 H), 7.17 (t, J=8.62 Hz, 2 H), 5.10 (d, J=4.03 Hz, 2 H), 5.00 (s, 2 H), 4.30 (q, J=7.15 Hz, 2 H), 2.77 (s, 3 H), 1.29 (t, J=7.15 Hz, 3 H). | Intermediate 19, Ethyl 2-Chloroacetate Cs₂CO₃ |
| | 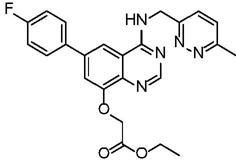 | | |

### Example 44

### (R)-1-(2-((6-(4-fluorophenyl)-4-((1-(2-(trifluoromethyl)pyrimidin-5-yl)ethyl)amino)quinazolin-8-yl)oxy)acetamido)cyclopropane-1-carboxylic acid

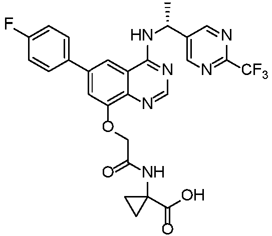

2N LiOH (0.2 ml, 0.400 mmol) was added to a stirred solution of methyl (R)-1-(2-((6-(4-fluorophenyl)-4-((1-(2-(trifluoromethyl)pyrimidin-5-yl)ethyl)amino)quinazolin-8-yl)oxy)acetamido)cyclopropane-1-carboxylate (50.9 mg, 0.087 mmol) (Example 43) in THF (3 ml). Stirring went on for 16 h at 80 °C. Volatiles were removed under reduced pressure then DMF (2 mL) was added to the residue followed by formic acid (20 µl, 0.530 mmol). The resulting suspension was purified by chromatography (Biotage Isolera, 12 g C18 Ultra cartridge, gradient elution from 0 to 35% B in A; A: water/acetonitrile 95:5 + 0.1% HCOOH, B: acetonitrile:water 95:5 +0.1% HCOOH) to yield (R)-1-(2-((6-(4-fluorophenyl)-4-((1-(2-(trifluoromethyl)pyrimidin-5-yl)ethyl)amino)quinazolin-8-yl)oxy)acetamido)cyclopropane-1-carboxylic acid (9.9 mg, 0.017 mmol, 19.93 % yield) as a white powder.
LCMS (Method5 ): 0.77 min, m/z 570.8 [M+H]+
¹H NMR (400 MHz, DMSO-d6) δ ppm 11.90 - 12.82 (bs, 1 H), 9.13 (s, 2 H), 8.90 (s, 1 H), 8.67 (br d, J=7.02 Hz, 1 H), 8.43 (s, 1 H), 8.26 (s, 1 H), 7.88 (dd, J=8.77, 5.48 Hz, 2 H), 7.61 (s, 1 H), 7.35 (t, J=8.77 Hz, 2 H), 5.66 (quin, J=6.80 Hz, 1 H), 4.75 (s, 2 H), 1.70 (d, J=7.23 Hz, 3 H), 1.23 - 1.40 (m, 2 H), 0.90 - 1.03 (m, 2 H).

### Intermediate 67a

### 2-[6-(4-fluorophenyl)-4-[(6-methylpyridazin-3-yl)methylamino]quinazolin-8-yl]oxyacetic acid, sodium salt

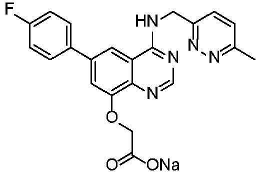

NaOH (10.5 mg, 0.26 mmol) was added to ethyl 2-[6-(4-fluorophenyl)-4-[(6-methylpyridazin-3-yl)methylamino]quinazolin-8-yl]oxyacetate (117 mg, 0.26 mmol) (Intermediate 67) in MeOH (2.2 mL). The mixture was stirred at rt for 3 days then diluted with diethyl ether and filtered to leave 2-[6-(4-fluorophenyl)-4-[(6-methylpyridazin-3-yl)methylamino]quinazolin-8-yl]oxyacetic acid sodium salt (87 mg, 75 % yield) as a white powder.

### Intermediate 68

### Ethyl (1R,2S)-2-(2-((6-(4-fluorophenyl)-4-(((6-methylpyridazin-3-yl)methyl)amino)quinazolin-8-yl)oxy)acetamido)cyclopentane-1-carboxylate

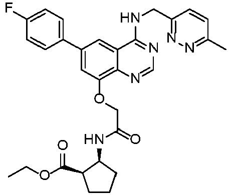

PyAOP (82 mg, 0.157 mmol) was added to a mixture of 2-((6-(4-fluorophenyl)-4-(((6-methylpyridazin-3-yl)methyl)amino)quinazolin-8-yl)oxy)acetic acid (55 mg, 0.131 mmol) (Intermediate 67a), ethyl (1R,2S)-2-aminocyclopentane-1-carboxylate (24.74 mg, 0.157 mmol) and DIPEA (0.069 mL, 0.393 mmol) in THF (3 mL). Stirring went on for 1 h at rt. The reaction was diluted by the addition of AcOEt, then it was washed with saturated aqueous ammonium chloride, saturated aqueous sodium bicarbonate and brine. The organic layer was dried over sodium sulfate, filtered and coincentrated under reduced pressure. Purification by chromatography (Biotage Isolera, 28 g NH cartridge, gradient elution from 0 to 100% EtOAc in dichloromethane in 25 CV) followed by RP chromatography (Biotage Isolera, 12 g C18 Ultra cartridge, gradient elution from 0 to 35% B in A in 25 CV; A: water/acetonitrile 95:5 + 0.1% HCOOH, B: acetonitrile:water 95:5 +0.1% HCOOH) yielded ethyl (1R,2S)-2-(2-((6-(4-fluorophenyl)-4-(((6-methylpyridazin-3-yl)methyl)amino)quinazolin-8-yl)oxy)acetamido)cyclopentane-1-carboxylate (21.4 mg, 0.038 mmol, 29.2 % yield) as an off-white powder.
LCMS (Method5 ): 0.72 min, 559.4 [M+H]+.
¹H NMR (400 MHz, DMSO-d6) δ ppm 9.11 (t, J=5.81 Hz, 1 H), 8.48 (d, J=8.55 Hz, 1 H), 8.41 (s, 1 H), 8.26 (d, J=1.53 Hz, 1 H), 7.84 - 7.91 (m, 2 H), 7.72 (d, J=1.53 Hz, 1 H), 7.49 - 7.53 (m, 1 H), 7.44 - 7.49 (m, 1 H), 7.34 (t, J=8.88 Hz, 2 H), 5.01 (d, J=5.70 Hz, 2 H), 4.70 - 4.79 (m, 2 H), 4.36 - 4.43 (m, 1 H), 3.60 - 3.77 (m, 2 H), 2.89 (q, J=7.31 Hz, 1 H), 2.55 (s, 3 H), 1.71 - 1.90 (m, 4 H), 1.47 - 1.62 (m, 2 H), 0.92 (t, J=7.13 Hz, 3 H).

### Example 45

### (1R,2S)-2-(2-((6-(4-fluorophenyl)-4-(((6-methylpyridazin-3-yl)methyl)amino)quinazolin-8-yl)oxy)acetamido)cyclopentane-1-carboxylic acid

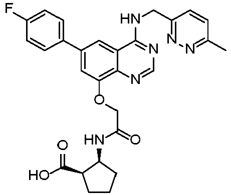

2N NaOH (0.050 mL, 0.100 mmol) was added to a stirred solution of ethyl (1*R*,2*5*)-2-(2-((6-(4-fluorophenyl)-4-(((6-methylpyridazin-3-yl)methyl)amino)quinazolin-8-yl)oxy)acetamido)cyclopentane-1-carboxylate (17.0 mg, 0.030 mmol) (Intermediate 68) in MeOH (2 mL). After 64 h. Volatiles were removed under reduced pressure. Purification by chromatography (Biotage Isolera, 12 g C18 cartridge, gradient elution from 0 to 25% B in A in 30 CV; A: water/acetonitrile 95:5 + 0.1% HCOOH, B: acetonitrile:water 95:5 +0.1% HCOOH) yielded (1R,2S)-2-(2-((6-(4-fluorophenyl)-4-(((6-methylpyridazin-3-yl)methyl)amino)quinazolin-8-yl)oxy)acetamido)cyclopentane-1-carboxylic acid (11.5 mg, 0.022 mmol, 71.2 % yield) as an off-white powder.
LCMS (Method5 ): 0.72min, 559.4 [M+H]+
¹H NMR (400 MHz, DMSO-d6) δ ppm 9.06 - 9.15 (m, 1 H), 8.73 (d, J=7.89 Hz, 1 H), 8.36 - 8.46 (m, 1 H), 8.22 - 8.29 (m, 1 H), 7.78 - 7.91 (m, 2 H), 7.64 - 7.72 (m, 1 H), 7.43 - 7.54 (m, 2 H), 7.29 - 7.38 (m, 2 H), 5.00 (d, J=5.48 Hz, 2 H), 4.70 - 4.78 (m, 2 H), 4.27 (t, J=7.13 Hz, 1 H), 3.45 - 3.65 (m, 1 H), 2.55 (s, 3 H), 1.35 - 1.97 (m, 6 H).

### Example 46

### Ethyl (1S,2R)-2-(2-((6-(4-fluorophenyl)-4-(((6-methylpyridazin-3-yl)methyl)amino)quinazolin-8-yl)oxy)acetamido)cyclopentane-1-carboxylate

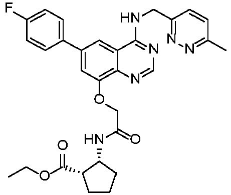

PyAOP (82 mg, 0.157 mmol) was added to a mixture of 2-((6-(4-fluorophenyl)-4-(((6-methylpyridazin-3-yl)methyl)amino)quinazolin-8-yl)oxy)acetic acid (55 mg, 0.131 mmol) (Intermediate 67a), ethyl (1*S*,2*R*)-2-aminocyclopentane-1-carboxylate (25 µL, 0.131 mmol) and DIPEA (68.5 µL, 0.393 mmol) in THF (3 mL). Stirring went on for 1 h at rt. Then formic acid (29.7 µL, 0.787 mmol) was added and volatiles were removed under reduced pressure. Purification by chromatography (Biotage Isolera, 30 g C18 cartridge, gradient elution from 0 to 30% B in A in 30 CV; A: water/acetonitrile 95:5 + 0.1% HCOOH, B: acetonitrile:water 95:5 +0.1% HCOOH) yielded ethyl (1*S*,2*R*)-2-(2-((6-(4-fluorophenyl)-4-(((6-methylpyridazin-3-yl)methyl)amino)quinazolin-8-yl)oxy)acetamido)cyclopentane-1-carboxylate (27.5 mg, 0.049 mmol, 37.5 % yield) as an off-white powder.
LCMS (Method5 ): 0.72 min, 559.4 [M+H]+
¹H NMR (400 MHz, DMSO-d6) δ ppm 9.11 (t, J=5.81 Hz, 1 H), 8.48 (d, J=8.55 Hz, 1 H), 8.41 (s, 1 H), 8.26 (d, J=1.53 Hz, 1 H), 7.84 - 7.91 (m, 2 H), 7.72 (d, J=1.53 Hz, 1 H), 7.49 - 7.53 (m, 1 H), 7.44 - 7.49 (m, 1 H), 7.34 (t, J=8.88 Hz, 2 H), 5.01 (d, J=5.70 Hz, 2 H), 4.70 - 4.79 (m, 2 H), 4.36 - 4.43 (m, 1 H), 3.60 - 3.77 (m, 2 H), 2.89 (q, J=7.31 Hz, 1 H), 2.55 (s, 3 H), 1.71 - 1.90 (m, 4 H), 1.47 - 1.62 (m, 2 H), 0.92 (t, J=7.13 Hz, 3 H).

### Intermediate 69

### 1-(tert-butyl) 4-ethyl (R)-4-(((6-(4-fluorophenyl)-4-((1-(2-(trifluoromethyl)pyrimidin-5-yl)ethyl)amino)quinazolin-8-yl)oxy)methyl)piperidine-1,4-dicarboxylate

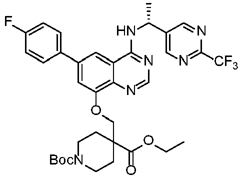

Caesium carbonate (152 mg, 0.466 mmol) was added to a stirred mixture of 1-(tert-butyl) 4-ethyl 4-(iodomethyl)piperidine-1,4-dicarboxylate (102 mg, 0.256 mmol) and (*R*)-6-(4-fluorophenyl)-4-((1-(2-(trifluoromethyl)pyrimidin-5-yl)ethyl)amino)quinazolin-8-ol (100 mg, 0.233 mmol) (Intermediate 10) in DMF (2 mL). Stirring went on at for 5 h at 100°C. The reaction mixture was diluted with AcOEt and washed with brine. The organic layer was dried over sodium sulfate then filtered and concentrated under reduced pressure. Purification by chromatography yielded 1-(tert-butyl) 4-ethyl (*R*)-4-(((6-(4-fluorophenyl)-4-((1-(2-(trifluoromethyl)pyrimidin-5-yl)ethyl)amino)quinazolin-8-yl)oxy)methyl)piperidine-1,4-dicarboxylate (39.9 mg, 0.057 mmol, 24.52 % yield).
LCMS (Method5 ): 1.07 min, 698.8 m/z [M+H]+

### Intermediate 70

### (R)-1-(tert-butoxycarbonyl)-4-(((6-(4-fluorophenyl)-4-((1-(2-(trifluoromethyl)pyrimidin-5-yl)ethyl)amino)quinazolin-8-yl)oxy)methyl)piperidine-4-carboxylic acid

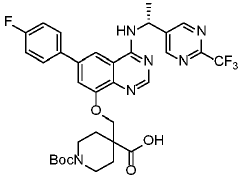

A solution of lithium hydroxide (140 mg, 5.85 mmol) in water (1 mL) was added to a solution of 1-(tert-butyl) 4-ethyl (*R*)-4-(((6-(4-fluorophenyl)-4-((1-(2-(trifluoromethyl)pyrimidin-5-yl)ethyl)amino)quinazolin-8-yl)oxy)methyl)piperidine-1,4-dicarboxylate (39.9 mg, 0.057 mmol) in THF (3 mL). Stirring went on for 16 h at rt. Formic acid (300 µl, 7.95 mmol)was added and the volatiles were removed under reduced pressure. Purification by chromatography (Biotage Isolera, 30 g C18 cartridge, gradient elution from 0 to 55% B in A, A: water/acetonitrile 95:5 + 0.1% HCOOH, B: acetonitrile:water 95:5 +0.1% HCOOH in 15 CV) yielded (*R*)-1-(tert-butoxycarbonyl)-4-(((6-(4-fluorophenyl)-4-((1-(2-(trifluoromethyl)pyrimidin-5-yl)ethyl)amino)quinazolin-8-yl)oxy)methyl)piperidine-4-carboxylic acid (17.9 mg, 0.027 mmol, 46.7 % yield) as an off-white powder.
LCMS (Method5 ): 0.96 min, 670.8 m/z [M+H]+,
¹H NMR (400 MHz, DMSO-d6) δ ppm 11.91 - 13.22 (bs, 1 H), 9.12 (s, 2 H), 8.56 (br d, J=6.80 Hz, 1 H), 8.38 (s, 1 H), 8.17 (s, 1 H), 7.90 (dd, J=8.66, 5.59 Hz, 2 H), 7.53 (s, 1 H), 7.34 (t, J=8.88 Hz, 2 H), 5.63 (br t, J=6.91 Hz, 1 H), 4.28 (s, 2 H), 3.66 (br d, J=13.59 Hz, 2 H), 2.94 - 3.20 (m, 2 H), 2.01 (br d, J=13.81 Hz, 2 H), 1.69 (d, J=7.02 Hz, 3 H), 1.57 - 1.66 (m, 2 H), 1.36 (s, 9 H).

### Example 47

### (R)-4-(((6-(4-fluorophenyl)-4-((1-(2-(trifluoromethyl)pyrimidin-5-yl)ethyl)amino)quinazolin-8-yl)oxy)methyl)piperidine-4-carboxylic acid hydrochloride

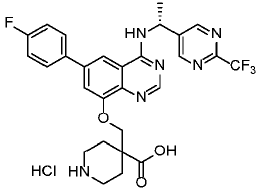

37% HCl (0.5 mL, 6.00 mmol) was added to a mixture of (*R*)-1-(tert-butoxycarbonyl)-4-(((6-(4-fluorophenyl)-4-((1-(2-(trifluoromethyl)pyrimidin-5-yl)ethyl)amino)quinazolin-8-yl)oxy)methyl)piperidine-4-carboxylic acid (12.3 mg, 0.018 mmol) (intermediate 65) in 1 M HCl in AcOEt (2 mL, 2.000 mmol). Stirring went on for 48 h at rt. Volatiles were removed under reduced pressure. Purification by RP chromatography (Biotage Isolera, 12 g C18 cartridge, gradient elution from 0 to 25% B in A, A: water/acetonitrile 95:5 + 0.1% conc HCl, B: acetonitrile:water 95:5 + 0.1% conc HCl in 15 CV) yielded (R)-4-(((6-(4-fluorophenyl)-4-((1-(2-(trifluoromethyl)pyrimidin-5-yl)ethyl)amino)quinazolin-8-yl)oxy)methyl)piperidine-4-carboxylic acid hydrochloride (6.1 mg, 10.05 µmol, 54.8 % yield).
LCMS (Method5 ): 0.59 min, 570.8 m/z [M+H]+
¹H NMR (400 MHz, DMSO-d6) □ ppm 13.9 (br s, 0.5 H), 13.1 (br s, 1 H), 10.7 (br s, 0.5 H), 9.19 (br s, 2 H), 9.05 - 8.30 (m, 4 H), 8.10 - 7.70 (m, 3 H), 7.39 (t, J=8.66 Hz, 2 H), 6.00 - 5.66 (m, 1 H), 4.56 - 4.30 (m, 2 H), 3.33 (m, 2 H), 3.01 (m, 2 H), 2.36 - 2.08 (m, 4 H), 1.78 (br d, J=5.26 Hz, 3 H).

### Example 48

### (R)-1-(((6-(4-fluorophenyl)-4-((1-(2-(trifluoromethyl)pyrimidin-5-yl)ethyl)amino)quinazolin-8-yl)oxy)methyl)cyclohexane-1-carboxylic acid

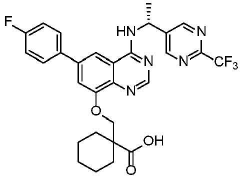

### Intermediate 71

### Methyl (R)-1-(((6-(4-fluorophenyl)-4-((1-(2-(trifluoromethyl)pyrimidin-5-yl)ethyl)amino)quinazolin-8-yl)oxy)methyl)cyclohexane-1-carboxylate

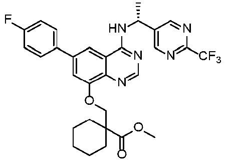

Methyl 1-(bromomethyl)cyclohexane-1-carboxylate (90 mg, 0.384 mmol) was added to a stirred mixture of (*R*)-6-(4-fluorophenyl)-4-((1-(2-(trifluoromethyl)pyrimidin-5-yl)ethyl)amino)quinazolin-8-ol (150 mg, 0.349 mmol) (Intermediate 10), sodium iodide (155 mg, 1.034 mmol) and cesium carbonate (240 mg, 0.737 mmol) in DMF (3 ml). Stirring went on at 110°C for 16 h, then the reaction mixture was allowed to cool down to rt. The reaction mixture was diluted with EtOAc and washed with saturated aqueous ammonium chloride, then with brine. The organic layer was dried over sodium sulfate, filtered and concentrated under reduced pressure. Purification by chromatography (Biotage Isolera, 28 g NH cartridge, elution in dichloromethane 15 CV) yielded methyl *(R)-1-(((6-(4-fluorophenyl)-4-((1-(2-(trifluoromethyl)pyrimidin-5-*yl)ethyl)amino)quinazolin-8-yl)oxy)methyl)cyclohexane-1-carboxylate (50.9 mg, 0.087 mmol, 24.97 % yield) a pale yellow powder.
LCMS (Method5 ): 1.02 min, m/z 583.9 [M+H]+

### Preparation of (R)-1-(((6-(4-fluorophenyl)-4-((1-(2-(trifluoromethyl)pyrimidin-5-yl)ethyl)amino)quinazolin-8-yl)oxy)methyl)cyclohexane-1-carboxylic acid (Example 48)

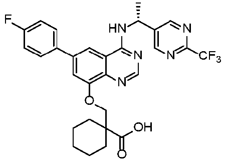

A solution of lithium hydroxide (40 mg, 1.670 mmol) in water (1 mL) was added to a solution of methyl (*R*)-1-(((6-(4-fluorophenyl)-4-((1-(2-(trifluoromethyl)pyrimidin-5-yl)ethyl)amino)quinazolin-8-yl)oxy)methyl)cyclohexane-1-carboxylate (50.9 mg, 0.087 mmol) in THF (3 ml). Stirring went on for 48 h at 70 °C. The reaction mixture was diluted with acetonitrile and volatiles were removed under reduced pressure. Purification by chromatography (Biotage Isolera, 30 g C18 cartridge, gradient elution from 0 to 20% B in A, A: water/acetonitrile 95:5 + 0.1% HCOOH, B: acetonitrile:water 95:5 +0.1% HCOOH in 15 CV) yielded (*R*)-1-(((6-(4-fluorophenyl)-4-((1-(2-(trifluoromethyl)pyrimidin-5-yl)ethyl)amino)quinazolin-8-yl)oxy)methyl)cyclohexane-1-carboxylic acid (44.1 mg, 0.077 mmol, 89 % yield) as a pale beige powder.
LCMS (Method5 ): 0.98 min, 570.2 m/z [M+H]+
¹H NMR (400 MHz, DMSO-d6) δ ppm 12.16 - 12.43 (bs, 1 H), 9.12 (s, 2 H), 8.56 (br s, 1 H), 8.39 (s, 1 H), 8.16 (s, 1 H), 7.90 (dd, J=8.77, 5.48 Hz, 2 H), 7.52 (s, 1 H), 7.34 (t, J=8.77 Hz, 2 H), 5.63 (quin, J=6.96 Hz, 1 H), 4.23 (s, 2 H), 2.00 (br dd, J=11.51, 4.49 Hz, 1 H), 1.95 - 2.06 (m, 2 H), 1.69 (d, J=7.23 Hz, 3 H), 1.38 - 1.57 (m, 6 H), 1.11 - 1.38 (m, 2 H).

### Intermediate 72

### 2-((4-(((6-methylpyridazin-3-yl)methyl)amino)-6-(5-methylpyrimidin-2-yl)quinazolin-8-yl)oxy)acetic acid dihydrochloride

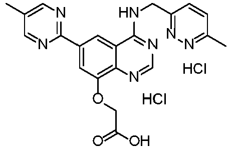

### Step 1: Preparation of tert-butyl 2-((4-(((6-methylpyridazin-3-yl)methyl)amino)-6-(5-methylpyrimidin-2-yl)quinazolin-8-yl)oxy)acetate

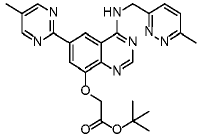

Tert-butyl 2-bromoacetate (247 µL, 1.669 mmol) was added to a stirred mixture of 4-(((6-methylpyridazin-3-yl)methyl)amino)-6-(5-methylpyrimidin-2-yl)quinazolin-8-ol (500 mg, 1.391 mmol) (intermediate 57) and potassium carbonate (456 mg, 3.30 mmol) in DMF/THF 2:1 (9 mL). Stirring went on at rt for 16 h. The reaction was quenched by the addition of formic acid (300 µL, 7.95 mmol) and volatiles were removed under reduced pressure. Purification by RP chromatography (Biotage Isolera, 60 g C18 cartridge, gradient elution from 0 to 30% B in A; A: water/acetonitrile 95:5 + 0.1% HCOOH, B: acetonitrile/water 95:5 + 0.1% HCOOH) yielded tert-butyl 2-((4-(((6-methylpyridazin-3-yl)methyl)amino)-6-(5-methylpyrimidin-2-yl)quinazolin-8-yl)oxy)acetate (451 mg, 0.952 mmol, 68.5 % yield) as an orange wax.
LCMS (Method5 ): 0.66 min, 473.8 [M+H]+

### Step 2: Preparation of 2-((4-(((6-methylpyridazin-3-yl)methyl)amino)-6-(5-methylpyrimidin-2-yl)quinazolin-8-yl)oxy)acetic acid dihydrochloride

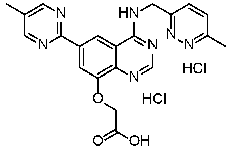

Tert-butyl 2-((4-(((6-methylpyridazin-3-yl)methyl)amino)-6-(5-methylpyrimidin-2-yl)quinazolin-8-yl)oxy)acetate was stirred in 4N HCl in 1,4-dioxane (20 ml, 80 mmol) for 16 h at rt. Volatiles were removed under reduced pressure to yield 2-((4-(((6-methylpyridazin-3-yl)methyl)amino)-6-(5-methylpyrimidin-2-yl)quinazolin-8-yl)oxy)acetic acid dihydrochloride (552 mg, 1.126 mmol, 81 % yield) as a pale yellow powder.
LCMS (Method5 ): 0.45 min, 418.0 [M+H]+,
¹H NMR (400 MHz, DMSO-d6) δ ppm 11.20 (br t, J=5.48 Hz, 1 H), 9.18 (s, 1 H), 8.88 (s, 2 H), 8.80 (s, 1 H), 8.41 (s, 1 H), 7.77 (d, J=8.55 Hz, 1 H), 7.65 (d, J=8.55 Hz, 1 H), 5.16 - 5.28 (m, 4 H), 2.63 (s, 3 H), 2.38 (s, 3 H)

### Example 49

### Ammonium (2-((4-(((6-methylpyridazin-3-yl)methyl)amino)-6-(5-methylpyrimidin-2-yl)quinazolin-8-yl)oxy)acetyl)(phenylsulfonyl)amide

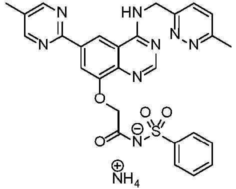

EDCI (58.6 mg, 0.306 mmol) was added to a stirred solution of 2-((4-(((6-methylpyridazin-3-yl)methyl)amino)-6-(5-methylpyrimidin-2-yl)quinazolin-8-yl)oxy)acetic acid dihydrochloride (100 mg, 0.204 mmol) (Intermediate 72, benzenesulfonamide (48.1 mg, 0.306 mmol), DMAP (100 mg, 0.816 mmol) and DIPEA (0.355 mL, 2.039 mmol) in DMF (2 mL). Stirring went on for 16 h at rt. The reaction was quenched by the addition of formic acid (0.115 mL, 3.06 mmol)., then volatiles were removed under reduced pressure. Purification by RP chromatography (Biotage Isolera, 12 g C18 cartridge, gradient elution from 0 to 25% B in A; A: water/MeOH 95:5 + 0.1% conc. ammonia, B: MeOH/water 95:5 +0.1% conc. ammonia) yielded ammonium (2-((4-(((6-methylpyridazin-3-yl)methyl)amino)-6-(5-methylpyrimidin-2-yl)quinazolin-8-yl)oxy)acetyl)(phenylsulfonyl)amide (34.8 mg, 0.061 mmol, 29.7 % yield) as a white powder.
LCMS (Method5 ): 0.64 min, 557.1 [M+H]+,
1H NMR (400 MHz, DMSO-d6) δ ppm 9.34 (br s, 1 H), 8.84 (s, 1 H), 8.80 (s, 2 H), 8.39 (s, 1 H), 8.03 (s, 1 H), 7.72 (d, J=7.67 Hz, 2 H), 7.40 - 7.50 (m, 2 H), 7.24 - 7.34 (m, 1 H), 7.18 (br t, J=7.45 Hz, 4 H), 4.98 (br d, J=5.26 Hz, 2 H), 4.60 (s, 2 H), 2.55 (s, 3 H), 2.35 (s, 3 H)

### Intermediate 73

### 2-((6-(4-fluorophenyl)-4-(((1-methylpiperidin-4-yl)methyl)amino)quinazolin-8-yl)oxy)acetic acid hydrochloride

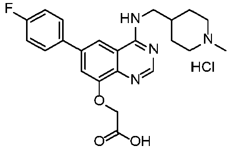

### Step 1: Preparation of tert-butyl (6-(4-fluorophenyl)-8-hydroxyquinazolin-4-yl)((1-methylpiperidin-4-yl)methyl)carbamate

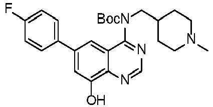

A solution of di-tert-butyl dicarbonate (1.641 mL, 7.14 mmol) in THF (10 mL) was added to a stirred solution of 6-(4-fluorophenyl)-4-(((1-methylpiperidin-4-yl)methyl)amino)quinazolin-8-ol (1.19 g, 3.25 mmol) (Intermediate 5) and DIPEA (1.244 mL, 7.14 mmol) in THF (20 mL). 4-dimethylamino pyridine (0.079 g, 0.649 mmol) was then added and stirring went on for 16 h at rt. LCMS showed complete conversion to tert-butyl (8-((tert-butoxycarbonyl)oxy)-6-(4-fluorophenyl)quinazolin-4-yl)((1-methylpiperidin-4-yl)methyl)carbamate. Volatiles were removed under reduced pressure and the residue was partitioned between EtOAc/THF 1:1 and saturated aqueous ammonium chloride. The layers were separated and the organic layer was washed with brine, dried over sodium sulfate, filtered and concentrated under reduced pressure. The crude material was redissolved in dichloromethane (20 mL) and treated with 2.0 M dimethylamine in THF (4 mL). Stirring went on at rt for 4 h, then volatiles were removed under reduced pressure. Purification by column chromatography (Biotage Isolera, 55 g NH cartridge, gradient elution from 0 to 30% EtOH in dichloromethane) yielded tert-butyl (6-(4-fluorophenyl)-8-hydroxyquinazolin-4-yl)((1-methylpiperidin-4-yl)methyl)carbamate (398 mg, 0.853 mmol, 26.3 % yield) as an orange powder.
LCMS (Method5 ): 1.32 min, 467.4 [M+H]+.

### Step 2: preparation of 2-((6-(4-fluorophenyl)-4-(((1-methylpiperidin-4-yl)methyl)amino)quinazolin-8-yl)oxy)acetic acid hydrochloride (Intermediate 73)

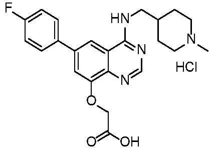

Tert-butyl 2-bromoacetate (0.075 mL, 0.509 mmol) was added to a stirred suspension of potassium carbonate (147 mg, 1.061 mmol) and tert-butyl (6-(4-fluorophenyl)-8-hydroxyquinazolin-4-yl)((1-methylpiperidin-4-yl)methyl)carbamate (198 mg, 0.424 mmol) in DMF (2 mL). Stirring went of for 16 h at rt. The reaction mixture was diluted with EtOAc and washed with brine (3 x). The organic layer was dried over sodium sulfate, filtered and concentrated under reduced pressure. Purification by column chromatography (Biotage Isolera, 28 g NH cartridge, gradient elution from 0 to 20% AcOEt in dichloromethane) yielded the intermediate tert-butyl 2-((4-((tert-butoxycarbonyl)((1-methylpiperidin-4-yl)methyl)amino)-6-(4-fluorophenyl)quinazolin-8-yl)oxy)acetate. This material was dissolved rt in 1,4-dioxane (3 mL) then treated with 4N HCl for 18h at rt. Removal of volatiles under reduced pressure yielded 2-((6-(4-fluorophenyl)-4-(((1-methylpiperidin-4-yl)methyl)amino)quinazolin-8-yl)oxy)acetic acid hydrochloride (88.1 mg, 0.191 mmol, 45.0 % yield) as a pale yellow powder.
LCMS (Method5 ): 0.33 min, 425.2 [M+H]+
¹H NMR (400 MHz, DMSO-d6) δ ppm 13.95 - 14.23 (bs, 1 H), 10.65 - 10.55 (bs, 1 H), 10.11 - 10.18 (m, 1 H), 8.72 (s, 1 H), 8.53 (s, 1 H), 7.95 (br dd, J=8.33, 5.48 Hz, 2 H), 7.87 (s, 1 H), 7.38 (t, J=8.77 Hz, 2 H), 5.19 (s, 2 H), 3.60 - 3.50 (m, 2 H), 3.17 - 3.34 (br s, 3H), 2.74 - 2.90 (m, 2 H), 2.67 (s, 3 H), 2.50 - 2.53 (m, 1 H), 1.97 - 2.05 (m, 1 H), 1.90 (br d, J=13.37 Hz, 2 H), 1.40 - 1.61 (m, 2 H)

### Intermediate 74

### (3-Methyl-1,2,4-oxadiazol-5-yl)methyl methanesulfonate

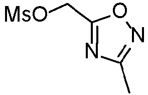

A solution of (3-methyl-1,2,4-oxadiazol-5-yl)methanol (300 mg, 2.63 mmol) and triethylamine (1.1 mL, 7.89 mmol) in DCM (6 mL) was stirred at 0 °C for 5 minutes. Methansulfonyl chloride (0.41 mL, 5.26 mmol) was added and the reaction mixture was stirred at 0 °C for 15 minutes. The reaction was then warmed to room temperature and stirred for 16 hours. The reaction was partitioned between DCM and water and the two phases were separated. The aqueous phase was extracted with DCM and the combined organic phases were passed through phase separating paper. The solvent was removed *in vacuo* to afford the title compound as an orange oil (290 mg, 57% yield).

¹H NMR (400 MHz, CDCl₃): δ 4.66 (s, 2 H), 3.49 (s, 3 H), 2.43 (s, 3 H).

### Intermediate 75

### 6-(4-Fluorophenyl)-8-[(3-methyl-1,2,4-oxadiazol-5-yl)methoxy]-N-[(6-methylpyridazin-3-yl)methyl]quinazolin-4-amine

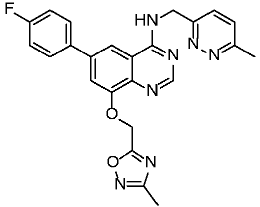

(3-Methyl-1,2,4-oxadiazol-5-yl)methyl methanesulfonate (176 mg, 0.46 mmol), 6-(4-fluorophenyl)-4-(((6-methylpyridazin-3-yl)methyl)amino)quinazolin-8-ol (150 mg, 0.23 mmol) and cesium carbonate (223 mg, 0.69 mmol) were dissolved in DMF (4 mL). The reaction mixture was heated at 55 °C for 16 hours. The reaction was cooled to room temperature and then it was partitioned between DCM and water. The two phases were separated and the aqueous phase was extracted with DCM (x2). The combined organic phases were passed through phase separating paper and concentrated *in vacuo.* Purification by preparative HPLC afforded the title compound (78.1 mg, 75%) as an off-white solid.
¹H NMR (400 MHz, DMSO): δ 9.13 (t, J = 5.9 Hz, 1 H), 8.45 (s, 1 H), 8.30 (d, J = 1.5 Hz, 1 H), 7.95-7.90 (m, 2 H), 7.75 (d, J = 1.6 Hz, 1 H), 7.59-7.49 (m, 2 H), 7.43-7.38 (m, 2 H), 5.80 (s, 2 H), 5.04 (d, J = 5.9 Hz, 2 H), 2.61-2.60 (m, 3 H), 2.39 (s, 3 H). LCMS (Method 3): [MH⁺] = 458 at 4.29 min.

The following compounds were prepared *via* adaptations of the above procedures starting from substrate reported in table.

| **Example No.** | **Structure** | **Analytical data ¹H NMR** | **Reagents** |
|---|---|---|---|
| | | **LC-MS** | |
| Example 50 | 6-(4-fluorophenyl)-8-[(3-methyloxetan-3-yl)methoxy]-*N*-[(6-methylpyridazin-3-yl)methyl]quinazolin-4-amine 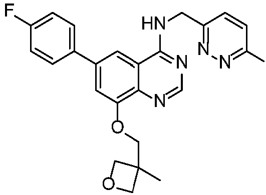 | ¹H NMR (400 MHz, DMSO): δ 9.05 (dd, J = 5.9, 5.9 Hz, 1 H), 8.46 (s, 1 H), 8.22 (d, J = 1.6 Hz, 1 H), 7.98-7.93 (m, 2 H), 7.65 (d, J = 1.5 Hz, 1 H), 7.55 (d, J = 8.7 Hz, 1 H), 7.50 (d, J = 8.7 Hz, 1 H), 7.39 (dd, J = 8.8, 8.8 Hz, 2 H), 5.04 (d, J = 5.8 Hz, 2 H), 4.60 (d, J = 5.8 Hz, 2 H), 4.39-4.35 (m, 4 H), 2.60 (s, 3 H), 1.47 (s, 3 H). LCMS (Method 3): [MH⁺] = 446 at 3.9 min. | Intermediate 11, 3-methyl-3-(*p-*toluenesulfonyl oxymethyl)oxet ane, Cs₂CO₃ |
| Example 51 | 6-(4-fluorophenyl)-*N*-[(6-methylpyridazin-3-yl)methyl]-8-[[1-(trifluoromethyl)cycloprop yl]methoxy]quinazolin-4-amine 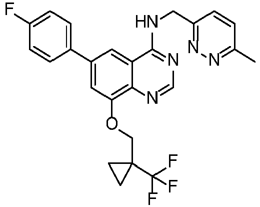 | ¹H NMR (400 MHz, DMSO): δ 9.05 (dd, J = 5.8, 5.8 Hz, 1 H), 8.46 (s, 1 H), 8.23 (d, J = 1.5 Hz, 1 H), 7.97-7.92 (m, 2 H), 7.60 (d, J = 1.5 Hz, 1 H), 7.56 (d, J = 8.7 Hz, 1 H), 7.50 (d, J = 8.7 Hz, 1 H), 7.38 (dd, J = 8.9, 8.9 Hz, 2 H), 5.03 (d, J = 5.8 Hz, 2 H), 4.44 (s, 2 H), 2.60 (s, 3 H), 1.19-1.08 (m, 4 H). LCMS (Method 3): [MH⁺] = 484 at 4.94 min. | Intermediate 11, (1-(trifluoromethyl )cyclopropyl)m ethyl methanesulfona te, Cs₂CO₃ |
| Example 52 | *tert*-butyl 3-[6-(4-fluorophenyl)-4-[(6-methylpyridazin-3-yl)methylamino]quinazolin -8-yl]oxyazetidine-1-carboxylate 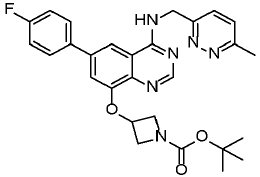 | ¹H NMR (400 MHz, DMSO): δ 9.10 (dd, J = 5.8, 5.8 Hz, 1 H), 8.45 (s, 1 H), 8.25 (d, J = 1.5 Hz, 1 H), 7.94-7.89 (m, 2 H), 7.56 (d, J = 8.7 Hz, 1 H), 7.50 (d, J = 8.8 Hz, 1 H), 7.38 (dd, J = 8.9, 8.9 Hz, 2 H), 7.30 (d, J = 1.4 Hz, 1 H), 5.38-5.31 (m, 1 H), 5.03 (d, J = 5.6 Hz, 2 H), 4.42 (dd, J = 7.2, 7.2 Hz, 2 H), 3.95 (dd, J = 2.6, 8.9 Hz, 2 H), 2.60 (s, 3 H), 1.42 (s, 9 H). LCMS (Method 3): [MH⁺] = 517 at 4.75 min. | Intermediate 11, *tert*-butyl *3-*iodoazetidine-1-carboxylate, Cs₂CO₃ |
| Example 53 | 2-[6-(4-fluorophenyl)-4-[(6-methylpyridazin-3-yl)methylamino]quinazo lin-8-yl]oxyacetonitrile 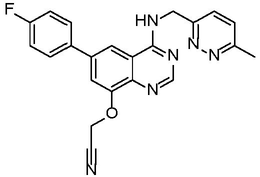 | ¹H NMR (400 MHz, DMSO): δ 9.18 (dd, J = 5.9, 5.9 Hz, 1 H), 8.46 (s, 1 H), 8.37 (d, J = 1.6 Hz, 1 H), 7.97-7.92 (m, 2 H), 7.79 (d, J = 1.6 Hz, 1 H), 7.58 (d, J = 8.5 Hz, 1 H), 7.50 (d, J = 8.7 Hz, 1 H), 7.42 (dd, J = 8.8, 8.8 Hz, 2 H), 5.50 (s, 2 H), 5.05 (d, J = 5.8 Hz, 2 H), 2.60 (s, 3 H). LCMS (Method 3): [MH⁺] = 401 at 4.07 min. | Intermediate 11, 2-bromoacetonitril e, Cs₂CO₃ |
| Example 54 | 2-[6-(4-fluorophenyl)-4-[(6-methylpyridazin-3-yl)methylamino]quinazo lin-8-yl]oxy-N-methoxy-N-methyl-acetamide 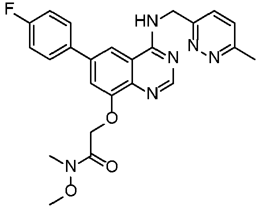 | ¹H NMR (400 MHz, DMSO): δ 9.07 (dd, J = 6.0, 6.0 Hz, 1 H), 8.44 (s, 1 H), 8.21 (d, J = 1.6 Hz, 1 H), 7.88 (ddd, J = 3.3, 5.4, 12.2 Hz, 2 H), 7.56 (d, J = 8.5 Hz, 1 H), 7.51-7.47 (m, 2 H), 7.39 (dd, J = 8.9, 8.9 Hz, 2 H), 5.24 (s, 2 H), 5.04 (d, J = 5.8 Hz, 2 H), 3.80 (s, 3 H), 3.16 (s, 3 H), 2.60 (s, 3 H). LCMS (Method 3): [MH⁺] = 463.5 at 4.08 min. | Intermediate 11, 2-bromo-N-methoxy-N-methylacetamide , Cs₂CO₃ |
| Example 55 | 2-[2-[6-(4-fluorophenyl)-4-[(6-methylpyridazin-3-yl)methylamino]quinazo lin-8-yl]oxyethoxy] ethanol 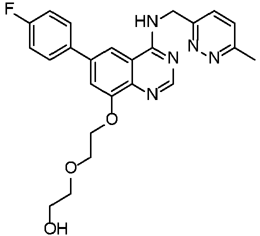 | ¹H NMR (400 MHz, DMSO): δ 9.04 (dd, J = 5.9, 5.9 Hz, 1 H), 8.43 (s, 1 H), 8.19 (d, J = 1.6 Hz, 1 H), 7.96-7.92 (m, 2 H), 7.59-7.55 (m, 2 H), 7.50 (d, J = 8.7 Hz, 1 H), 7.38 (dd, J = 8.9, 8.9 Hz, 2 H), 5.03 (d, J = 5.8 Hz, 2 H), 4.74-4.70 (m, 1 H), 4.39 (dd, J = 4.6, 4.6 Hz, 2 H), 3.89 (dd, J = 4.6, 4.6 Hz, 2 H), 3.57 (s, 4 H), 2.60 (s, 3 H). LCMS (Method 4): [MH⁺] = 450 at 2.86 min. | Intermediate 11, 2-(2-bromoethoxy)et han-1-ol , Cs₂CO₃ |
| Example 56 | 3-[6-(4-fluorophenyl)-4-[(6-methylpyridazin-3-yl)methylamino]quinazo lin-8-yl]oxy-1-methyl-pyrrolidin-2-one 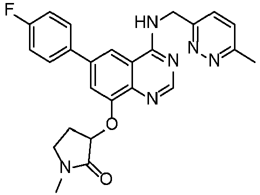 | ¹H NMR (400 MHz, DMSO): δ 9.07 (dd, J = 5.9, 5.9 Hz, 1 H), 8.42 (s, 1 H), 8.24 (d, J = 1.5 Hz, 1 H), 7.93-7.88 (m, 2 H), 7.82 (d, J = 1.5 Hz, 1 H), 7.56 (d, J = 8.7 Hz, 1 H), 7.50 (d, J = 8.7 Hz, 1 H), 7.40 (dd, J = 8.9, 8.9 Hz, 2 H), 5.44 (dd, J = 7.3, 7.3 Hz, 1 H), 5.04 (d, J = 5.8 Hz, 2 H), 3.52-3.37 (m, 2 H), 2.84 (s, 3 H), 2.61-2.60 (m, 3 H), 2.15-2.06 (m, 2 H). LCMS (Method 4): [MH⁺] = 459 at 2.93 min. | Intermediate 11, 3-bromo-1-methylpyrrolidi n-2-one , Cs₂CO₃ |
| Example 57 | 6-(4-fluorophenyl)-*N-*[(6-methylpyridazin-3-yl)methyl]-8-(oxazol-2-ylmethoxy)quinazolin-4-amine 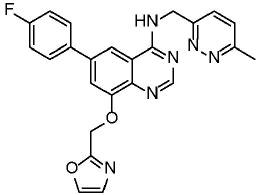 | ¹H NMR (400 MHz, DMSO): δ 9.09 (dd, J = 5.8, 5.8 Hz, 1 H), 8.42 (s, 1 H), 8.26 (d, J = 1.6 Hz, 1 H), 8.23 (s, 1 H), 7.95-7.91 (m, 2 H), 7.76 (d, J = 1.5 Hz, 1 H), 7.56 (d, J = 8.7 Hz, 1 H), 7.50 (d, J = 8.7 Hz, 1 H), 7.40 (dd, J = 8.9, 8.9 Hz, 2 H), 7.32 (s, 1 H), 5.55 (s, 2 H), 5.03 (d, J = 5.8 Hz, 2 H), 2.60 (s, 3 H). LCMS (Method 3): [MH⁺] = 443 at 4.12 min. | Intermediate 11, 2-(chloromethyl)o xazole, Cs₂CO₃ |
| Example 58 | 6-(4-fluorophenyl)-8-[(5-methyl-1,2,4-oxadiazol-3-yl)methoxy]-*N*-[(6-methylpyridazin-3-yl)methyl]quinazolin-4-amine 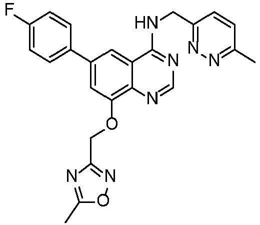 | ¹H NMR (400 MHz, DMSO): δ 9.09 (dd, J = 5.9, 5.9 Hz, 1 H), 8.43 (s, 1 H), 8.26 (d, J = 1.6 Hz, 1 H), 7.96-7.92 (m, 2 H), 7.76 (d, J = 1.5 Hz, 1 H), 7.57 (d, J = 8.5 Hz, 1 H), 7.50 (d, J = 8.8 Hz, 1 H), 7.40 (dd, J = 8.8, 8.8 Hz, 2 H), 5.55 (s, 2 H), 5.04 (d, J = 5.8 Hz, 2 H), 2.65 (s, 3 H), 2.60 (s, 3 H). LCMS (Method 4): [MH⁺] = 458 at 3.15 min. | Intermediate 11, 3-(chloromethyl)-5-methyl-1,2,4-oxadiazole, Cs₂CO₃ |
| Example 59 | 8-[(3-ethyl-1,2,4-oxadiazol-5-yl)methoxy]-6-(4-fluorophenyl)-*N*-[(6-methylpyridazin-3-yl)methyl]quinazolin-4-amine 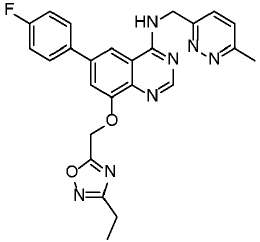 | ¹H NMR (400 MHz, DMSO): δ 9.13 (dd, J = 5.8, 5.8 Hz, 1 H), 8.45 (s, 1 H), 8.30 (d, J = 1.6 Hz, 1 H), 7.95-7.90 (m, 2 H), 7.75 (d, J = 1.5 Hz, 1 H), 7.57 (d, J = 8.5 Hz, 1 H), 7.50 (d, J = 8.7 Hz, 1 H), 7.40 (dd, J = 8.9, 8.9 Hz, 2 H), 5.79 (s, 2 H), 5.04 (d, J = 5.8 Hz, 2 H), 2.78 (q, J = 7.5 Hz, 2 H), 2.60 (s, 3 H), 1.25 (dd, J = 7.5, 7.5 Hz, 3 H). LCMS (Method 4): [MH⁺] = 472 at 3.36 min. | Intermediate 11, 5-(chloromethyl)-3-ethyl-1,2,4-oxadiazole, Cs₂CO₃ |
| Example 60 | 8-[(5-cyclopropyl-1,3,4-thiadiazol-2-yl)methoxy]-6-(4-fluorophenyl)-*N*-[(6-methylpyridazin-3-yl)methyl]quinazolin-4-amine 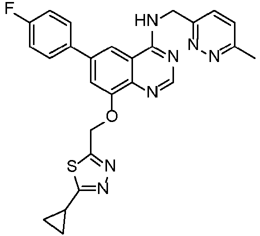 | ¹H NMR (400 MHz, DMSO): δ 9.11 (dd, J = 5.9, 5.9 Hz, 1 H), 8.45 (s, 1 H), 8.29 (d, J = 1.5 Hz, 1 H), 7.97-7.92 (m, 2 H), 7.80 (d, J = 1.5 Hz, 1 H), 7.57 (d, J = 8.7 Hz, 1 H), 7.50 (d, J = 8.7 Hz, 1 H), 7.40 (dd, J = 8.9, 8.9 Hz, 2 H), 5.82 (s, 2 H), 5.04 (d, J = 5.8 Hz, 2H), 2.60 (s, 3 H), 2.59-2.54 (m, 1 H), 1.26-1.20 (m, 2 H), 1.08-1.03 (m, 2 H). LCMS (Method 4): [MH⁺] = 500 at 3.23 min. | Intermediate 11, 2-(chloromethyl)-5-cyclopropyl-1,3,4-thiadiazole, Cs₂CO₃ |
| Example 61 | 2-[6-(4-fluorophenyl)-4-[(6-methylpyridazin-3-yl)methylamino]quinazo lin-8-yl]oxy-*N*-(2,2,2-trifluoroethyl)acetamide 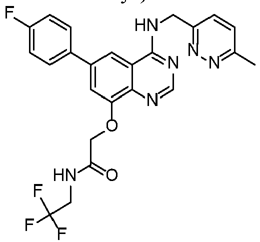 | ¹H NMR (400 MHz, DMSO): δ 9.28-9.15 (m, 2 H), 8.47 (s, 1 H), 8.32 (d, J = 1.6 Hz, 1 H), 7.93-7.88 (m, 2 H), 7.72 (d, J = 1.6 Hz, 1 H), 7.57 (d, J = 8.7 Hz, 1 H), 7.51 (d, J = 8.7 Hz, 1 H), 7.39 (dd, J = 8.9, 8.9 Hz, 2 H), 5.05 (d, J = 5.8 Hz, 2 H), 4.95 (s, 2 H), 4.09-3.98 (m, 2 H), 2.60 (s, 3 H). LCMS (Method 4): [MH⁺] = 501 at 3.22 min. | Intermediate 11, 2-chloro-N-(2,2,2-trifluoroethyl)ac etamide, Cs₂CO₃ |
| Example 62 | 8-((5-cyclopropyl-1,3,4-oxadiazol-2-yl)methoxy)-6-(4-fluorophenyl)-N-((6-methylpyridazin-3-yl)methyl)quinazolin-4-amine 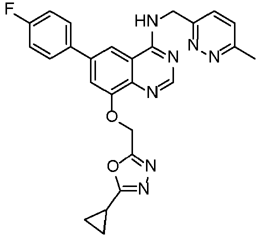 | ¹H NMR (400 MHz, DMSO): δ 9.12 (dd, J = 5.8, 5.8 Hz, 1 H), 8.43 (s, 1 H), 8.30 (d, J = 1.5 Hz, 1 H), 7.96-7.92 (m, 2 H), 7.79 (d, J = 1.6 Hz, 1 H), 7.57 (d, J = 8.7 Hz, 1 H), 7.50 (d, J = 8.7 Hz, 1 H), 7.41 (dd, J = 8.9, 8.9 Hz, 2 H), 5.64 (s, 2 H), 5.04 (d, J = 5.8 Hz, 2 H), 2.60 (s, 3 H), 2.35-2.26 (m, 1 H), 1.20-1.14 (m, 2 H), 1.04-0.99 (m, 2 H). LCMS (Method 4): [MH+] = 484 at 3.06 min. | Intermediate 11, 2-(chloromethyl)-5-cyclopropyl-1,3,4-oxadiazole, Cs₂CO₃ |
| Example 63 | 6-(4-fluorophenyl)-N-((6-methylpyridazin-3-yl)methyl)-8-(oxetan-3-yloxy)quinazolin-4-amine 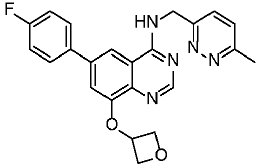 | ¹H NMR (400 MHz, DMSO): δ 8.29 (s, 1 H), 8.17 (s, 1 H), 8.03 (d, J = 1.6 Hz, 1 H), 7.80-7.75 (m, 2 H), 7.68 (d, J = 8.5 Hz, 1 H), 7.55-7.51 (m, 2 H), 7.32 (dd, J = 8.9, 8.9 Hz, 2 H), 5.07 (s, 2 H), 4.43-4.37 (m, 2 H), 4.02-3.95 (m, 1 H), 3.83-3.72 (m, 2 H), 2.61 (s, 3 H). LCMS (Method 4): [MH+] = 418 at 2.69 min. | Intermediate 11, 3-iodooxetane, Cs₂CO₃ |
| Example 64 | Methyl 4-[[6-(4-fluorophenyl)-4-[(6-methylpyridazin-3-yl)methylamino]quinazo lin-8-yl]oxymethyl]piperidine-1-carboxylate 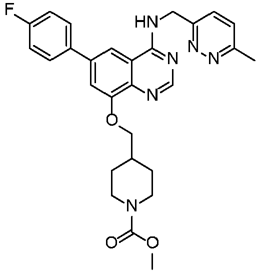 | ¹H NMR (400 MHz, DMSO): δ 9.03 (dd, J = 5.8, 5.8 Hz, 1 H), 8.44 (s, 1 H), 8.17 (d, J = 1.4 Hz, 1 H), 7.95-7.90 (m, 2 H), 7.55 (d, J = 8.7 Hz, 2 H), 7.50 (d, J = 8.7 Hz, 1 H), 7.38 (dd, J = 8.9, 8.9 Hz, 2 H), 5.03 (d, J = 5.8 Hz, 2 H), 4.10 (dd, J = 6.5, 16.4 Hz, 2 H), 4.06-3.93 (m, 4 H), 3.59 (s, 3 H), 3.18 (s, 3 H), 2.13-2.06 (m, 1 H), 1.93-1.84 (m, 1 H), 1.70-1.66 (m, 1 H), 1.34-1.07 (m, 2 H). LCMS (Method 4): [MH⁺] = 517.5 at 3.36 min. | Intermediate 11, Methyl 4-(((methylsulfon yl)oxy)methyl)p iperidine-1-carboxylate, Cs₂CO₃ |
| Example 65 | Methyl 4-[6-(4-fluorophenyl)-4-[(6-methylpyridazin-3-yl)methylamino]quinazo lin-8-yl]oxypiperidine-1-carboxylate 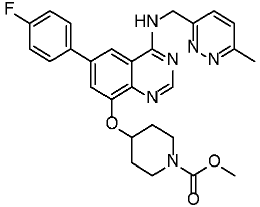 | ¹H NMR (400 MHz, DMSO): δ 9.08 (s, 1 H), 8.45-8.44 (m, 1 H), 8.23 (d, J = 1.6 Hz, 1 H), 7.94-7.90 (m, 2 H), 7.69 (d, J = 1.5 Hz, 1 H), 7.58-7.49 (m, 2 H), 7.41-7.36 (m, 2 H), 5.04-4.99 (m, 3 H), 3.80-3.72 (m, 2 H), 3.62 (s, 3 H), 3.33-3.29 (m, 2H), 2.60 (s, 3 H), 2.01-1.92 (m, 2 H), 1.75-1.65 (m, 2 H). LCMS (Method 3): [MH⁺] = 503 at 3.98 min. | Intermediate 11, Methyl 4-((methylsulfony l)oxy)piperidine -1-carboxylate, Cs₂CO₃ |
| Example 66 | Methyl 3-[[6-(4-fluorophenyl)-4-[(6-methylpyridazin-3-yl)methylamino]quinazo lin-8-yl]oxymethyl]piperidine-1-carboxylate 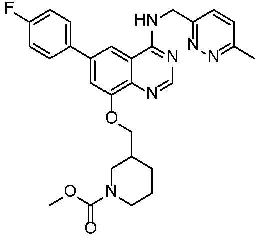 | ¹H NMR (400 MHz, DMSO): δ 9.04 (t, J = 5.9 Hz, 1 H), 8.46 (s, 1 H), 8.19 (d, J = 1.5 Hz, 1 H), 7.96-7.91 (m, 2 H), 7.57-7.49 (m, 3 H), 7.41-7.36 (m, 2 H), 5.03 (d, J = 5.8 Hz, 2 H), 4.18-4.11 (m, 3 H), 3.89 (d, J = 12.9 Hz, 1 H), 3.61-3.56 (m, 3 H), 2.92-2.87 (m, 2 H), 2.60 (s, 3 H), 2.10-2.01 (m, 1 H), 1.97-1.91 (m, 1 H), 1.74-1.69 (m, 1 H), 1.49-1.32 (m, 2 H). LCMS (Method 3): [MH⁺] = 517 at 4.34 min. | Intermediate 11, Methyl 3-(((methylsulfon yl)oxy)methyl)p iperidine-1-carboxylate, Cs₂CO₃ |
| Example 67 | Methyl 3-[[6-(4-fluorophenyl)-4-[(6-methylpyridazin-3-yl)methylamino]quinazo lin-8-yl]oxymethyl]pyrrolidin e-1-carboxylate 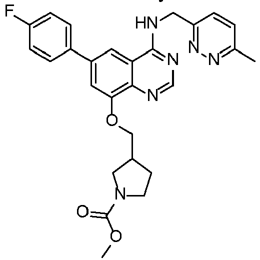 | ¹H NMR (400 MHz, DMSO): δ 9.04 (t, J = 5.9 Hz, 1 H), 8.45 (s, 1 H), 8.19 (d, J = 1.5 Hz, 1 H), 7.96-7.91 (m, 2 H), 7.60-7.49 (m, 3 H), 7.41-7.35 (m, 2 H), 5.03 (d, J = 5.8 Hz, 2 H), 4.30-4.16 (m, 2 H), 3.59 (s, 3 H), 3.57-3.53 (m, 1 H), 3.51-3.45 (m, 1 H), 3.32-3.26 (m, 1 H), 2.84-2.67 (m, 2 H), 2.61-2.60 (m, 3 H), 2.15-2.04 (m, 1 H), 1.91-1.81 (m, 1 H). LCMS (Method 3): [MH⁺] = 503 at 3.95 min. | Intermediate 11, Methyl 3-(((methylsulfon yl)oxy)methyl)p yrrolidine-1-carboxylate, Cs₂CO₃ |
| Example 68 | Methyl 3-[6-(4-fluorophenyl)-4-[(6-methylpyridazin-3-yl)methylamino]quinazo lin-8-yl]oxypyrrolidine-1-carboxylate 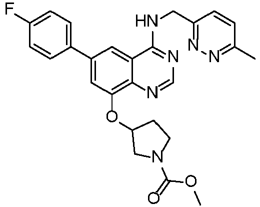 | ¹H NMR (400 MHz, DMSO): δ 9.08 (t, J = 5.9 Hz, 1 H), 8.43-8.43 (m, 1 H), 8.25 (d, J = 1.6 Hz, 1 H), 7.95-7.91 (m, 2 H), 7.63-7.48 (m, 3 H), 7.41-7.36 (m, 2 H), 5.05-5.01 (m, 2 H), 3.64-3.59 (m, 6 H), 3.52-3.44 (m, 5 H), 2.21-2.19 (m, 2 H). LCMS (Method 3): [MH+] = 489 at 3.78 min. | Intermediate 11, Methyl 3-((methylsulfony l)oxy)pyrrolidin e-1-carboxylate, Cs₂CO₃ |
| Example 69 | Methyl 3-[6-(4-fluorophenyl)-4-[(6-methylpyridazin-3-yl)methylamino]quinazo lin-8 yl]-oxypiperidine-1-carboxylate 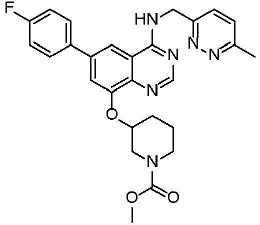 | ¹H NMR (400 MHz, DMSO): δ 9.08 (t, J = 5.5 Hz, 1 H), 8.43 (s, 1 H), 8.25 (s, 1 H), 7.93 (dd, J = 5.5, 8.6 Hz, 2 H), 7.68 (s, 1 H), 7.58-7.48 (m, 2 H), 7.42-7.36 (m, 2 H), 5.05-5.01 (m, 2 H), 4.80 (s, 1 H), 3.85-3.79 (m, 1 H), 3.63-3.44 (m, 5 H), 2.60 (s, 3 H), 2.06-2.02 (m, 1 H), 1.90-1.81 (m, 2 H), 1.59-1.48 (m, 1 H). LCMS (Method 4): [MH⁺] = 503 at 3.23 min. | Intermediate 11, Methyl 3-((methylsulfony l)oxy)piperidine -1-carboxylate, Cs₂CO₃ |

### PHARMACOLOGICAL ACTIVITY OF THE COMPOUNDS OF THE INVENTION.

### In vitro Electrophysiology Assay for P2X₃

Cells expressing P2X₃ receptors were grown according to standard practice and maintained at 37 °C in a 5% humidified CO₂ atmosphere. The cells were seeded into T175 flask 2 days prior to the day of the assay and dissociated from the flasks using TrypLE when grown to confluence of 80-90%. The dissociated cells were resuspended in serum free media at a cell density of 3×10⁶ cells/ml and loaded onto the Sophion Qube automated patch-clamp system. The extracellular assay buffer contained 145 mM NaCl, 4 mM KCl, 2 mM CaCl₂, 1 mM MgCl₂, 10 mM HEPES, and 10 mM glucose at pH 7.4. The intracellular assay solution contained 140 mM CsF, 10 mM NaCl, 10 mM EGTA, 10 mM HEPES at pH 7.2. Agonist stock solutions were prepared in H₂O and diluted in bath solution prior to use. All antagonists were prepared as 10 mM stock solutions in DMSO and diluted in bath solution prior to use. All experiments were performed under the wholecell patch clamp configuration at room temperature with 384 individual cells being voltage clamped at -60 mV simultaneously on the Sophion Qube instrument. Two baseline responses were established with the application of α,β-MeATP (800 nM), with the subsequent agonist applications being washed out using extracellular assay buffer containing 0.5 U/ml apyrase. Following the second agonist application, antagonist was incubated in the absence of α,β-MeATP for 10 minutes. After antagonist preincubation, 800 nM α,β-MeATP and antagonist were co-administered to determine the inhibitory effect of the antagonist. One concentration of an antagonist was assessed against a single cell, with different concentrations of the antagonist applied to other cells on the 384 recording substrate. The control P2X₃ current amplitude was taken from the peak current amplitude from the second agonist response prior to preincubation with antagonist. The peak P2X₃ current amplitude in the presence of antagonist was used to calculate the inhibitory effect at each concentration of the antagonist according to the following equation: Percentage inhibition of P2X3 = (P2X3 control peak amplitude-P2X3 antagonist peak amplitude)/ P2X3 control peak amplitude)* 100

Concentration-response curves were constructed from ten different concentrations with each concentration of antagonist tested on at least two individual cells. The concentration of the antagonist to inhibit P2X₃ current by 50% (IC₅₀) was determined by fitting the data with the following equation: $Y = a + \left[\left(b-a\right)/\left(1+{10}^{∧}\left(\left(log c-x\right)d\right)\right.\right]$

Where 'a' is minimum response, 'b' is maximum response, 'c' is IC₅₀ and 'd' is Hill slope.

The results for individual compounds are provided below in Table 2 and are expressed as range of activity.

**Table 2**

| **Example No.** | **h P2X₃** |
|---|---|
| 1 | +++ |
| 2 | +++ |
| 4 | ++ |
| 5 | ++ |
| 6 | +++ |
| 7 | + |
| 8 | +++ |
| 9 | +++ |
| 10 | ++ |
| 11 | +++ |
| 12 | +++ |
| 13 | +++ |
| 14 | +++ |
| 15 | +++ |
| 16 | +++ |
| 17 | +++ |
| 18 | ++ |
| 19 | ++ |
| 20 | +++ |
| 21 | +++ |
| 22 | +++ |
| 23 | ++ |
| 24 | ++ |
| 25 | ++ |
| 26 | ++ |
| 27 | +++ |
| 28 | + |
| 29 | + |
| 30 | ++ |
| 31 | ++ |
| 32 | ++ |
| 33 | ++ |
| 34 | +++ |
| 35 | ++ |
| 36 | ++ |
| 37 | +++ |
| 38 | +++ |
| 39 | +++ |
| 40 | +++ |
| 41 | ++ |
| 42 | ++ |
| 43 | ++ |
| 44 | ++ |
| 45 | +++ |
| 46 | ++ |
| 47 | ++ |
| 48 | ++ |
| 49 | +++ |
| 50 | +++ |
| 51 | ++ |
| 52 | ++ |
| 53 | +++ |
| 54 | +++ |
| 55 | +++ |
| 56 | ++ |
| 57 | +++ |
| 58 | +++ |
| 59 | +++ |
| 60 | +++ |
| 61 | ++ |
| 62 | +++ |
| 63 | ++ |
| 64 | ++ |
| 65 | ++ |
| 66 | ++ |
| 67 | ++ |
| 68 | ++ |

| | |
|---|---|
| wherein the compounds are classified in term of potency with respect to their inhibitory activity on P2X₃ according to the following classification criterion: +++: pIC₅₀ h P2X₃ > 6.5 ++: 6.5 > pIC₅₀ h P2X₃ > 5.5 +: 5.5 > pIC₅₀ h P2X₃ > 4.5 | |

### In vitro Electrophysiology Assay for P2X_{2/3}

Representative compound of the present invention have been also tested for P2X_{2/3} receptor.

The same assay protocol was used for the P2X_{2/3} assay as the P2X₃ assay with two modifications: 1) 10 µM ATP was used as the agonist; and 2) the mean current amplitude was measured seven seconds after the application of agonist.

The results of Table 3 indicate that representative compounds of the present invention are selective P2X₃ antagonist.

**Table 3**

| **Example No.** | **h P2X₃** | **h P2X_{2/3}** |
|---|---|---|
| 1 | +++ | + |
| 2 | +++ | ++ |
| 6 | +++ | + |
| 9 | +++ | ++ |
| 15 | +++ | ++ |
| 16 | +++ | ++ |
| 17 | +++ | ++ |
| 21 | +++ | + |
| 22 | +++ | + |
| 27 | +++ | ++ |
| 34 | +++ | ++ |
| 42 | ++ | + |
| 43 | ++ | + |
| 47 | ++ | + |
| 62 | +++ | ++ |
| 65 | ++ | + |
| 68 | ++ | + |

| | | |
|---|---|---|
| wherein the compounds are classified in term of potency with respect to their inhibitory activity on P2X₃ or P2X_{2/3} isoforms according to the following classification criterion: +++: pIC₅₀ h P2X₃ or h P2X_{2/3} > 6.5 ++: 6.5 > pIC₅₀ h P2X₃ or h P2X_{2/3} > 5.5 +: 5.5 > pIC₅₀ h P2X₃ or h P2X_{2/3} > 4.5 | | |

### Comparative Example A

### N-(6-(4-fluorophenyl)chroman-4-yl)-2-(trifluoromethyl)pyrimidine-5-carboxamide

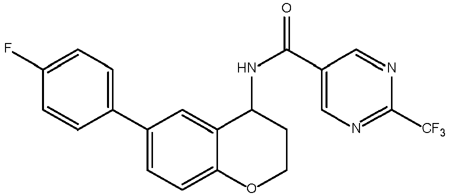

The activity of comparative Example A as has been tested in the in vitro assay for the determination of activity on P2X₃ receptor as described above.

Differently from the compounds of formula (I) of the present invention, the comparative Example A do not show a proper inhibitory activity on P2X₃, in fact the activity on receptor P2X₃ expressed as pIC₅₀ is < 4.5.

The above results demonstrate that the amino quinazoline scaffold in combination with a proper substituents, in particular in R₂ position, unexpectedly lead to a series of compounds that is active against the receptor P2X₃.

## Claims

1. A compound of formula (I) 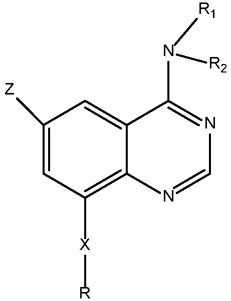 wherein
**X** is selected from S, SO₂, SO, or O;
**Z** is selected from the group consisting of heteroaryl and aryl, wherein any of such heteroaryl and aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R** is selected from the group consisting of (C₁-C₆)alkyl-, (C₁-C₆)alkyl-CN, (C₁-C₆)haloalkyl, -NR_{A}R_{B}, (C₃-C₈)heterocycloalkyl-(C₁-C₆)alkyl-, (C₃-C₈)heterocycloalkyl-, (C₃-C₈)heteroaryl-(C₁-C₆)alkyl-, (C₃-C₈)cycloalkyl-(C₁-C₆)alkyl-, (C₃-C₈)cycloalkyl-, R_{A}O-(C₁-C₆)alkyl-O-(C₁-C₆)alkyl-, R_{A}NH-C(O)-(C₁-C₄)alkyl-, R_{A}R_{B}N-C(O)-(C₁-C₄)alkyl-, R_{A}O-C(O)(C₁-C₆)alkyl-,
wherein any of such heterocycloalkyl is unsubstituted or substituted by one or more groups selected from OH, -C(O)Rₐ, -C(O)OR_{A}, (C₁-C₆)haloalkyl, -NH-C(O)R₁, and oxo;
any of such cycloalkyl is substituted by one or more groups selected from - C(O)OR_{A} and (C₁-C₆)haloalkyl-,
any of such heteroaryl may be optionally substituted by one or more groups selected from (C₁-C₆)alkyl-, -OH and (C₃-C₆)cycloalkyl-;
**R_{A}** and **R_{B}** are at each occurrence independently H or selected from the group consisting of (C₁-C₆)alkyl-, (C₁-C₆)haloalkyl-, -OR₁, -SO₂R_{C} and (C₃-C₆)cycloalkyl wherein such cycloalkyl may be optionally substituted by one or more -C(O)OR₁, or alternatively
**R_{A}** and **R_{B}** may form together with the nitrogen atom to which they are attached a 5 or 6 membered saturated heterocyclic monocyclic ring system optionally containing a further heteroatom which is nitrogen or oxygen, which may be optionally substituted by one or more groups selected from halo and -OR₁; and **R_{C}** is aryl;
with the proviso that R is (C₁-C₆)alkyl- or unsubstituted (C₃-C₈)heterocycloalkyl-only when X is S or SO.

2. The compound of formula (I) according to claim 1, selected from the group consisting of:
8-(2,2-difluoroethoxy)-6-(4-fluorophenyl)-N-((6-methylpyridazin-3-yl)methyl)quinazolin-4-amine,
(R)-8-(2,2-difluoroethoxy)-6-(4-fluorophenyl)-N-(1-(2-(trifluoromethyl)pyrimidin-5-yl)ethyl)quinazolin-4-amine,
8-(2,2-difluoroethoxy)-6-(5-fluoropyridin-2-yl)-N-((6-methylpyridazin-3-yl)methyl)quinazolin-4-amine,
(R)-8-(2,2-difluoroethoxy)-6-(5-fluoropyridin-2-yl)-N-(1-(6-methylpyridazin-3-yl)ethyl)quinazolin-4-amine,
Single enantiomer 1 of 8-(2,2-difluoroethoxy)-6-(5-fluoro-2-pyridyl)-N-[1-(5-methyl-1,3,4-thiadiazol-2-yl)ethyl]quinazolin-4-amine,
Single enantiomer 2 of 8-(2,2-difluoroethoxy)-6-(5-fluoro-2-pyridyl)-N-[1-(5-methyl-1,3,4-thiadiazol-2-yl)ethyl]quinazolin-4-amine,
8-(2,2-Difluoroethoxy)-N-((6-methylpyridazin-3-yl) methyl)-6-(5-methylpyridin-2-yl) quinazolin-4-amine,
8-(2,2-difluoroethoxy)-N-[(6-methylpyridazin-3-yl)methyl]-6-(5-methylpyrimidin-2-yl)quinazolin-4-amine,
(R)-6-(4-fluorophenyl)-N-(1-(6-methylpyridazin-3-yl)ethyl)-8-(morpholinosulfonyl)quinazolin-4-amine,
((R)-6-(4-fluorophenyl)-8-(morpholinosulfonyl)-N-(1-(2-(trifluoromethyl)pyrimidin-5-yl)ethyl)quinazolin-4-amine,
(Rac)-6-(4-fluorophenyl)-N-(1-(5-methyl-1,3,4-thiadiazol-2-yl)ethyl)-8-(morpholinosulfonyl)quinazolin-4-amine,
(R)-6-(4-fluorophenyl)-N,N-dimethyl-4-((1-(6-methylpyridazin-3-yl)ethyl)amino)quinazoline-8-sulfonamide,
6-(4-fluorophenyl)-N,N-dimethyl-4-((1-(5-methyl-1,3,4-thiadiazol-2-yl)ethyl)amino)quinazoline-8-sulfonamide,
6-(4-fluorophenyl)-N,N-dimethyl-4-((1-(5-methyl-1,2,4-oxadiazol-3-yl)ethyl)amino)quinazoline-8-sulfonamide,
(R)-8-((3,3-difluoropyrrolidin-1-yl)sulfonyl)-6-(4-fluorophenyl)-N-(1-(6-methylpyridazin-3-yl)ethyl)quinazolin-4-amine,
(R)-8-((3,3-difluoropyrrolidin-1-yl)sulfonyl)-6-(4-fluorophenyl)-N-(1-(5-methyl-1,2,4-oxadiazol-3-yl)ethyl)quinazolin-4-amine,
8-((3,3-difluoropyrrolidin-1-yl)sulfonyl)-6-(4-fluorophenyl)-N-(1-(5-methyl-1,3,4-thiadiazol-2-yl)ethyl)quinazolin-4-amine,
(R)-1-((6-(4-fluorophenyl)-4-((1-(6-methylpyridazin-3-yl)ethyl)amino)quinazolin-8-yl)sulfonyl)piperidin-4-ol,
(R)-1-((6-(4-fluorophenyl)-4-((1-(5-methyl-1,2,4-oxadiazol-3-yl)ethyl)amino)quinazolin-8-yl)sulfonyl)piperidin-4-ol,
(R)-6-(4-fluorophenyl)-N-methyl-4-((1-(6-methylpyridazin-3-yl)ethyl)amino)quinazoline-8-sulfonamide,
(R)-6-(4-fluorophenyl)-N-methyl-4-((1-(5-methyl-1,2,4-oxadiazol-3-yl)ethyl)amino)quinazoline-8-sulfonamide,
(6-(4-fluorophenyl)-N-methyl-4-((1-(5-methyl-1,3,4-thiadiazol-2-yl)ethyl)amino)quinazoline-8-sulfonamide,
(R)-6-(4-fluorophenyl)-N-(1-(6-methylpyridazin-3-yl)ethyl)-8-(piperazin-1-ylsulfonyl)quinazolin-4-amine,
(R)-6-(4-fluorophenyl)-N-(1-(5-methyl-1,2,4-oxadiazol-3-yl)ethyl)-8-(piperazin-1-ylsulfonyl)quinazolin-4-amine,
(R)-6-(4-fluorophenyl)-N-(1-(6-methylpyridazin-3-yl)ethyl)-8-(methylthio) quinazolin-4-amine,
6-(4-fluorophenyl)-N-((R)-1-(6-methylpyridazin-3-yl)ethyl)-8-(methylsulfinyl) quinazolin-4-amine,
(R)-6-(4-fluorophenyl)-N-(1-(6-methylpyridazin-3-yl)ethyl)-8-(oxetan-3-ylthio)quinazolin-4-amine,
6-(4-fluorophenyl)-N-((R)-1-(6-methylpyridazin-3-yl)ethyl)-8-(oxetan-3-ylsulfinyl)quinazolin-4-amine,
(1-((S)-3-((6-(4-fluorophenyl)-4-(((R)-1-(2-(trifluoromethyl)pyrimidin-5-yl)ethyl)amino)quinazolin-8-yl)oxy)pyrrolidin-1-yl)ethan-1-one,
(1-((R)-3-((6-(4-fluorophenyl)-4-(((R)-1-(2-(trifluoromethyl)pyrimidin-5-yl)ethyl)amino)quinazolin-8-yl)oxy)pyrrolidin-1-yl)ethan-1-one,
N-((1 S,4s)-4-((6-(4-fluorophenyl)-4-(((R)-1-(2-(trifluoromethyl)pyrimidin-5-yl)ethyl)amino)quinazolin-8-yl)oxy)cyclohexyl)acetamide,
6-(4-fluorophenyl)-8-((1-(2,2,2-trifluoroethyl)pyrrolidin-3-yl)oxy)-N-((R)-1-(2-(trifluoromethyl)pyrimidin-5-yl)ethyl)quinazolin-4-amine,
3-((6-(4-fluorophenyl)-4-(((R)-1-(2-(trifluoromethyl)pyrimidin-5-yl)ethyl)amino)quinazolin-8-yl)oxy)pyrrolidin-2-one,
(R)-1-(4-((6-(4-fluorophenyl)-4-((1-(2-(trifluoromethyl)pyrimidin-5-yl)ethyl)amino)quinazolin-8-yl)oxy)piperidin-1-yl)ethan-1-one,
(R)-6-(4-fluorophenyl)-8-((1-(2,2,2-trifluoroethyl)piperidin-4-yl)oxy)-N-(1-(2-(trifluoromethyl)pyrimidin-5-yl)ethyl)quinazolin-4-amine,
(R)-3-(((6-(4-fluorophenyl)-4-((1-(2-(trifluoromethyl)pyrimidin-5-yl)ethyl)amino)quinazolin-8-yl)oxy)methyl)oxetan-3-ol,
(R)-5-(((6-(4-fluorophenyl)-4-((1-(2-(trifluoromethyl)pyrimidin-5-yl)ethyl)amino)quinazolin-8-yl)oxy)methyl)isoxazol-3-ol,
(R)-2-((6-(4-fluorophenyl)-4-((1-(2-(trifluoromethyl)pyrimidin-5-yl)ethyl)amino)quinazolin-8-yl)oxy)acetic acid,
(R)-2-((6-(4-fluorophenyl)-4-((1-(2-(trifluoromethyl)pyrimidin-5-yl)ethyl)amino)quinazolin-8-yl)oxy)-N-(phenylsulfonyl)acetamide,
(R)-1-(((6-(4-fluorophenyl)-4-((1-(2-(trifluoromethyl)pyrimidin-5-yl)ethyl)amino)quinazolin-8-yl)oxy)methyl)cyclopropane-1-carboxylic acid,
Methyl 1-(2-((6-(4-fluorophenyl)-4-(((6-methylpyridazin-3-yl)methyl)amino)quinazolin-8-yl)oxy)acetamido)cyclopropane-1-carboxylate,
Methyl (R)-1-(2-((6-(4-fluorophenyl)-4-((1-(2-(trifluoromethyl)pyrimidin-5-yl)ethyl)amino) quinazolin-8-yl)oxy) acetamido) cyclopropane-1-carboxylate,
(R)-1-(2-((6-(4-fluorophenyl)-4-((1-(2-(trifluoromethyl)pyrimidin-5-yl)ethyl)amino)quinazolin-8-yl)oxy)acetamido)cyclopropane-1-carboxylic acid,
(1R,2S)-2-(2-((6-(4-fluorophenyl)-4-(((6-methylpyridazin-3 yl)methyl)amino)quinazolin-8-yl)oxy)acetamido)cyclopentane-1-carboxylic acid,
Ethyl (1S,2R)-2-(2-((6-(4-fluorophenyl)-4-(((6-methylpyridazin-3-yl)methyl)amino)quinazolin-8-yl)oxy)acetamido)cyclopentane-1-carboxylate,
(R)-4-(((6-(4-fluorophenyl)-4-((1-(2-(trifluoromethyl)pyrimidin-5-yl)ethyl)amino)quinazolin-8-yl)oxy)methyl)piperidine-4-carboxylic acid hydrochloride,
(R)-1-(((6-(4-fluorophenyl)-4-((1-(2-(trifluoromethyl)pyrimidin-5-yl)ethyl)amino)quinazolin-8-yl)oxy)methyl)cyclohexane-1-carboxylic acid,
Ammonium (2-((4-(((6-methylpyridazin-3-yl)methyl)amino)-6-(5-methylpyrimidin-2-yl)quinazolin-8-yl)oxy)acetyl)(phenylsulfonyl)amide,
6-(4-fluorophenyl)-8-[(3-methyloxetan-3-yl)methoxy]-N-[(6-methylpyridazin-3-yl)methyl]quinazolin-4-amine,
6-(4-fluorophenyl)-N-[(6-methylpyridazin-3-yl)methyl]-8-[[1-(trifluoromethyl)cyclopropyl]methoxy]quinazolin-4-amine,
tert-butyl 3-[6-(4-fluorophenyl)-4-[(6-methylpyridazin-3-yl)methylamino]quinazolin-8-yl]oxyazetidine-1-carboxylate,
2-[6-(4-fluorophenyl)-4-[(6-methylpyridazin-3-yl)methylamino]quinazolin-8-yl]oxyacetonitrile,
2-[6-(4-fluorophenyl)-4-[(6-methylpyridazin-3-yl)methylamino]quinazolin-8-yl]oxy-N-methoxy-N-methyl-acetamide,
2-[2-[6-(4-fluorophenyl)-4-[(6-methylpyridazin-3-yl)methylamino]quinazolin-8-yl]oxyethoxy]ethanol,
3-[6-(4-fluorophenyl)-4-[(6-methylpyridazin-3-yl)methylamino]quinazolin-8-yl]oxy-1-methyl-pyrrolidin-2-one,
6-(4-fluorophenyl)-N-[(6-methylpyridazin-3-yl)methyl]-8-(oxazol-2-ylmethoxy)quinazolin-4-amine,
6-(4-fluorophenyl)-8-[(5-methyl-1,2,4-oxadiazol-3-yl)methoxy]-N-[(6-methylpyridazin-3-yl)methyl]quinazolin-4-amine,
8-[(3-ethyl-1,2,4-oxadiazol-5-yl)methoxy]-6-(4-fluorophenyl)-N-[(6-methylpyridazin-3-yl)methyl]quinazolin-4-amine,
8-[(5-cyclopropyl-1,3,4-thiadiazol-2-yl)methoxy]-6-(4-fluorophenyl)-N-[(6-methylpyridazin-3-yl)methyl]quinazolin-4-amine,
2-[6-(4-fluorophenyl)-4-[(6-methylpyridazin-3-yl)methylamino]quinazolin-8-yl]oxy-N-(2,2,2-trifluoroethyl)acetamide,
8-((5-cyclopropyl-1,3,4-oxadiazol-2-yl)methoxy)-6-(4-fluorophenyl)-N-((6-methylpyridazin-3-yl)methyl)quinazolin-4-amine,
6-(4-fluorophenyl)-N-((6-methylpyridazin-3-yl)methyl)-8-(oxetan-3-yloxy)quinazolin-4-amine,
Methyl 4-[[6-(4-fluorophenyl)-4-[(6-methylpyridazin-3-yl)methylamino]quinazolin-8-yl]oxymethyl]piperidine-1-carboxylate,
Methyl 4-[6-(4-fluorophenyl)-4-[(6-methylpyridazin-3-yl)methylamino]quinazolin-8-yl]oxypiperidine-1-carboxylate,
Methyl 3-[[6-(4-fluorophenyl)-4-[(6-methylpyridazin-3-yl)methylamino]quinazolin-8-yl]oxymethyl]piperidine-1-carboxylate,
Methyl 3-[[6-(4-fluorophenyl)-4-[(6-methylpyridazin-3-yl)methylamino]quinazolin-8-yl]oxymethyl]pyrrolidine-1-carboxylate,
Methyl 3-[6-(4-fluorophenyl)-4-[(6-methylpyridazin-3-yl)methylamino]quinazolin-8-yl]oxypyrrolidine-1-carboxylate,

3. The compound of formula (I) according to claim 1, wherein
**X** is selected from S or SO;
**Z** is aryl, wherein such aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R** is selected from the group consisting of (C₁-C₆)alkyl- and (C₃-C₈)heterocycloalkyl-.

4. The compound of formula (I), according to claim 1, wherein X is O, represented by the formula (Ia) 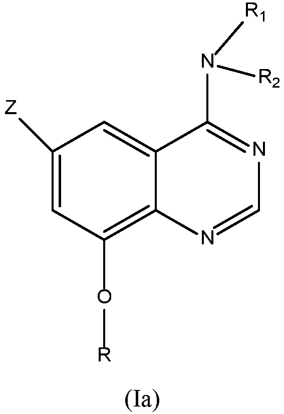 wherein
**Z** is selected from the group consisting of heteroaryl and aryl, wherein any of such heteroaryl and aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R** is selected from the group consisting of (C₁-C₆)alkyl-CN, (C₁-C₆)haloalkyl, -NR_{A}R_{B}, (C₃-C₈)heterocycloalkyl-(C₁-C₆)alkyl-, (C₃-C₈)heterocycloalkyl-, (C₃-C₈)heteroaryl-(C₁-C₆)alkyl-, (C₃-C₈)cycloalkyl-(Cl-C₆)alkyl-, (C₃-C₈)cycloalkyl-, R_{A}O-(C₁-C₆)alkyl-O-(C₁-C₆)alkyl-, R_{A}NH-C(O)-(C₁-C₄)alkyl-, RₐR_{B}N-C(O)-(C₁-C₄)alkyl- and R_{A}O-C(O)(C₁-C₆)alkyl-,
wherein any of such heterocycloalkyl is substituted by one or more groups selected from -OH, -C(O)R_{A}, -C(O)OR_{A}, (C₁-C₆)haloalkyl, -NH-C(O)R₁, and oxo;
any of such cycloalkyl is substituted by one or more groups selected from -C(O)OR_{A} and (C₁-C₆)haloalkyl-,
any of such heteroaryl may be optionally substituted by one or more groups selected from (C₁-C₆)alkyl-, -OH and (C₃-C₆)cycloalkyl-;
**R_{A}** and **R_{B}** are at each occurrence independently H or selected from the group consisting of (C₁-C₆)alkyl-, (C₁-C₆)haloalkyl-, -OR₁, -SO₂R_{C} and (C₃-C₆)cycloalkyl wherein such cycloalkyl may be optionally substituted by one or more -C(O)OR₁; and
**R_{C}** is aryl.

5. The compound of formula (I) according to claim 1, wherein X is SO₂, represented by the formula (Ib) 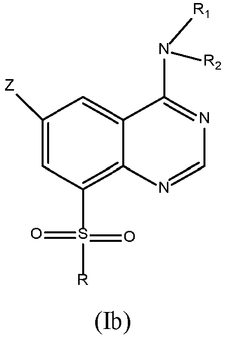 wherein
**Z** is aryl, wherein any of such aryl may be optionally substituted by one or more groups selected from (C₁-C₃)alkyl-, halo and (C₁-C₆)haloalkyl-;
**R₁** is H or (C₁-C₄)alkyl;
**R₂** is heteroaryl(C₁-C₄)alkyl-, wherein any of such alkyl and heteroaryl may be optionally substituted by one or more groups selected from (C₁-C₃)alkyl, (C₁-C₆)haloalkyl and halo;
**R** is -NR_{A}R_{B};
**R_{A}** and **R_{B}** are at each occurrence independently H or (C₁-C₆)alkyl-, or alternatively **R_{A}** and **R_{B}** may form together with the nitrogen atom to which they are attached a 5 or 6 membered saturated heterocyclic monocyclic ring system optionally containing a further heteroatom which is nitrogen or oxygen, which may be optionally substituted by one or more groups selected from halo and -OR₁.

6. A pharmaceutical composition comprising a compound as defined in any one of claims 1 to 5, or a pharmaceutically acceptable salt thereof, either alone or in combination with another one or more active ingredient, in admixture with one or more pharmaceutically acceptable carrier or excipient.

7. The pharmaceutical composition according to claim 6 for oral administration.

8. The compound according to any one of claims 1 to 5 or the pharmaceutical composition according to claims 6 and 7 for use as a medicament.

9. The compound according to any one of claims 1 to 5 or the pharmaceutical composition according to claims 6 and 7 for the use in the treatment of any disease wherein the P2X3 receptors are involved.

10. The compound according to any one of claims 1 to 5 or the pharmaceutical composition according to claims 6 and 7 for use in the prevention and/or treatment of respiratory diseases including cough, sub-acute or chronic cough, treatmentresistant cough, idiopathic chronic cough, post-viral cough, iatrogenic cough, asthma, idiopathic pulmonary fibrosis (IPF), chronic obstructive pulmonary disease (COPD) and cough associated with respiratory diseases such as COPD, asthma and bronchospasm.

11. The compound according to any one of claims 1 to 5 or the pharmaceutical composition according to claims 6 and 7 for use in the prevention and/or treatment of chronic cough.

## Patentansprüche

1. Eine Verbindung der Formel (I) 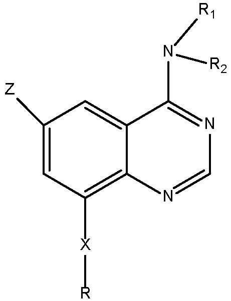 wobei
**X** ausgewählt wird aus S, SO₂, SO oder O;
**Z** ausgewählt wird aus der Gruppe bestehend aus Heteroaryl und Aryl, wobei jedes Heteroaryl und Aryl optional durch eine oder mehrere Gruppen bestehend aus (C₁-C₃)Alkyl-, Halogen und (C₁-C₆)Haloalkyl- substituiert werden kann;
**R₁** gleich H oder (C₁-C₄)Alkyl ist;
**R₂** gleich Heteroaryl(C₁-C₄)Alkyl- ist, wobei jedes Alkyl und Heteroaryl optional durch eine oder mehrere Gruppen ausgewählt aus (C₁-C₃)Alkyl, (C₁-C₆)Haloalkyl und Halogen substituiert werden kann;
**R** ausgewählt wird aus der Gruppe bestehend aus (C₁-C₆)Alkyl-, (C₁-C₆)Alkyl-CN, (C₁-C₆)Haloalkyl, -NR_{A}R_{B}, (C₃-C₈)Heterocycloalkyl-(C₁-C₆)Alkyl-, (C₃-C₈)Heterocycloalkyl-, (C₃-C₈)Heteroaryl-(C₁-C₆)Alkyl-, (C₃-C₈)Cycloalkyl-(C₁-C₆)Alkyl-, (C₃-C₈)Cycloalkyl-, R_{A}O-(C₁-C₆)Alkyl-O-(C₁-C₆)Alkyl-, R_{A}NH-C(O)-(C₁-C₄)Alkyl-, R_{A}R_{B}N-C(O)-(C₁-C₄)Alkyl-, R_{A}O-C(O)(C₁-C₆)Alkyl-,
wobei eines dieser Heterocycloalkyle durch eine oder mehrere Gruppen ausgewählt aus OH, -C(O)R_{A}, -C(O)OR_{A}, (C₁-C₆)Haloalkyl, -NH-C(O)R₁ und Oxo unsubstituiert oder substituiert wird;
jedes dieser Cycloalkyle durch eine oder mehrere Gruppen ausgewählt aus - C(O)OR_{A} und (C₁-C₆)Haloalkyl- substituiert wird;
jedes dieser Heteroaryle optional durch eine oder mehrere Gruppen ausgewählt aus (C₁-C₆)Alkyl-, -OH und (C₃-C₆)Cycloalkyl- substituiert werden kann;
**R_{A}** und **R_{B}** sind bei jedem Auftreten unabhängig H oder ausgewählt aus der Gruppe bestehend aus (C₁-C₆)Alkyl-, (C₁-C₆)Haloalkyl-, -OR₁, -SO₂R_{C} und (C₃-C₆)Cycloalkyl, wobei ein solches Cycloalkyl optional durch ein oder mehrere - C(O)OR₁ substituiert werden kann, oder alternativ
können **R_{A}** und **R_{B}** zusammen mit dem Stickstoffatom, an dem sie angehängt sind, ein 5- oder 6-gliedriges gesättigtes heterozyklisches monozyklisches Ringsystem bilden, das optional ein weiteres Heteroatom enthält, das Stickstoff oder Sauerstoff ist, das optional durch eine oder mehrere Gruppen bestehend aus Halo und -OR₁ substituiert werden kann; und
**R_{C}** ein Aryl ist;
mit der Maßgabe, dass R nur (C₁-C₆)Alkyl- oder unsubstituiertes (C₃-C₈)Heterocycloalkyl- ist, wenn X S oder SO ist.

2. Die Verbindung der Formel (I) nach Anspruch 1, ausgewählt aus der Gruppe bestehend aus:
8-(2,2-Difluorethoxy)-6-(4-Fluorphenyl)-N-((6-Methylpyridazin-3-yl)methyl)Chinazolin-4-Amin,
(R)-8-(2,2-Difluorethoxy)-6-(4-Fluorphenyl)-N-(1-(2-(Trifluormethyl)pyrimidin-5-yl)ethyl)Chinazolin-4-Amin,
8-(2,2-Difluorethoxy)-6-(5-Fluorpyridin-2-yl)-N-((6-Methylpyridazin-3-yl)methyl)Chinazolin-4-Amin,
(R)-8-(2,2-Difluorethoxy)-6-(5-Fluorpyridin-2-yl)-N-(1-(6-Methylpyridazin-3-yl)ethyl)Chinazolin-4-Amin,
Einzelenantiomer 1 von 8-(2,2-Difluorethoxy)-6-(5-Fluor-2-pyridyl)-N-[1-(5-Methyl-1,3,4-Thiadiazol-2-yl)ethyl] Chinazolin-4-Amin,
Einzelenantiomer 2 von 8-(2,2-Difluorethoxy)-6-(5-Fluor-2-pyridyl)-N-[1-(5-Methyl-1,3,4-thiadiazol-2-yl)ethyl]Chinazolin-4-Amin,
8-(2,2-Difluorethoxy)-N-((6-Methylpyridazin-3-yl) methyl)-6-(5-Methylpyridin-2-yl)Chinazolin-4-Amin,
8-(2,2-Difluorethoxy)-N-[(6-Methylpyridazin-3-yl)methyl]-6-(5-Methylpyrimidin-2-yl)Chinazolin-4-Amin,
(R)-6-(4-Fluorphenyl)-N-(1-(6-Methylpyridazin-3-yl)ethyl)-8-(Morpholinosulfonyl)Chinazolin-4-Amin,
((R)-6-(4-Fluorphenyl)-8-(Morpholinosulfonyl)-N-(1-(2-(Trifluoromethyl)pyrimidin-5-yl)ethyl)Chinazolin-4-Amin,
(Rac)-6-(4-Fluorphenyl)-N-(1-(5-Methyl-1,3,4-Thiadiazol-2-yl)ethyl)-8-(Morpholinosulfonyl)Chinazolin-4-Amin,
(R)-6-(4-Fluorphenyl)-N,N-Dimethyl-4-((1-(6-Methylpyridazin-3-yl)ethyl)amino)Chinazolin-8-Sulfonamid,
6-(4-Fluorphenyl)-N,N-dimethyl-4-((1-(5-methyl-1,3,4-Thiadiazol-2-yl)ethyl)amino)Chinazolin-8-Sulfonamid,
6-(4-Fluorphenyl)-N,N-Dimethyl-4-((1-(5-Methyl-1,2,4-Oxadiazol-3-yl)ethyl)amino)Chinazolin-8-Sulfonamid,
(R)-8-((3,3-Difluorpyrrolidin-1-yl)sulfonyl)-6-(4-Fluorphenyl)-N-(1-(6-Methylpyridazin-3-yl)ethyl)Chinazolin-4-Amin,
(R)-8-((3,3-Difluorpyrrolidin-1-yl)sulfonyl)-6-(4-Fluorphenyl)-N-(1-(5-Methyl-1,2,4-Oxadiazol-3-yl)ethyl) Chinazolin-4-Amin,
8-((3,3-Difluorpyrrolidin-1-yl)sulfonyl)-6-(4-Fluorophenyl)-N-(1-(5-Methyl-1,3,4-Thiadiazol-2-yl)ethyl)Chinazolin-4-Amin,
(R)-1-((6-(4-Fluorphenyl)-4-((1-(6-Methylpyridazin-3-yl)ethyl)amino)Chinazolin-8-yl)sulfonyl)piperidin-4-ol,
(R)-1-((6-(4-Fluorphenyl)-4-((1-(5-Methyl-1,2,4-Oxadiazol-3-yl)ethyl)amino)Chinazolin-8-yl)sulfonyl)piperidin-4-ol,
(R)-6-(4-Fluorphenyl)-N-Methyl-4-((1-(6-Methylpyridazin-3-yl)ethyl)amino)Chinazolin-8-Sulfonamid,
(R)-6-(4-Fluorphenyl)-N-Methyl-4-((1-(5-Methyl-1,2,4-Oxadiazol-3-yl)ethyl)amino)Chinazolin-8-Sulfonamid,
(6-(4-Fluorphenyl)-N-Methyl-4-((1-(5-Methyl-1,3,4-Thiadiazol-2-yl)ethyl)amino)Chinazolin-8-Sulfonamid,
(R)-6-(4-Fluorphenyl)-N-(1-(6-Methylpyridazin-3-yl)ethyl)-8-(piperazin-1-ylsulfonyl)Chinazolin-4-Amin,
(R)-6-(4-Fluorphenyl)-N-(1-(5-Methyl-1,2,4-Oxadiazol-3-yl)ethyl)-8-(piperazin-1-ylsulfonyl)Chinazolin-4-Amin,
(R)-6-(4-Fluorphenyl)-N-(1-(6-Methylpyridazin-3-yl)ethyl)-8-(Methylthio)Chinazolin-4-Amin,
6-(4-Fluorphenyl)-N-((R)-1-(6-Methylpyridazin-3-yl)ethyl)-8-(Methylsulfinyl)Chinazolin-4-Amin,
(R)-6-(4-Fluorphenyl)-N-(1-(6-Methylpyridazin-3-yl)ethyl)-8-(Oxetan-3-ylthio)Chinazolin-4-Amin,
6-(4-Fluorphenyl)-N-((R)-1-(6-Methylpyridazin-3-yl)ethyl)-8-(Oxetan-3-ylsulfinyl)Chinazolin-4-Amin,
(1-((S)-3-((6-(4-Fluorphenyl)-4-(((R)-1-(2-(Trifluormethyl)pyrimidin-5-yl)ethyl)amino)Chinazolin-8-yl)oxy)pyrrolidin-1-yl)ethan-1-on,
(1-((R)-3-((6-(4-Fluorphenyl)-4-(((R)-1-(2-(Trifluormethyl)pyrimidin-5-yl)ethyl)amino)Chinazolin-8-yl)oxy)pyrrolidin-1-yl)ethan-1-on,
N-((1S,4s)-4-((6-(4-Fluorphenyl)-4-(((R)-1-(2-(Trifluormethyl)pyrimidin-5-yl)amino)Chinazolin-8-yl)oxy)cyclohexyl)acetamid,
6-(4-Fluorphenyl)-8-((1-(2,2,2-Trifluorethyl)pyrrolidin-3-yl)oxy)-N-((R)-1-(2-(Trifluormethyl)pyrimidin-5-yl)ethyl)Chinazolin-4-Amin,
3-((6-(4-Fluorphenyl)-4-(((R)-1-(2-(Trifluormethyl)pyrimidin-5-yl)ethyl)amino)Chinazolin-8-yl)oxy)pyrrolidin-2-on,
(R)-1-(4-((6-(4-Fluorphenyl)-4-((1-(2-(Trifluormethyl)pyrimidin-5-yl)ethyl)amino)Chinazolin-8-yl)oxy)piperidin-1-yl)ethan-1-on,
(R)-6-(4-Fluorphenyl)-8-((1-(2,2,2-trifluoroethyl)piperidin-4-yl)oxy)-N-(1-(2-(trifluoromethyl)pyrimidin-5-yl)ethyl)Chinazolin-4-Amin,
(R)-3-(((6-(4-Fluorphenyl)-4-((1-(2-(Trifluormethyl)pyrimidin-5-yl)ethyl)amino)Chinazolin-8-yl)oxy)methyl)oxetan-3-ol,
(R)-5-(((6-(4-Fluorphenyl)-4-((1-(2-(Trifluormethyl)pyrimidin-5-yl)ethyl)amino)Chinazolin-8-yl)oxy)methyl)isoxazol-3-ol,
(R)-2-((6-(4-Fluorphenyl)-4-((1-(2-(Trifluormethyl)pyrimidin-5-yl)ethyl)amino)Chinazolin-8-yl)oxy)Essigsäure,
(R)-2-((6-(4-Fluorphenyl)-4-((1-(2-(Trifluormethyl)pyrimidin-5-yl)ethyl)amino)Chinazolin-8-yl)oxy)-N-(phenylsulfonyl)acetamid,
(R)-1-(((6-(4-Fluorphenyl)-4-((1-(2-(trifluoromethyl)pyrimidin-5-yl)ethyl)amino)Chinazolin-8-yl)oxy)methyl)cyclopropan-1-Carbonsäure,
Methyl 1-(2-((6-(4-Fluorphenyl)-4-(((6-Methylpyridazin-3-yl)methyl)amino)Chinazolin-8-yl)oxy)acetamido)cyclopropan-1-Carboxylat,
Methyl (R)-1-(2-((6-(4-Fluorphenyl)-4-((1-(2-(Trifluormethyl)pyrimidin-5-yl)ethyl)amino)Chinazolin-8-yl)oxy) acetamido)cyclopropan-1-Carboxylat,
(R)-1-(2-((6-(4-Fluorphenyl)-4-((1-(2-(Trifluormethyl)pyrimidin-5-yl)ethyl)amino)Chinazolin-8-yl)oxy)acetamido)cyclopropan-1-Carbonsäure,
(1R,2S)-2-(2-((6-(4-Fluorphenyl)-4-(((6-Methylpyridazin-3 yl)methyl)amino)Chinazolin-8-yl)oxy)acetamido)cyclopentan-1-Carbonsäure,
Ethyl (1S,2R)-2-(2-((6-(4-Fluorphenyl)-4-(((6-Methylpyridazin-3-yl)methyl)amino)Chinazolin-8-yl)oxy)acetamido)cyclopentan-1-Carboxylat,
(R)-4-(((6-(4-Fluorphenyl)-4-((1-(2-(Trifluormethyl)pyrimidin-5-yl)ethyl)amino)Chinazolin-8-yl)oxy)methyl)piperidin-4-Carbonsäurehydrochlorid,
(R)-1-(((6-(4-Fluorphenyl)-4-((1-(2-(Trifluormethyl)pyrimidin-5-yl)ethyl)amino)Chinazolin-8-yl)oxy)methyl)cyclohexan-1-Carbonsäure,
Ammonium (2-((4-(((6-Methylpyridazin-3-yl)methyl)amino)-6-(5-Methylpyrimidin-2-yl)Chinazolin-8-yl)oxy)acetyl)(phenylsulfonyl)Amid,
6-(4-Fluorphenyl)-8-[(3-Methyloxetan-3-yl)methoxy]-N-[(6-Methylpyridazin-3-yl)methyl]Chinazolin-4-Amin,
6-(4-Fluorphenyl)-N-[(6-Methylpyridazin-3-yl)methyl]-8-[[1-(Trifluormethyl)cyclopropyl]methoxy]Chinazolin-4-Amin,
Tert-butyl 3-[6-(4-Fluorphenyl)-4-[(6-Methylpyridazin-3-yl)methylamino]Chinazolin-8-yl]oxyazetidin-1-Carboxylat,
2-[6-(4-Fluorphenyl)-4-[(6-Methylpyridazin-3-yl)methylamino]Chinazolin-8-yl]Oxyacetonitril,
2-[6-(4-Fluorphenyl)-4-[(6-Methylpyridazin-3-yl)methylamino]Chinazolin-8-yl]oxy-N-methoxy-N-Methyl-Acetamid,
2-[2-[6-(4-Fluorphenyl)-4-[(6-Methylpyridazin-3-yl)methylamino]Chinazolin-8-yl]oxyethoxy]ethanol,
3-[6-(4-Fluorphenyl)-4-[(6-Methylpyridazin-3-yl)methylamino]Chinazolin-8-yl]oxy-1-methyl-pyrrolidin-2-on,
6-(4-Fluorphenyl)-N-[(6-Methylpyridazin-3-yl)methyl]-8-(Oxazol-2-ylmethoxy)Chinazolin-4-Amin,
6-(4-Fluorphenyl)-8-[(5-Methyl-1,2,4-Oxadiazol-3-yl)methoxy]-N-[(6-Methylpyridazin-3-yl)methyl]Chinazolin-4-Amin,
8-[(3-Ethyl-1,2,4-Oxadiazol-5-yl)methoxy]-6-(4-Fluorphenyl)-N-[(6-Methylpyridazin-3-yl)methyl]Chinazolin-4-Amin,
8-[(5-Cyclopropyl-1,3,4-Thiadiazol-2-yl)methoxy]-6-(4-Fluorphenyl)-N-[(6-Methylpyridazin-3-yl)methyl]Chinazolin-4-Amin,
2-[6-(4-Fluorphenyl)-4-[(6-Methylpyridazin-3-yl)methylamino]Chinazolin-8-yl]oxy-N-(2,2,2-Trifluorethyl)acetamid,
8-((5-Cyclopropyl-1,3,4-Oxadiazol-2-yl)methoxy)-6-(4-Fluorphenyl)-N-((6-Methylpyridazin-3-yl)methyl)Chinazolin-4-Amin,
6-(4-Fluorphenyl)-N-((6-Methylpyridazin-3-yl)methyl)-8-(Oxetan-3-yloxy)Chinazolin-4-Amin,
Methyl 4-[[6-(4-Fluorphenyl)-4-[(6-Methylpyridazin-3-yl)methylamino]Chinazolin-8-yl]oxymethyl]piperidin-1-Carboxylat,
Methyl 4-[6-(4-Fluorphenyl)-4-[(6-Methylpyridazin-3-yl)methylamino]Chinazolin-8-yl]oxypiperidin-1-Carboxylat,
Methyl 3-[[6-(4-Fluorphenyl)-4-[(6-Methylpyridazin-3-yl)methylamino]Chinazolin-8-yl]oxymethyl]piperidin-1-Carboxylat,
Methyl 3-[[6-(4-Fluorphenyl)-4-[(6-Methylpyridazin-3-yl)methylamino]Chinazolin-8-yl]oxymethyl]pyrrolidin-1-Carboxylat,
Methyl 3-[6-(4-Fluorphenyl)-4-[(6-Methylpyridazin-3-yl)methylamino]Chinazolin-8-yl]oxypyrrolidin-1-Carboxylat,

3. Verbindung der Formel (I) nach Anspruch 1, wobei
**X** aus S oder SO ausgewählt ist;
**Z** gleich Aryl ist, wobei ein solches Aryl optional durch eine oder mehrere Gruppen ausgewählt aus (C₁-C₃)Alkyl, Halogen und (C₁-C₆)Haloalkyl substituiert werden kann;
**R₁** gleich H oder (C₁-C₄)Alkyl ist;
**R2** gleich Heteroaryl(C₁-C₄)Alkyl- ist, wobei jedes Alkyl und Heteroaryl optional durch eine oder mehrere Gruppen ausgewählt aus (C₁-C₃)Alkyl, (C₁-C₆)Haloalkyl und Halogen substituiert werden kann;
**R** ausgewählt wird aus der Gruppe bestehend aus (C₁-C₆)Alkyl- und (C₃-C₈)Heterocycloalkyl-.

4. Verbindung der Formel (I) nach Anspruch 1, wobei X gleich O ist, dargestellt durch die Formel (Ia) 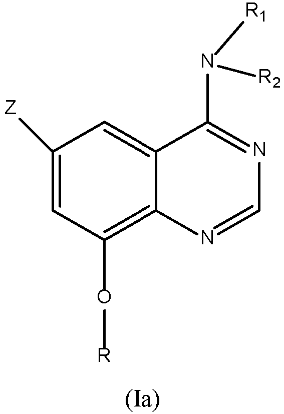 wobei
**Z** ausgewählt wird aus der Gruppe bestehend aus Heteroaryl und Aryl, wobei jedes Heteroaryl und Aryl optional durch eine oder mehrere Gruppen bestehend aus (C₁-C₃)Alkyl-, Halogen und (C₁-C₆)Haloalkyl- substituiert werden kann;
**R₁** gleich H oder (C₁-C₄)Alkyl ist;
**R₂** gleich Heteroaryl(C1-C4)alkyl- ist, wobei jedes Alkyl und Heteroaryl optional durch eine oder mehrere Gruppen ausgewählt aus (C₁-C₃)Alkyl, (C₁-C₆)Haloalkyl und Halogen substituiert werden kann,
**R** ausgewählt wird aus der Gruppe bestehend aus (C₁-C₆)Alkyl-CN, (C₁-C₆)Haloalkyl, -NR_{A}R_{B}, (C₃-C₈)Heterocycloalkyl-(C₁-C₆)Alkyl-, (C₃-C₈)Heterocycloalkyl-, (C₃-C₈)Heteroaryl-(C₁-C₆)Alkyl-, (C₃-C₈)Cycloalkyl-(C1-C₆)Alkyl-, (C₃-C₈)Cycloalkyl-, R_{A}O-(C₁-C₆)Alkyl-O-(C₁-C₆)Alkyl-, R_{A}NH-C(O)-(C₁-C₄)Alkyl-, R_{A}R_{B}N-C(O)-(C₁-C₄)Alkyl- und R_{A}O-C(O)(C₁-C₆)Alkyl-,
wobei jedes dieser Heterocycloalkyle durch eine oder mehrere Gruppen ausgewählt aus -OH, -C(O)R_{A}, -C(O)OR_{A}, (C₁-C₆)Haloalkyl, -NH-C(O)R₁ und Oxo substituiert wird;
jedes dieser Cycloalkyle durch eine oder mehrere Gruppen ausgewählt aus - C(O)OR_{A} und (C₁-C₆)Haloalkyl- substituiert wird;
jedes dieser Heteroaryle optional durch eine oder mehrere Gruppen ausgewählt aus (C₁-C₆)Alkyl-, -OH und (C₃-C₆)Cycloalkyl- substituiert werden kann;
**R_{A}** und **R_{B}** bei jedem Auftreten unabhängig H oder ausgewählt aus der Gruppe bestehend aus (C₁-C₆)Alkyl-, (C₁-C₆)Haloalkyl-, -OR₁, -SO₂R_{C} und (C₃-C₆)Cycloalkyl sind, wobei solches Cycloalkyl optional durch ein oder mehrere - C(O)OR₁ substituiert werden kann; und
**R_{C}** gleich Aryl ist.

5. Die Verbindung der Formel (I) nach Anspruch 1, wobei X gleich SO₂ ist, dargestellt durch die Formel (Ib) 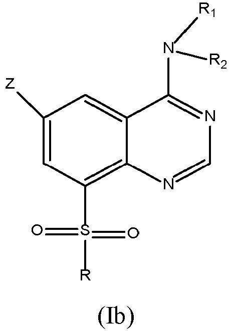 wobei
**Z** gleich Aryl ist, wobei jedes Aryl optional durch eine oder mehrere Gruppen ausgewählt aus (C₁-C₃)Alkyl, Halogen und (C₁-C₆)Haloalkyl substituiert werden kann;
**R₁** gleich H oder (C₁-C₄)Alkyl ist;
**R2** gleich Heteroaryl(C₁-C₄)Alkyl- ist, wobei jedes Alkyl und Heteroaryl optional durch eine oder mehrere Gruppen ausgewählt aus (C₁-C₃)Alkyl, (C₁-C₆)Haloalkyl und Halogen substituiert werden kann;
**R** gleich -NR_{A}R_{B} ist;
**R_{A}** und **R_{B}** bei jedem Auftreten unabhängig H oder (C₁-C₆)Alkyl-, sind oder alternativ
**R_{A}** und **R_{B}** zusammen mit dem Stickstoffatom, an dem sie angehängt sind, ein 5-oder 6-gliedriges gesättigtes heterozyklisches monozyklisches Ringsystem bilden können, das optional ein weiteres Heteroatom enthält, das Stickstoff oder Sauerstoff ist, das optional durch eine oder mehrere Gruppen bestehend aus Halogen und -OR₁ substituiert werden kann.

6. Eine pharmazeutische Zusammensetzung, die eine Verbindung nach einem der Ansprüche 1 bis 5 oder ein pharmazeutisch verträgliches Salz davon umfasst, entweder allein gegeben oder in Kombination mit einem oder mehreren anderen Wirkstoffen, gemischt mit einem oder mehreren pharmazeutisch verträglichen Trägern oder Hilfsstoffen.

7. Pharmazeutische Zusammensetzung nach Anspruch 6 zur oralen Verabreichung.

8. Verbindung gemäß einem der Ansprüche 1 bis 5 oder pharmazeutische Zusammensetzung gemäß den Ansprüchen 6 und 7 zur Verwendung als Arzneimittel.

9. Verbindung nach einem der Ansprüche 1 bis 5 oder eine pharmazeutische Zusammensetzung nach den Ansprüchen 6 und 7 zur Anwendung bei der Behandlung einer Krankheit, an der die P2X3-Rezeptoren beteiligt sind.

10. Verbindung nach einem der Ansprüche 1 bis 5 oder eine pharmazeutische Zusammensetzung nach den Ansprüchen 6 und 7 zur Anwendung bei der Vorbeugung und/oder Behandlung von Atemwegserkrankungen, einschließlich Husten, subakutem oder chronischem Husten, behandlungsresistentem Husten, idiopathischem chronischem Husten, postviralem Husten, iatrogenem Husten, Asthma, idiopathischer Lungenfibrose (IPF), chronisch obstruktiver Lungenerkrankung (COPD) und Husten im Zusammenhang mit Atemwegserkrankungen wie COPD, Asthma und Bronchospasmus.

11. Verbindung nach einem der Ansprüche 1 bis 5 oder eine pharmazeutische Zusammensetzung nach den Ansprüchen 6 und 7 zur Anwendung bei der Vorbeugung und/oder Behandlung von chronischem Husten.

## Revendications

1. Un composé de formule (I) 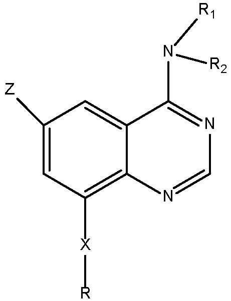 dans lequel
**X** est choisi parmi S, SO₂, SO ou O ;
**Z** est choisi parmi le groupe constitué par un hétéroaryle et un aryle, dans lequel ledit hétéroaryle et aryle est éventuellement substitué de manière facultative par un ou plusieurs groupes choisis parmi un alkyle (C1-C3), un halo et un haloalkyle (C1-C6) ;
**R₁** est H ou alkyle (C₁-C₄) ;
**R₂** est un hétéroaryle alkyle (C1-C4) ; dans lequel ledit alkyle ou hétéroaryle est éventuellement substitué de manière facultative par un ou plusieurs groupes choisis parmi un alkyle (C₁-C₃), un haloalkyle (C₁-C₆) et un halo ;
**R** est choisi parmi le groupe constitué par un alkyle (C₁-C₆), alkyle (C₁-C₆)-CN, haloalkyle (C₁-C₆), -NR_{A}R_{B}, hétérocycloalkyle (C₃-C₈)-alkyle (C₁-C₆), hétérocycloalkyle (C₃-C₈), hétéroaryle (C₃-C₈) alkyle (C₁-C₆), cycloalkyle (C₃-C₈)-alkyle (C₁-C₆), cycloalkyle (C₃-C₈), R_{A}O-alkyle (C₁-C₆)-O-alkyle (C₁-C₆), R_{A}NH-C(O)-alkyle (C₁-C₄), R_{A}R_{B}N-C(O)-alkyle (C₁-C₄), R_{A}O-C(O)alkyle (C₁-C₆),
dans lequel ledit hétérocycloalkyle est non substitué ou substitué par un ou plusieurs groupes choisis parmi OH, -C(O)R_{A}, -C(O)OR_{A}, un haloalkyle (C1-C6), -NH-C(O)R₁ et un oxo ;
ledit cycloalkyle est substitué par un ou plusieurs groupes choisis parmi -C(O)OR_{A} et un haloalkyle (C₁-C₆),
ledit hétéroaryle est éventuellement substitué de manière facultative par un ou plusieurs groupes choisis parmi un alkyle (C₁-C₆), -OH et un cycloalkyle (C₃-C₆) ; **R_{A}** et **R_{B}** sont à chaque occurrence indépendamment H ou choisis parmi le groupe constitué par un alkyle (C₁-C₆), haloalkyle (C₁-C₆), -OR₁, -SO₂R_{C} et cycloalkyle (C₃-C₆) dans lequel ledit cycloalkyle est éventuellement substitué de manière facultative par un ou plusieurs -C(O)OR₁ ou alternativement
**R_{A}** et **R_{B}** peuvent former ensemble, avec l'atome d'azote auquel ils sont liés, un système d'anneau monocyclique hétérocyclique saturé à 5 ou 6 chaînons contenant de manière facultative un autre hétéroatome qui est l'azote ou l'oxygène, qui est éventuellement substitué de manière facultative par un ou plusieurs groupes choisis parmi un halo et -OR₁ ; et
**R_{C}** est un aryle ;
à la condition que R soit alkyle (C₁-C₆) alkyle ou hétérocycloalkyle non substitué (C₃-C₈) uniquement lorsque X est S ou SO.

2. Composé de formule (I) selon la revendication 1, choisi parmi le groupe constitué par :
8-(2,2-difluoroéthoxy)-6-(4-fluorophényl)-N-((6-méthylpyridazin-3-yl)méthyl)quinazoline-4-amine,
(R)-8-(2,2-difluoroéthoxy)-6-(4-fluorophényl)-N-(1-(2-(trifluorométhyl)pyrimidin-5-yl)ethyl)quinazoline-4-amine,
8-(2,2-difluoroéthoxy)-6-(5-fluoropyridine-2-yl)-N-((6-méthylpyridazin-3-yl)methyl)quinazoline-4-amine,
(R)-8-(2,2-difluoroéthoxy)-6-(5-fluoropyridine-2-yl)-N-(1-(6-methylpyridazine-3-yl)éthyl)quinazoline-4-amine,
Énantiomère unique 1 de 8-(2,2-difluoroéthoxy)-6-(5-fluoro-2-pyridyl)-N-[1-(5-méthyl-1,3,4-thiadiazol-2-yl)éthyl]quinazoline-4-amine,
Énantiomère unique 2 de 8-(2,2-difluoroéthoxy)-6-(5-fluoro-2-pyridyl)-N-[1-(5-méthyl-1,3,4-thiadiazol-2-yl)éthyl]quinazoline-4-amine,
8-(2,2-Difluoroéthoxy)-N-((6-méthylpyridazine-3-yl) méthyl)-6-(5-méthylpyridine-2-yl) quinazoline-4-amine,
8-(2,2-difluoroéthoxy)-N-[(6-méthylpyridazine-3-yl)méthyl]-6-(5-méthylpyrimidine-2-yl)quinazoline-4-amine,
(R)-6-(4-fluorophényl)-N-(1-(6-méthylpyridazine-3-yl)éthyl)-8-(morpholinosulfonyl)quinazoline-4-amine,
((R)-6-(4-fluorophényl)-8-(morpholinosulfonyl)-N-(1-(2-(trifluorométhyl)pyrimidine-5-yl)éthyl)quinazoline-4-amine,
(Rac)-6-(4-fluorophényl)-N-(1-(5-méthyl-1,3,4-thiadiazol-2-yl)éthyl)-8-(morpholinosulfonyl)quinazoline-4-amine,
(R)-6-(4-fluorophényl)-N,N-diméthyl-4-((1-(6-méthylpyridazin-3-yl)éthyl)amino)quinazoline-8-sulfonamide,
6-(4-fluorophényl)-N,N-diméthyl-4-((1-(5-méthyl-1,3,4-thiadiazol-2-yl)éthyl)amino)quinazoline-8-sulfamide,
6-(4-fluorophényl)-N,N-diméthyl-4-((1-(5-méthyl-1,2,4-oxadiazol-3-yl)éthyl)amino)quinazoline-8-sulfonamide,
(R)-8-((3,3-difluoropyrrolidine-1-yl)sulfonyl)-6-(4-fluorophényl)-N-(1-(6-méthylpyridazin-3-yl)éthyl)quinazoline-4-amine,
(R)-8-((3,3-difluoropyrrolidine-1-yl)sulfonyl)-6-(4-fluorophényl)-N-(1-(5-méthyl-1,2,4-oxadiazol-3-yl)éthyl)quinazoline-4-amine,
8-((3,3-difluoropyrrolidine-1-yl)sulfonyl)-6-(4-fluorophényl)-N-(1-(5-méthyl-1,3,4-thiadiazol-2-yl)éthyl)quinazoline-4-amine,
(R)-1-((6-(4-fluorophényl)-4-((1-(6-méthylpyridazine-3-yl)éthyl)amino)quinazoline-8-yl)sulfonyl)pipéridine-4-ol,
(R)-1-((6-(4-fluorophényl)-4-((1-(5-méthyl-1,2,4-oxadiazol-3-yl)éthyl)amino)quinazolin-8-yl)sulfonyl)pipéridine-4-ol,
(R)-6-(4-fluorophényl)-N-méthyl-4-((1-(6-méthylpyridazin-3-yl)éthyl)amino)quinazoline-8-sulfonamide,
(R)-6-(4-fluorophényl)-N-méthyl-4-((1-(5-méthyl-1,2,4-oxadiazol-3-yl)éthyl)amino)quinazoline-8-sulfonamide,
(6-(4-fluorophényl)-N-méthyl-4-((1-(5-méthyl-1,3,4-thiadiazol-2-yl)éthyl)amino)quinazoline-8-sulfonamide,
(R)-6-(4-fluorophényl)-N-(1-(6-méthylpyridazine-3-yl)éthyl)-8-(pipérazine-1-ylsulfonyl)quinazoline-4-amine,
(R)-6-(4-fluorophényl)-N-(1-(5-méthyl-1,2,4-oxadiazol-3-yl)éthyl)-8-(pipérazine-1-ylsulfonyl)quinazoline-4-amine,
(R)-6-(4-fluorophényl)-N-(1-(6-méthylpyridazine-3-yl)éthyl)-8-(méthylthio)quinazoline-4-amine,
6-(4-fluorophényl)-N-((R)-1-(6-méthylpyridazine-3-yl)éthyl)-8-(méthylsulfinyl) quinazoline-4-amine,
(R)-6-(4-fluorophényl)-N-(1-(6-méthylpyridazine-3-yl)éthyl)-8-(oxétan-3-ylthio)quinazoline-4-amine,
6-(4-fluorophényl)-N-((R)-1-(6-méthylpyridazine-3-yl)éthyl)-8-(oxétan-3-ylsulfinyl)quinazoline-4-amine,
(1-((S)-3-((6-(4-fluorophényl)-4-(((R)-1-(2-(trifluorométhyl)pyrimidine-5-yl)éthyl)amino)quinazoline-8-yl)oxy)pyrrolidine-1-yl)éthan-1-one,
(1-((R)-3-((6-(4-fluorophényl)-4-(((R)-1-(2-(trifluorométhyl)pyrimidine-5-yl)éthyl)amino)quinazoline-8-yl)oxy)pyrrolidine-1-yl)éthan-1-one,
N-((1 S,4s)-4-((6-(4-fluorophényl)-4-(((R)-1-(2-(trifluorométhyl)pyrimidine-5-yl)éthyl)amino)quinazoline-8-yl)oxy)cyclohéxyl)acétamide,
6-(4-fluorophényl)-8-((1-(2,2,2-trifluoroéthyl)pyrrolidine-3-yl)oxy)-N-((R)-1-(2-(trifluorométhyl)pyrimidine-5-yl)éthyl)quinazoline-4-amine,
3-((6-(4-fluorophényl)-4-(((R)-1-(2-(trifluorométhyl)pyrimidine-5-yl)éthyl)amino)quinazoline-8-yl)oxy)pyrrolidine-2-one,
(R)-1-(4-((6-(4-fluorophényl)-4-((1-(2-(trifluorométhyl)pyrimidine-5-yl)éthyl)amino)quinazoline-8-yl)oxy)pipéridine-1-yl)éthan-1-one,
(R)-6-(4-fluorophényl)-8-((1-(2,2,2-trifluoroéthyl)pipéridine-4-yl)oxy)-N-(1-(2-(trifluorométhyl)pyrimidine-5-yl)éthyl)quinazoline-4-amine,
(R)-3-(((6-(4-fluorophényl)-4-((1-(2-(trifluorométhyl)pyrimidine-5-yl)éthyl)amino)quinazoline-8-yl)oxy)méthyl)oxétan-3-ol,
(R)-5-(((6-(4-fluorophényl)-4-((1-(2-(trifluorométhyl)pyrimidine-5-yl)éthyl)amino)quinazoline-8-yl)oxy)méthyl)isoxazol-3-ol,
(R)-2-((6-(4-fluorophényl)-4-((1-(2-(trifluoromethyl)pyrimidine-5-yl)ethyl)amino)quinazoline-8-yl)oxy)acide acétique,
(R)-2-((6-(4-fluorophényl)-4-((1-(2-(trifluorométhyl)pyrimidine-5-yl)éthyl)amino)quinazoline-8-yl)oxy)-N-(phénylsulfonyl)acétamide,
(R)-1-(((6-(4-fluorophényl)-4-((1-(2-(trifluorométhyl)pyrimidine-5-yl)éthyl)amino)quinazoline-8-yl)oxy)méthyl)cyclopropane-1-acide carboxylique,
Méthyl 1-(2-((6-(4-fluorophényl)-4-(((6-méthylpyridazine-3-yl)méthyl)amino)quinazoline-8-yl)oxy)acétamido)cyclopropane-1-carboxylate,
Méthyle (R)-1-(2-((6-(4-fluorophényl)-4-((1-(2-(trifluorométhyl)pyrimidin-5-yl)éthyl)amino) quinazoline-8-yl)oxy) acétamido) cyclopropane-1-carboxylate,
(R)-1-(2-((6-(4-fluorophényl)-4-((1-(2-(trifluorométhyl)pyrimidine-5-yl)éthyl)amino)quinazoline-8-yl)oxy)acetamido)acide cyclopropane-1-acide carboxylique,
(1R,2S)-2-(2-((6-(4-fluorophényl)-4-(((6-méthylpyridazine-3 yl)méthyl)amino)quinazoline-8-yl)oxy)acetamido)cyclopentane-1-acide carboxylique,
Éthyle (1S,2R)-2-(2-((6-(4-fluorophényl)-4-(((6-méthylpyridazine-3-yl)méthyl)amino)quinazoline-8-yl)oxy)acétamid)cyclopentane-1-carboxylate,
(R)-4-(((6-(4-fluorophényl)-4-((1-(2-(trifluorométhyl)pyrimidine-5-yl)éthyl)amino)quinazoline-8-yl)oxy)méthyl)pipéridine-4-chlorhydrate d'acide carboxylique,
(R)-1-(((6-(4-fluorophényl)-4-((1-(2-(trifluorométhyl)pyrimidine-5-yl)éthyl)amino)quinazoline-8-yl)oxy)méthyl)cyclohexane-1-acide carboxylique,
Ammonium (2-((4-((6-méthylpyridazine-3-yl)méthyl)amino)-6-(5-méthylpyrimidin-2-yl)quinazoline-8-yl)oxy)acétyl)(phénylsulfonyl)amide,
6-(4-fluorophényl)-8-[(3-méthyloxétan-3-yl)méthoxy]-N-[(6-méthylpyridazine-3-yl)méthyl]quinazoline-4-amine,
6-(4-fluorophényl)-N-[(6-méthylpyridazine-3-yl)méthyl]-8-[[1-(trifluorométhyl)cyclopropyl]méthoxy]quinazoline-4-amine,
tert-butyl 3-[6-(4-fluorophényl)-4-[(6-méthylpyridazine-3-yl)méthylamino]quinazoline-8-yl]oxyazétidine-1-carboxylate,
2-[6-(4-fluorophényl)-4-[(6-méthylpyridazine-3-yl)méthylamino]quinazoline-8-yl]oxyacétonitrile,
2-[6-(4-fluorophényl)-4-[(6-méthylpyridazine-3-yl)méthylamino]quinazoline-8-yl]oxy-N-méthoxy-N-méthyl-acétamide,
2-[2-[6-(4-fluorophényl)-4-[(6-méthylpyridazine-3-yl)méthylamino]quinazoline-8-yl]oxyethoxy]éthanol,
3-[6-(4-fluorophényl)-4-[(6-méthylpyridazine-3-yl)méthylamino]quinazoline-8-yl]oxy-1-méthyl-pyrrolidine-2-one,
6-(4-fluorophényl)-N-[(6-méthylpyridazine-3-yl)méthyl]-8-(oxazol-2-ylméthoxy)quinazoline-4-amine,
6-(4-fluorophényl)-8-[(5-méthyl-1,2,4-oxadiazol-3-yl)méthoxy]-N-[(6-méthylpyridazine-3-yl)méthyl]quinazoline-4-amine,
8-[(3-éthyl-1,2,4-oxadiazol-5-yl)méthoxy]-6-(4-fluorophényl)-N-[(6-méthylpyridazine-3-yl)méthyl]quinazoline-4-amine,
8-[(5-cyclopropyl-1,3,4-thiadiazol-2-yl)méthoxy]-6-(4-fluorophényl)-N-[(6-méthylpyridazine-3-yl)méthyl]quinazoline-4-amine,
2-[6-(4-fluorophényl)-4-[(6-méthylpyridazine-3-yl)méthylamino]quinazoline-8-yl]oxy-N-(2,2,2-trifluoroéthyl)acétamide,
8-((5-cyclopropyl-1,3,4-oxadiazol-2-yl)méthoxy)-6-(4-fluorophényl)-N-((6-méthylpyridazine-3-yl)méthyl)quinazoline-4-amine,
6-(4-fluorophényl)-N-((6-méthylpyridazine-3-yl)méthyl)-8-(oxétan-3-yloxy)quinazoline-4-amine,
Méthyl 4-[[6-(4-fluorophényl)-4-[(6-méthylpyridazine-3-yl)méthylamino]quinazoline-8-yl]oxyméthyl]pipéridine-1-carboxylate,
Méthyl 4-[6-(4-fluorophényl)-4-[(6-méthylpyridazine-3-yl)méthylamino]quinazoline-8-yl]oxypipéridine-1-carboxylate,
Méthyl 3-[[6-(4-fluorophényl)-4-[(6-méthylpyridazine-3-yl)méthylamino]quinazoline-8-yl]oxyméthyl]pipéridine-1-carboxylate,
Méthyl 3-[[6-(4-fluorophényl)-4-[(6-méthylpyridazine-3-yl)méthylamino]quinazoline-8-yl]oxyméthyl]pyrrolidine-1-carboxylate,
Méthyl 3-[6-(4-fluorophényl)-4-[(6-méthylpyridazine-3-yl)méthylamino]quinazoline-8-yl]oxyméthyl]pyrrolidine-1-carboxylate,

3. Composé de formule (I) selon la revendication 1, dans lequel
**X** est choisi parmi S ou SO ;
**Z** est un aryle, dans lequel ledit aryle est éventuellement substitué de manière facultative par un ou plusieurs groupes choisis parmi un alkyle (C1-C3), un halo (C1-C6) et un haloalkyle ;
**R₁** est H ou un alkyle (C₁-C₄) ;
**R₂** est un hétéroaryle alkyle (C1-C4) ; dans lequel ledit alkyle ou hétéroaryle est éventuellement substitué de manière facultative par un ou plusieurs groupes choisis parmi un alkyle (C₁-C₃), un haloalkyle (C1-C6) et un halo ;
**R** est choisi parmi le groupe constitué par un alkyle (C1-C6) et un hétérocycloalkyle (C₃-C₈).

4. Composé de formule (I) selon la revendication 1, dans lequel X est O, représenté par la formule (Ia) 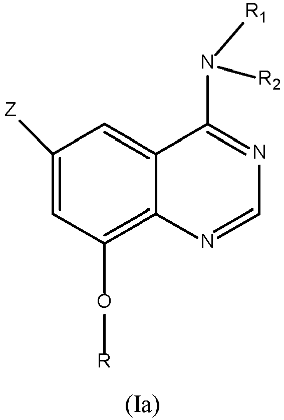 dans lequel
**Z** est choisi parmi le groupe constitué par un hétéroaryle et un aryle, dans lequel ledit hétéroaryle ou aryle est éventuellement substitué de manière facultative par un ou plusieurs groupes choisis parmi un alkyle (C1-C3), un halo et un haloalkyle (C1-C6) ;
**R₁** est H ou un alkyle (C₁-C₄) ;
**R₂** est un hétéroaryle alkyle (C₁-C₄), dans lequel ledit alkyle ou hétéroaryle est éventuellement substitué de manière facultative par un ou plusieurs groupes choisis parmi un alkyle (C₁-C₃), un haloalkyle (C1-C6) et un halo ;
R est choisi parmi le groupe constitué par un alkyle (C1-C6)-CN, haloalkyle (C1-C6), -NRARB, hétérocycloalkyle (C3-C8)-alkyle (C1-C6), hétérocycloalkyle (C3-C8), hétéroaryle (C3-C8)-alkyle (C1-C6), cycloalkyle (C3-C8)-alkyle (C1-C6), cycloalkyle (C3-C8), RAO-alkyle (C1-C6)-O-alkyle (C1-C6), RANH-C(O)-alkyle (C1-C4), RARBN-C(O)-alkyle (C1-C4) et RAO-C(O) alkyle (C1-C6),
dans lequel ledit hétérocycloalkyle est substitué par un ou plusieurs groupes choisis parmi -OH, -C(O)R_{A}, -C(O)OR_{A}, un haloalkyle (C₁-C₆), -NH-C(O)R₁ et un oxo ;
ledit cycloalkyle est substitué par un ou plusieurs groupes choisis parmi -C(O)OR_{A} et un haloalkyle (C₁-C₆),
ledit hétéroaryle est éventuellement substitué de manière facultative par un ou plusieurs groupes choisis parmi un alkyle (C₁-C₆), -OH et un cycloalkyle (C₃-C₆) ; **R_{A}** et **R_{B}** sont à chaque occurrence indépendamment H ou choisis parmi le groupe constitué par un alkyle (C₁-C₆), haloalkyle (C₁-C₆), -OR₁, -SO₂R_{C} et cycloalkyle (C₃-C₆) dans lequel ledit cycloalkyle est éventuellement substitué de manière facultative par un ou plusieurs C(O)OR₁ ; et
**R_{C}** est un aryle.

5. Composé de formule (I) selon la revendication 1, dans lequel X est SO₂, représenté par la formule (Ib) 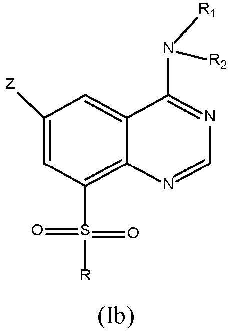 dans lequel
**Z** est un aryle, dans lequel ledit aryle est éventuellement substitué de manière facultative par un ou plusieurs groupes choisis parmi un alkyle (C1-C3), un halo et un haloalkyle (C1-C6) ;
**R₁** est H ou un alkyle (C₁-C₄) ;
**R₂** est un hétéroaryle alkyle (C₁-C₄), dans lequel ledit alkyle ou hétéroaryle est éventuellement substitué de manière facultative par un ou plusieurs groupes choisis parmi un alkyle (C₁-C₃), un haloalkyle (C1-C6) et un halo ;
**R** est -NR_{A} R_{B};
**R_{A}** et **R_{B}** sont à chaque occurrence indépendamment H ou alkyle (C₁-C₆), ou alternativement
**R_{A}** et **R_{B}** peuvent former ensemble, avec l'atome d'azote auquel ils sont liés un système d'anneau monocyclique hétérocyclique saturé à 5 ou 6 chaînons contenant de manière facultative un autre hétéroatome qui est l'azote ou l'oxygène, qui est éventuellement substitué de manière facultative par un ou plusieurs groupes choisis parmi un halo et -OR₁

6. Composition pharmaceutique comprenant un composé tel que défini dans l'une des revendications 1 à 5, ou un sel pharmaceutiquement acceptable de celui-ci, seul ou en association avec un ou plusieurs autres principes actifs, en mélange avec un ou plusieurs supports ou excipients pharmaceutiquement acceptables.

7. Composition pharmaceutique selon la revendication 6 pour administration par voie orale.

8. Composé selon l'une des revendications 1 à 5, ou composition pharmaceutique selon les revendications 6 et 7 pour une utilisation comme médicament.

9. Composé selon l'une des revendications 1 à 5 ou composition pharmaceutique selon les revendications 6 et 7 pour une utilisation dans le traitement de toute maladie dans laquelle les récepteurs P2X3 sont impliqués.

10. Composé selon l'une des revendications 1 à 5 ou composition pharmaceutique selon les revendications 6 et 7 pour une utilisation dans la prévention et/ou le traitement des maladies respiratoires, y compris la toux, la toux subaiguë ou chronique, la toux résistante au traitement, la toux chronique idiopathique, la toux post-virale, la toux iatrogène, l'asthme, la fibrose pulmonaire idiopathique (FPI), la bronchopneumopathie chronique obstructive (BPCO) et la toux associée à des maladies respiratoires telles que la BPCO, l'asthme et le bronchospasme.

11. Composé selon l'une des revendications 1 à 5 ou composition pharmaceutique selon les revendications 6 et 7 pour une utilisation dans la prévention et/ou le traitement de la toux chronique.
